# EUROPEAN PATENT APPLICATION

(11) **EP 2 286 840 A2**
(43) Date of publication of application: **23.02.2011**
(21) Application number: 10178805.7
(22) Date of filing: 01.11.2005
(51) Int. Cl.: A61K 45/06, A61K 38/22, A61P 3/00, A61P 3/04

(54) **Treatment of obesity and related diseases**

(30) Priority: 01.11.2004 US 624357 P
(62) Divisional of application: 09003342.4
(71) Applicant: AMYLIN PHARMACEUTICALS, INC., San Diego, CA 92121 (US)
(72) Inventor: Roth, Jonathan D., San Diego, CA 92121 (US); Anderson, Christen M., Encinitas, CA 92024 (US); Baron, Alain, San Diego, CA 92121 (US)
(74) Representative: Siegert, Georg

(57) **Abstract**

Methods for treating obesity or obesity related disorders are disclosed. These methods include the use of anti-obesity agents directed to the forebrain in combination with anti-obesity agents directed to the hindbrain.

SEQUENCE LISTING

<110>  Amylin Pharmaceuticals, Inc.

<120>  Methods for Treating Obesity and Obesity Related Diseases and
       Disorders

<130>  144 061

<150>  US 60/624,357
<151>  2004-11-01

<160>  191

<170>  PatentIn version 3.3

<210>  1
<211>  37
<212>  PRT
<213>  Homo sapiens

<400>  1

<210>  2
<211>  37
<212>  PRT
<213>  Rattus norvegicus

<400>  2

Lys Cys Asn Thr Ala Thr Cys Ala Thr Gln Arg Leu Ala Asn Phe Leu
1               5                   10                  15

Val Arg Ser Ser Asn Asn Leu Gly Pro Val Leu Pro Pro Thr Asn Val
            20                  25                  30

Gly Ser Asn Thr Tyr
        35

<210>  3
<211>  37
<212>  PRT
<213>  Artificial Sequence

<220>
<223>  Synthetic peptide construct

<220>
<221>  MISC_FEATURE
<223>  residues 2 and 7 are independently selected amino acid residues
       having side chains which are chemically bonded to each other to

form an intramolecular linkage

<220>
<221> MISC_FEATURE
<223> c-term may be amino, alkylamino, dialkylamino, cycloalkylamino,
      arylamino, aralkylamino, alkyloxy, aryloxy, or aralkyloxy

<220>
<221> MISC_FEATURE
<222> (1)..(1)
<223> Lys, Ala, Ser, or hydrogen

<220>
<221> MISC_FEATURE
<222> (2)..(2)
<223> variable amino acid

<220>
<221> MISC_FEATURE
<222> (7)..(7)
<223> variable amino acid

<220>
<221> MISC_FEATURE
<222> (13)..(13)
<223> Ala, Ser, or Thr

<220>
<221> MISC_FEATURE
<222> (17)..(17)
<223> Val, Leu or Ile

<220>
<221> MISC_FEATURE
<222> (18)..(18)
<223> His or Arg

<220>
<221> MISC_FEATURE
<222> (19)..(19)
<223> Ser or Thr

<220>
<221> MISC_FEATURE
<222> (20)..(20)
<223> Ser, Thr, Gln, or Asn

<220>
<221> MISC_FEATURE
<222> (21)..(21)
<223> Asn, Gln, or His

<220>
<221> MISC_FEATURE
<222> (23)..(23)
<223> Phe, Leu, or Tyr

<220>
<221> MISC_FEATURE
<222> (25)..(25)
<223> Ala or Pro

<220>
<221> MISC_FEATURE
<222> (26)..(26)
<223> Ile, Val, Ala, or Leu

<220>

<221>  MISC_FEATURE
<222>  (28)..(28)
<223>  Ser, Pro, Leu, Ile, or Thr

<220>
<221>  MISC_FEATURE
<222>  (29)..(29)
<223>  Ser, Pro, or Thr

<220>
<221>  MISC_FEATURE
<222>  (31)..(31)
<223>  Asn, Asp, or Gln

<400>  3

Xaa Xaa Asn Thr Ala Thr Xaa Ala Thr Gln Arg Leu Xaa Asn Phe Leu
1               5                   10                  15

Xaa Xaa Xaa Xaa Xaa Asn Xaa Gly Xaa Xaa Leu Xaa Xaa Thr Xaa Val
            20                  25                  30

Gly Ser Asn Thr Tyr
        35

<210>  4
<211>  36
<212>  PRT
<213>  Artificial Sequence

<220>
<223>  Synthetic peptide construct

<400>  4

Cys Asn Thr Ala Thr Cys Ala Thr Gln Arg Leu Ala Asn Phe Leu Val
1               5                   10                  15

His Ser Ser Asn Asn Phe Gly Ala Ile Leu Ser Ser Thr Asn Val Gly
            20                  25                  30

Ser Asn Thr Tyr
        35

<210>  5
<211>  37
<212>  PRT
<213>  Artificial Sequence

<220>
<223>  Synthetic peptide construct

<400>  5

Lys Cys Asn Thr Ala Thr Cys Ala Thr Gln Arg Leu Ala Asn Phe Leu
1               5                   10                  15

Val His Ser Ser Asn Asn Phe Gly Ala Ile Leu Pro Ser Thr Asn Val
            20                  25                  30

Gly Ser Asn Thr Tyr
        35


<210>  6
<211>  37
<212>  PRT
<213>  Artificial Sequence

<220>
<223>  Synthetic peptide construct

<400>  6

Lys Cys Asn Thr Ala Thr Cys Ala Thr Gln Arg Leu Ala Asn Phe Leu
1               5                   10                  15


Val His Ser Ser Asn Asn Phe Gly Pro Ile Leu Pro Pro Thr Asn Val
            20                  25                  30


Gly Ser Asn Thr Tyr
        35


<210>  7
<211>  37
<212>  PRT
<213>  Artificial Sequence

<220>
<223>  Synthetic peptide construct

<400>  7

Lys Cys Asn Thr Ala Thr Cys Ala Thr Gln Arg Leu Ala Asn Phe Leu
1               5                   10                  15


Val Arg Ser Ser Asn Asn Phe Gly Pro Ile Leu Pro Ser Thr Asn Val
            20                  25                  30


Gly Ser Asn Thr Tyr
        35


<210>  8
<211>  36
<212>  PRT
<213>  Artificial Sequence

<220>
<223>  Synthetic peptide construct

<400>  8

Cys Asn Thr Ala Thr Cys Ala Thr Gln Arg Leu Ala Asn Phe Leu Val
1               5                   10                  15


His Arg Ser Asn Asn Phe Gly Pro Ile Leu Pro Ser Thr Asn Val Gly
            20                  25                  30


Ser Asn Thr Tyr
        35

<210>  9
<211>  37
<212>  PRT
<213>  Artificial Sequence

<220>
<223>  Synthetic peptide construct

<400>  9

Lys Cys Asn Thr Ala Thr Cys Ala Thr Gln Arg Leu Ala Asn Phe Leu
1               5                   10                  15

Val His Ser Ser Asn Asn Phe Gly Pro Val Leu Pro Pro Thr Asn Val
            20                  25                  30

Gly Ser Asn Thr Tyr
        35

<210>  10
<211>  37
<212>  PRT
<213>  Artificial Sequence

<220>
<223>  Synthetic peptide construct

<400>  10

Lys Cys Asn Thr Ala Thr Cys Ala Thr Gln Arg Leu Ala Asn Phe Leu
1               5                   10                  15

Val His Ser Ser Asn Asn Phe Gly Pro Ile Leu Pro Pro Thr Asn Val
            20                  25                  30

Gly Ser Asn Thr Tyr
        35

<210>  11
<211>  37
<212>  PRT
<213>  Artificial Sequence

<220>
<223>  Synthetic peptide construct

<400>  11

Lys Cys Asn Thr Ala Thr Cys Ala Thr Gln Arg Leu Ala Asn Phe Leu
1               5                   10                  15

Val Arg Ser Ser Asn Asn Phe Gly Pro Ile Leu Pro Pro Thr Asn Val
            20                  25                  30

Gly Ser Asn Thr Tyr
        35

<210>  12
<211>  36
<212>  PRT
<213>  Artificial Sequence

<220>
<223>  Synthetic peptide construct

<400>  12

Cys Asn Thr Ala Thr Cys Ala Thr Gln Arg Leu Ala Asn Phe Leu Val
1               5                   10                  15

Arg Ser Ser Asn Asn Phe Gly Pro Ile Leu Pro Pro Ser Asn Val Gly
            20                  25                  30

Ser Asn Thr Tyr
        35

<210>  13
<211>  36
<212>  PRT
<213>  Artificial Sequence

<220>
<223>  Synthetic peptide construct

<400>  13

Cys Asn Thr Ala Thr Cys Ala Thr Gln Arg Leu Ala Asn Phe Leu Val
1               5                   10                  15

His Ser Ser Asn Asn Phe Gly Pro Ile Leu Pro Pro Ser Asn Val Gly
            20                  25                  30

Ser Asn Thr Tyr
        35

<210>  14
<211>  37
<212>  PRT
<213>  Artificial Sequence

<220>
<223>  Synthetic peptide construct

<400>  14

Lys Cys Asn Thr Ala Thr Cys Ala Thr Gln Arg Leu Ala Asn Phe Leu
1               5                   10                  15

Val His Ser Ser Asn Asn Phe Gly Pro Val Leu Pro Pro Thr Asn Val
            20                  25                  30

Gly Ser Asn Thr Tyr
        35

<210>  15
<211>  37

<212>  PRT
<213>  Artificial Sequence

<220>
<223>  Synthetic peptide construct

<400>  15

Lys Cys Asn Thr Ala Thr Cys Ala Thr Gln Arg Leu Ala Asn Phe Leu
1               5                   10                  15

Val His Ser Ser Asn Asn Leu Gly Pro Val Leu Pro Pro Thr Asn Val
            20                  25                  30

Gly Ser Asn Thr Tyr
        35

<210>  16
<211>  37
<212>  PRT
<213>  Artificial Sequence

<220>
<223>  Synthetic peptide construct

<400>  16

Lys Cys Asn Thr Ala Thr Cys Ala Thr Gln Arg Leu Ala Asn Phe Leu
1               5                   10                  15

Val His Ser Ser Asn Asn Leu Gly Pro Val Leu Pro Ser Thr Asn Val
            20                  25                  30

Gly Ser Asn Thr Tyr
        35

<210>  17
<211>  36
<212>  PRT
<213>  Artificial Sequence

<220>
<223>  Synthetic peptide construct

<400>  17

Cys Asn Thr Ala Thr Cys Ala Thr Gln Arg Leu Ala Asn Phe Leu Val
1               5                   10                  15

His Ser Ser Asn Asn Leu Gly Pro Val Leu Pro Ser Thr Asn Val Gly
            20                  25                  30

Ser Asn Thr Tyr
        35

<210>  18
<211>  37
<212>  PRT
<213>  Artificial Sequence

<220>
<223>  Synthetic peptide construct

<400>  18

Lys Cys Asn Thr Ala Thr Cys Ala Thr Gln Arg Leu Ala Asn Phe Leu
1               5                   10                  15

Val Arg Ser Ser Asn Asn Leu Gly Pro Val Leu Pro Ser Thr Asn Val
            20                  25                  30

Gly Ser Asn Thr Tyr
        35

<210>  19
<211>  37
<212>  PRT
<213>  Artificial Sequence

<220>
<223>  Synthetic peptide construct

<400>  19

Lys Cys Asn Thr Ala Thr Cys Ala Thr Gln Arg Leu Ala Asn Phe Leu
1               5                   10                  15

Val Arg Ser Ser Asn Asn Leu Gly Pro Ile Leu Pro Pro Thr Asn Val
            20                  25                  30

Gly Ser Asn Thr Tyr
        35

<210>  20
<211>  37
<212>  PRT
<213>  Artificial Sequence

<220>
<223>  Synthetic peptide construct

<400>  20

Lys Cys Asn Thr Ala Thr Cys Ala Thr Gln Arg Leu Ala Asn Phe Leu
1               5                   10                  15

Val Arg Ser Ser Asn Asn Leu Gly Pro Ile Leu Pro Ser Thr Asn Val
            20                  25                  30

Gly Ser Asn Thr Tyr
        35

<210>  21
<211>  37
<212>  PRT
<213>  Artificial Sequence

<220>

<223>  Synthetic peptide construct

<400>  21

Lys Cys Asn Thr Ala Thr Cys Ala Thr Gln Arg Leu Ala Asn Phe Leu
1               5                   10                  15

Ile His Ser Ser Asn Asn Leu Gly Pro Ile Leu Pro Pro Thr Asn Val
            20                  25                  30

Gly Ser Asn Thr Tyr
        35

<210>  22
<211>  37
<212>  PRT
<213>  Artificial Sequence

<220>
<223>  Synthetic peptide construct

<400>  22

Lys Cys Asn Thr Ala Thr Cys Ala Thr Gln Arg Leu Ala Asn Phe Leu
1               5                   10                  15

Ile His Ser Ser Asn Asn Phe Gly Pro Ile Leu Pro Pro Thr Asn Val
            20                  25                  30

Gly Ser Asn Thr Tyr
        35

<210>  23
<211>  36
<212>  PRT
<213>  Artificial Sequence

<220>
<223>  Synthetic peptide construct

<400>  23

Cys Asn Thr Ala Thr Cys Ala Thr Gln Arg Leu Ala Asn Phe Leu Ile
1               5                   10                  15

His Ser Ser Asn Asn Leu Gly Pro Ile Leu Pro Pro Thr Asn Val Gly
            20                  25                  30

Ser Asn Thr Tyr
        35

<210>  24
<211>  37
<212>  PRT
<213>  Artificial Sequence

<220>
<223>  Synthetic peptide construct

<400>  24

Lys Cys Asn Thr Ala Thr Cys Ala Thr Gln Arg Leu Ala Asn Phe Leu
1               5                   10                  15

Ile Arg Ser Ser Asn Asn Leu Gly Ala Ile Leu Ser Ser Thr Asn Val
            20                  25                  30

Gly Ser Asn Thr Tyr
        35

<210>  25
<211>  37
<212>  PRT
<213>  Artificial Sequence

<220>
<223>  Synthetic peptide construct

<400>  25

Lys Cys Asn Thr Ala Thr Cys Ala Thr Gln Arg Leu Ala Asn Phe Leu
1               5                   10                  15

Ile Arg Ser Ser Asn Asn Leu Gly Ala Val Leu Ser Pro Thr Asn Val
            20                  25                  30

Gly Ser Asn Thr Tyr
        35

<210>  26
<211>  37
<212>  PRT
<213>  Artificial Sequence

<220>
<223>  Synthetic peptide construct

<400>  26

Lys Cys Asn Thr Ala Thr Cys Ala Thr Gln Arg Leu Ala Asn Phe Leu
1               5                   10                  15

Ile Arg Ser Ser Asn Asn Leu Gly Pro Val Leu Pro Pro Thr Asn Val
            20                  25                  30

Gly Ser Asn Thr Tyr
        35

<210>  27
<211>  37
<212>  PRT
<213>  Artificial Sequence

<220>
<223>  Synthetic peptide construct

<400>  27

Lys Cys Asn Thr Ala Thr Cys Ala Thr Gln Arg Leu Thr Asn Phe Leu
1               5                   10                  15

Val His Ser Ser His Asn Leu Gly Ala Ala Leu Leu Pro Thr Asp Val
            20                  25                  30

Gly Ser Asn Thr Tyr
        35

<210>  28
<211>  37
<212>  PRT
<213>  Artificial Sequence

<220>
<223>  Synthetic peptide construct

<400>  28

Lys Cys Asn Thr Ala Thr Cys Ala Thr Gln Arg Leu Thr Asn Phe Leu
1               5                   10                  15

Val His Ser Ser His Asn Leu Gly Ala Ala Leu Ser Pro Thr Asp Val
            20                  25                  30

Gly Ser Asn Thr Tyr
        35

<210>  29
<211>  36
<212>  PRT
<213>  Artificial Sequence

<220>
<223>  Synthetic peptide construct

<400>  29

Cys Asn Thr Ala Thr Cys Ala Thr Gln Arg Leu Thr Asn Phe Leu Val
1               5                   10                  15

His Ser Ser His Asn Leu Gly Ala Ala Leu Pro Ser Thr Asp Val Gly
            20                  25                  30

Ser Asn Thr Tyr
        35

<210>  30
<211>  37
<212>  PRT
<213>  Artificial Sequence

<220>
<223>  Synthetic peptide construct

<400>  30

Lys Cys Asn Thr Ala Thr Cys Ala Thr Gln Arg Leu Thr Asn Phe Leu
1               5                   10                  15

Val Arg Ser Ser His Asn Leu Gly Ala Ala Leu Ser Pro Thr Asp Val
            20                  25                  30

Gly Ser Asn Thr Tyr
        35

<210>  31
<211>  37
<212>  PRT
<213>  Artificial Sequence

<220>
<223>  Synthetic peptide construct

<400>  31

Lys Cys Asn Thr Ala Thr Cys Ala Thr Gln Arg Leu Thr Asn Phe Leu
1               5                   10                  15

Val Arg Ser Ser His Asn Leu Gly Ala Ile Leu Pro Pro Thr Asp Val
            20                  25                  30

Gly Ser Asn Thr Tyr
        35

<210>  32
<211>  37
<212>  PRT
<213>  Artificial Sequence

<220>
<223>  Synthetic peptide construct

<400>  32

Lys Cys Asn Thr Ala Thr Cys Ala Thr Gln Arg Leu Thr Asn Phe Leu
1               5                   10                  15

Val Arg Ser Ser His Asn Leu Gly Pro Ala Leu Pro Pro Thr Asp Val
            20                  25                  30

Gly Ser Asn Thr Tyr
        35

<210>  33
<211>  32
<212>  PRT
<213>  Oncorhynchus sp.

<400>  33

Cys Ser Asn Leu Ser Thr Cys Val Leu Gly Lys Leu Ser Gln Glu Leu
1               5                   10                  15

His Lys Leu Gln Thr Tyr Pro Arg Thr Asn Thr Gly Ser Gly Thr Pro
            20                  25                  30

<210>  34
<211>  32
<212>  PRT
<213>  Homo sapiens

<400>  34

Cys Gly Asn Leu Ser Thr Cys Met Leu Gly Thr Tyr Thr Gln Asp Phe
1               5                   10                  15

Asn Lys Phe His Thr Phe Pro Gln Thr Ala Ile Gly Val Gly Ala Pro
            20                  25                  30

<210>  35
<211>  6
<212>  PRT
<213>  Artificial Sequence

<220>
<223>  Synthetic peptide construct

<220>
<221>  MISC_FEATURE
<222>  (1)..(1)
<223>  Any amino acid, see specification for detailed description of
       substitutions and embodiments

<220>
<221>  MISC_FEATURE
<222>  (2)..(2)
<223>  Any amino acid, see specification for detailed description of
       substitutions and embodiments

<220>
<221>  MISC_FEATURE
<222>  (3)..(3)
<223>  Any amino acid, see specification for detailed description of
       substitutions and embodiments

<220>
<221>  MISC_FEATURE
<222>  (4)..(4)
<223>  Any amino acid, see specification for detailed description of
       substitutions and embodiments

<220>
<221>  MISC_FEATURE
<222>  (5)..(5)
<223>  Any amino acid, see specification for detailed description of
       substitutions and embodiments

<220>
<221>  MISC_FEATURE
<222>  (6)..(6)
<223>  Any amino acid, see specification for detailed description of
       substitutions and embodiments

<400>  35

Xaa Xaa Xaa Xaa Xaa Xaa
1               5

<210>  36

<211>  6
<212>  PRT
<213>  Artificial Sequence

<220>
<223>  Synthetic peptide construct

<400>  36

Cys Asn Thr Ala Thr Cys
1               5


<210>  37
<211>  6
<212>  PRT
<213>  Artificial Sequence

<220>
<223>  Synthetic peptide construct

<400>  37

Cys Ala Thr Ala Thr Cys
1               5


<210>  38
<211>  6
<212>  PRT
<213>  Artificial Sequence

<220>
<223>  Synthetic peptide construct

<400>  38

Cys Asp Thr Ala Thr Cys
1               5


<210>  39
<211>  6
<212>  PRT
<213>  Artificial Sequence

<220>
<223>  Synthetic peptide construct

<400>  39

Cys Gly Thr Ala Thr Cys
1               5


<210>  40
<211>  6
<212>  PRT
<213>  Artificial Sequence

<220>
<223>  Synthetic peptide construct

<400>  40

Cys Asn Ala Ala Thr Cys
1               5

<210>  41
<211>  6
<212>  PRT
<213>  Artificial Sequence

<220>
<223>  Synthetic peptide construct

<400>  41

Cys Asn Thr Ser Thr Cys
1               5


<210>  42
<211>  6
<212>  PRT
<213>  Artificial Sequence

<220>
<223>  Synthetic peptide construct


<220>
<221>  MISC_FEATURE
<222>  (5)..(5)
<223>  D-Thr

<400>  42

Cys Asn Thr Ala Xaa Cys
1               5


<210>  43
<211>  6
<212>  PRT
<213>  Artificial Sequence

<220>
<223>  Synthetic peptide construct


<220>
<221>  MOD_RES
<222>  (5)..(5)
<223>  Thr(OPO3H2)

<400>  43

Cys Asn Thr Ala Xaa Cys
1               5


<210>  44
<211>  6
<212>  PRT
<213>  Artificial Sequence

<220>
<223>  Synthetic peptide construct

<400>  44

Cys Asn Thr Ala Ser Cys
1               5

<210>  45
<211>  6
<212>  PRT
<213>  Artificial Sequence

<220>
<223>  Synthetic peptide construct

<400>  45

Cys Asn Thr Ala Ala Cys
1               5


<210>  46
<211>  6
<212>  PRT
<213>  Artificial Sequence

<220>
<223>  Synthetic peptide construct

<400>  46

Cys Asn Thr Ala Val Cys
1               5


<210>  47
<211>  6
<212>  PRT
<213>  Artificial Sequence

<220>
<223>  Synthetic peptide construct


<220>
<221>  MOD_RES
<222>  (5)..(5)
<223>  Hse

<400>  47

Cys Asn Thr Ala Xaa Cys
1               5


<210>  48
<211>  6
<212>  PRT
<213>  Artificial Sequence

<220>
<223>  Synthetic peptide construct


<220>
<221>  MOD_RES
<222>  (5)..(5)
<223>  Ahb

<400>  48

Cys Asn Thr Ala Xaa Cys
1               5

<210>  49
<211>  6
<212>  PRT
<213>  Artificial Sequence

<220>
<223>  Synthetic peptide construct


<220>
<221>  MOD_RES
<222>  (5)..(5)
<223>  Ahp

<400>  49

Cys Asn Thr Ala Xaa Cys
1               5


<210>  50
<211>  7
<212>  PRT
<213>  Artificial Sequence

<220>
<223>  Synthetic peptide construct

<400>  50

Cys Ser Asn Leu Ser Thr Cys
1               5


<210>  51
<211>  7
<212>  PRT
<213>  Artificial Sequence

<220>
<223>  Synthetic peptide construct

<400>  51

Cys Gly Asn Leu Ser Thr Cys
1               5


<210>  52
<211>  7
<212>  PRT
<213>  Artificial Sequence

<220>
<223>  Synthetic peptide construct

<400>  52

Cys Ala Asn Leu Ser Thr Cys
1               5


<210>  53
<211>  7
<212>  PRT
<213>  Artificial Sequence

<220>
<223>  Synthetic peptide construct

<400>  53

Cys Ser Ala Leu Ser Thr Cys
1               5


<210>  54
<211>  7
<212>  PRT
<213>  Artificial Sequence

<220>
<223>  Synthetic peptide construct

<400>  54

Cys Ser Asn Ala Ser Thr Cys
1               5


<210>  55
<211>  7
<212>  PRT
<213>  Artificial Sequence

<220>
<223>  Synthetic peptide construct

<400>  55

Cys Ser Asn Leu Ala Thr Cys
1               5


<210>  56
<211>  7
<212>  PRT
<213>  Artificial Sequence

<220>
<223>  Synthetic peptide construct

<400>  56

Cys Ser Asn Leu Ser Ala Cys
1               5


<210>  57
<211>  7
<212>  PRT
<213>  Artificial Sequence

<220>
<223>  Synthetic peptide construct

<400>  57

Lys Cys Asn Thr Ala Thr Cys
1               5


<210>  58
<211>  15
<212>  PRT

<213>  Artificial Sequence

<220>
<223>  Synthetic peptide construct

<400>  58

Leu Leu Gln Gln Leu Gln Lys Leu Leu Gln Lys Leu Lys Gln Tyr
1               5                   10                  15


<210>  59
<211>  28
<212>  PRT
<213>  Artificial Sequence

<220>
<223>  Synthetic peptide construct


<220>
<221>  MISC_FEATURE
<222>  (1)..(4)
<223>  This region may be absent or encompass 1-4 variable amino acids

<220>
<221>  MISC_FEATURE
<222>  (7)..(7)
<223>  Gly or Aib

<220>
<221>  MISC_FEATURE
<222>  (8)..(8)
<223>  Lys, Arg, Orn, hArg, Cit, hLys, or Lys(for)

<220>
<221>  MISC_FEATURE
<222>  (12)..(12)
<223>  Glu or Phe

<220>
<221>  MISC_FEATURE
<222>  (14)..(14)
<223>  His or Aib

<220>
<221>  MISC_FEATURE
<222>  (15)..(15)
<223>  Lys, Arg, Orn, hArg, Cit, hLys, Lys(for), or Lys(PEG 5000)

<220>
<221>  MISC_FEATURE
<222>  (19)..(19)
<223>  Tyr or Leu

<220>
<221>  MISC_FEATURE
<222>  (21)..(21)
<223>  Arg or Pro

<220>
<221>  MISC_FEATURE
<222>  (24)..(27)
<223>  This region may be absent or encompass 1-4 variable amino acids

<220>
<221>  misc_feature
<222>  (28)..(28)

<223>  Xaa can be any naturally occurring amino acid

<400>  59

Xaa Xaa Xaa Xaa Val Leu Xaa Xaa Leu Ser Gln Xaa Leu Xaa Xaa Leu
1               5                   10                  15

Gln Thr Xaa Pro Xaa Thr Asn Thr Xaa Xaa Xaa Xaa
            20                  25

<210>  60
<211>  28
<212>  PRT
<213>  Artificial Sequence

<220>
<223>  Synthetic peptide construct

<220>
<221>  MISC_FEATURE
<222>  (1)..(4)
<223>  This region may be absent or encompass 1-4 variable amino acids

<220>
<221>  MISC_FEATURE
<222>  (5)..(5)
<223>  Ala or Val

<220>
<221>  MISC_FEATURE
<222>  (6)..(6)
<223>  Thr, Met or Leu

<220>
<221>  MISC_FEATURE
<222>  (7)..(7)
<223>  Gln, Gly, or His

<220>
<221>  MISC_FEATURE
<222>  (8)..(8)
<223>  Leu or Thr

<220>
<221>  MISC_FEATURE
<222>  (9)..(9)
<223>  Leu or Thr

<220>
<221>  MISC_FEATURE
<222>  (10)..(10)
<223>  Ala, Thr, Asn, Phe, Tyr, Ser, or Thr

<220>
<221>  MISC_FEATURE
<222>  (11)..(11)
<223>  Asn, Arg, Ala, Asp, Glu, Gln, Thr, or Gly

<220>
<221>  MISC_FEATURE
<222>  (12)..(12)
<223>  Phe, Leu, Ser, Glu, Ala, Asp, or Tyr

<220>
<221>  MISC_FEATURE

<222>  (13)..(13)
<223>  Leu or Asp

<220>
<221>  MISC_FEATURE
<222>  (14)..(14)
<223>  Val, His, Ser, Phe, or Aib

<220>
<221>  MISC_FEATURE
<222>  (15)..(15)
<223>  His, Arg, Lys, Orn, hArg, Cit, hLys, Lys(for), or Lys(PEG5000)

<220>
<221>  MISC_FEATURE
<222>  (16)..(16)
<223>  Leu, Ser, or Phe

<220>
<221>  MISC_FEATURE
<222>  (17)..(17)
<223>  Gln or His

<220>
<221>  MISC_FEATURE
<222>  (18)..(18)
<223>  Thr or Asn

<220>
<221>  MISC_FEATURE
<222>  (19)..(19)
<223>  Tyr, Val, Phe, Leu or Met

<220>
<221>  MISC_FEATURE
<222>  (21)..(21)
<223>  Arg or Pro

<220>
<221>  MISC_FEATURE
<222>  (25)..(28)
<223>  This region may be absent or encompass 1-4 variable amino acids

<400>  60

Xaa Xaa Xaa Xaa Xaa Xaa Xaa Arg Xaa Xaa Xaa Xaa Xaa Xaa Xaa Xaa
1               5                   10                  15


Xaa Xaa Xaa Pro Xaa Thr Asn Thr Xaa Xaa Xaa Xaa
            20                  25


<210>  61
<211>  11
<212>  PRT
<213>  Artificial Sequence

<220>
<223>  Synthetic peptide construct


<220>
<221>  MISC_FEATURE
<222>  (1)..(1)
<223>  Lys, Tyr, or absent

<220>

<221>  MISC_FEATURE
<222>  (2)..(2)
<223>  Ser, Pro, or absent

<220>
<221>  MISC_FEATURE
<222>  (3)..(3)
<223>  Ser, Pro, Arg, or absent

<220>
<221>  MISC_FEATURE
<222>  (4)..(4)
<223>  Thr or absent

<220>
<221>  MISC_FEATURE
<222>  (5)..(5)
<223>  Asn or absent

<220>
<221>  MISC_FEATURE
<222>  (6)..(6)
<223>  Val, Thr, or absent

<220>
<221>  MISC_FEATURE
<222>  (8)..(8)
<223>  Ser or Glu

<220>
<221>  MISC_FEATURE
<222>  (9)..(9)
<223>  Asn, Lys, or Gly

<220>
<221>  MISC_FEATURE
<222>  (10)..(10)
<223>  Thr, Phe, or Ala

<220>
<221>  MISC_FEATURE
<222>  (11)..(11)
<223>  Tyr, Phe, Pro, or absent

<400>  61

Xaa Xaa Xaa Xaa Xaa Xaa Gly Xaa Xaa Xaa Xaa
1               5                   10


<210>  62
<211>  8
<212>  PRT
<213>  Artificial Sequence

<220>
<223>  Synthetic peptide construct

<400>  62

Lys Ser Asn Phe Val Pro Thr Asn
1               5


<210>  63
<211>  8
<212>  PRT
<213>  Artificial Sequence

<220>
<223>  Synthetic peptide construct

<400>  63

Ser Asn Phe Val Pro Thr Asn Val
1               5


<210>  64
<211>  32
<212>  PRT
<213>  Artificial Sequence

<220>
<223>  Synthetic peptide construct


<220>
<221>  MISC_FEATURE
<223>  residues 2 and 7 are independently selected residues having side
       chains which are chemically bonded to each other to form an
       intramolecular linkage

<220>
<221>  MISC_FEATURE
<222>  (1)..(1)
<223>  A, C, hC, D, E, F, I, L, K, hK, R, hR, S, Hse, T, G, Q, N, M, Y,
       W, P, Hyp, H, V, or absent

<220>
<221>  MISC_FEATURE
<222>  (2)..(2)
<223>  variable amino acid

<220>
<221>  MISC_FEATURE
<222>  (3)..(3)
<223>  A, D, E, N, Q, G, V, R, K, hK, hR, H, I, L, M, or absent

<220>
<221>  MISC_FEATURE
<222>  (4)..(4)
<223>  A, I, L, S, Hse, T, V, M, or absent

<220>
<221>  MISC_FEATURE
<222>  (5)..(5)
<223>  A, S, T, Hse, Y, V, I, L, or M

<220>
<221>  MISC_FEATURE
<222>  (6)..(6)
<223>  T, A, S, Hse, Y, V, I, L, or M

<220>
<221>  MISC_FEATURE
<222>  (7)..(7)
<223>  variable amino acid

<220>
<221>  MISC_FEATURE
<222>  (8)..(8)
<223>  A, V, I, L, F, or M

<220>
<221>  MISC_FEATURE

<222>   (9)..(9)
<223>   L, T, S, Hse, V, I, or M

<220>
<221>   MISC_FEATURE
<222>   (10)..(10)
<223>   G, H, Q, K, R, N, hK, or hR

<220>
<221>   MISC_FEATURE
<222>   (11)..(11)
<223>   K, R, Q, N, hK, hR, or H

<220>
<221>   MISC_FEATURE
<222>   (12)..(12)
<223>   L, I, V, F, M, W, or Y

<220>
<221>   MISC_FEATURE
<222>   (13)..(13)
<223>   A, F, Y, N, Q, S, Hse, or T

<220>
<221>   MISC_FEATURE
<222>   (14)..(14)
<223>   A, D, E, G, N, K, Q, R, H, hR, or hK

<220>
<221>   MISC_FEATURE
<222>   (15)..(15)
<223>   A, D, E, F, L, S, Y, I, V, or M

<220>
<221>   MISC_FEATURE
<222>   (16)..(16)
<223>   L, F, M, V, Y, or I

<220>
<221>   MISC_FEATURE
<222>   (17)..(17)
<223>   H, Q, N, S, Hse, T, or V

<220>
<221>   MISC_FEATURE
<222>   (18)..(18)
<223>   K, hK, R, hR, H, u (Cit), or n (Orn)

<220>
<221>   MISC_FEATURE
<222>   (19)..(19)
<223>   F, L, S, Hse,V, I, T, or absent

<220>
<221>   MISC_FEATURE
<222>   (20)..(20)
<223>   H, R, K, hR, hK, N, Q, or absent

<220>
<221>   MISC_FEATURE
<222>   (21)..(21)
<223>   T, S, Hse, V, I, L, Q, N, or absent

<220>
<221>   MISC_FEATURE
<222>   (22)..(22)
<223>   F, L, M, V, Y, or I

<220>
<221> MISC_FEATURE
<222> (23)..(23)
<223> P or Hyp

<220>
<221> MISC_FEATURE
<222> (24)..(24)
<223> P, Hyp, R,K, hR, hK, or H

<220>
<221> MISC_FEATURE
<222> (25)..(25)
<223> T, S, Hse, V, I, L, F, or Y

<220>
<221> MISC_FEATURE
<222> (26)..(26)
<223> N, Q, D, or E

<220>
<221> MISC_FEATURE
<222> (27)..(27)
<223> T, V, S, F, I, or L

<220>
<221> MISC_FEATURE
<222> (28)..(28)
<223> G or A

<220>
<221> MISC_FEATURE
<222> (29)..(29)
<223> S, Hse, T, V, I, L, or Y

<220>
<221> MISC_FEATURE
<222> (30)..(30)
<223> E, G, K, N, D, R, hR, hK, H, or Q

<220>
<221> MISC_FEATURE
<222> (31)..(31)
<223> A, T, S, Hse, V, I, L, F, or Y

<220>
<221> MISC_FEATURE
<222> (32)..(32)
<223> F, P, Y, Hse, S, T, or Hyp

<400> 64

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Xaa Xaa Xaa Xaa Xaa Xaa Xaa Xaa Xaa Xaa
            20                  25                  30

<210> 65
<211> 32
<212> PRT
<213> Artificial Sequence

<220>
<223> Synthetic peptide construct

<220>
<221>  MISC_FEATURE
<222>  (1)..(1)
<223>  A, C, D, F, I, K, S, T, or absent

<220>
<221>  MISC_FEATURE
<222>  (2)..(2)
<223>  C, D, S, or absent

<220>
<221>  MISC_FEATURE
<222>  (3)..(3)
<223>  A, D, N, or absent

<220>
<221>  MISC_FEATURE
<222>  (4)..(4)
<223>  A, L, T, or absent

<220>
<221>  MISC_FEATURE
<222>  (5)..(5)
<223>  A or S

<220>
<221>  MISC_FEATURE
<222>  (6)..(6)
<223>  T, A, S, or V

<220>
<221>  MISC_FEATURE
<222>  (7)..(7)
<223>  C, K, or A

<220>
<221>  MISC_FEATURE
<222>  (8)..(8)
<223>  A, V, L, or M

<220>
<221>  MISC_FEATURE
<222>  (9)..(9)
<223>  L or T

<220>
<221>  MISC_FEATURE
<222>  (10)..(10)
<223>  G, H, or Q

<220>
<221>  MISC_FEATURE
<222>  (11)..(11)
<223>  K, R, Q, or hArg

<220>
<221>  MISC_FEATURE
<222>  (12)..(12)
<223>  L, W, or Y

<220>
<221>  MISC_FEATURE
<222>  (13)..(13)
<223>  A, F, N, Q, S, or T

<220>
<221>  MISC_FEATURE

<222>  (14)..(14)
<223>  A, D, E, G, N, K, Q, or R

<220>
<221>  MISC_FEATURE
<222>  (15)..(15)
<223>  A, D, E, F, L, S, or Y

<220>
<221>  MISC_FEATURE
<222>  (16)..(16)
<223>  L or F

<220>
<221>  MISC_FEATURE
<222>  (17)..(17)
<223>  H, Q, S, or V

<220>
<221>  MISC_FEATURE
<222>  (18)..(18)
<223>  K, R, hArg, u (Cit), or n (Orn)

<220>
<221>  MISC_FEATURE
<222>  (19)..(19)
<223>  F, L, S, or absent

<220>
<221>  MISC_FEATURE
<222>  (20)..(20)
<223>  H, Q, or absent

<220>
<221>  MISC_FEATURE
<222>  (21)..(21)
<223>  T, N, or absent

<220>
<221>  MISC_FEATURE
<222>  (22)..(22)
<223>  F, L, M, V, or Y

<220>
<221>  MISC_FEATURE
<222>  (24)..(24)
<223>  P or R

<220>
<221>  MISC_FEATURE
<222>  (27)..(27)
<223>  T or V

<220>
<221>  MISC_FEATURE
<222>  (30)..(30)
<223>  E, G, K, or N

<220>
<221>  MISC_FEATURE
<222>  (31)..(31)
<223>  A or T

<220>
<221>  MISC_FEATURE
<222>  (32)..(32)
<223>  F, P, or Y

<400>  65

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Pro Xaa Thr Asn Xaa Gly Ser Xaa Xaa Xaa
            20                  25                  30

<210>  66
<211>  32
<212>  PRT
<213>  Artificial Sequence

<220>
<223>  Synthetic peptide construct

<220>
<221>  MISC_FEATURE
<222>  (1)..(1)
<223>  A, C, F, I, K, S, or absent

<220>
<221>  MISC_FEATURE
<222>  (2)..(2)
<223>  C, D, or S

<220>
<221>  MISC_FEATURE
<222>  (3)..(3)
<223>  A, D or N

<220>
<221>  MISC_FEATURE
<222>  (4)..(4)
<223>  A, L or T

<220>
<221>  MISC_FEATURE
<222>  (5)..(5)
<223>  A or S

<220>
<221>  MISC_FEATURE
<222>  (7)..(7)
<223>  C or K

<220>
<221>  MISC_FEATURE
<222>  (8)..(8)
<223>  A or V

<220>
<221>  MISC_FEATURE
<222>  (9)..(9)
<223>  L or T

<220>
<221>  MISC_FEATURE
<222>  (10)..(10)
<223>  G, H, or Q

<220>
<221>  MISC_FEATURE
<222>  (11)..(11)
<223>  K, R, or hArg

<220>
<221>  MISC_FEATURE
<222>  (13)..(13)
<223>  A, F, N, S, or T

<220>
<221>  MISC_FEATURE
<222>  (14)..(14)
<223>  A, D, E, G, N, Q, or R

<220>
<221>  MISC_FEATURE
<222>  (15)..(15)
<223>  A, E, F, L, S, or Y

<220>
<221>  MISC_FEATURE
<222>  (17)..(17)
<223>  H, S, or V

<220>
<221>  MISC_FEATURE
<222>  (18)..(18)
<223>  K, R, hArg, u (Cit), or n (Orn)

<220>
<221>  MISC_FEATURE
<222>  (19)..(19)
<223>  F, L, or S

<220>
<221>  MISC_FEATURE
<222>  (20)..(20)
<223>  H or Q

<220>
<221>  MISC_FEATURE
<222>  (21)..(21)
<223>  T or N

<220>
<221>  MISC_FEATURE
<222>  (22)..(22)
<223>  F, L, M, V, or Y

<220>
<221>  MISC_FEATURE
<222>  (24)..(24)
<223>  P or R

<220>
<221>  MISC_FEATURE
<222>  (27)..(27)
<223>  T or V

<220>
<221>  MISC_FEATURE
<222>  (30)..(30)
<223>  E, G, K, or N

<220>
<221>  MISC_FEATURE
<222>  (31)..(31)
<223>  A or T

<220>
<221>  MISC_FEATURE

<222>  (32)..(32)
<223>  F, P, or Y

<400>  66

Xaa Xaa Xaa Xaa Xaa Thr Xaa Xaa Xaa Xaa Xaa Leu Xaa Xaa Xaa Leu
1               5                   10                  15

Xaa Xaa Xaa Xaa Xaa Xaa Pro Xaa Thr Asn Xaa Gly Ser Xaa Xaa Xaa
            20                  25                  30

<210>  67
<211>  4
<212>  PRT
<213>  Artificial Sequence

<220>
<223>  Synthetic peptide construct

<400>  67

Leu Gln Thr Tyr
1

<210>  68
<211>  32
<212>  PRT
<213>  Artificial Sequence

<220>
<223>  Synthetic peptide construct

<220>
<221>  MISC_FEATURE
<222>  (1)..(1)
<223>  A, C, D, F, K, T, or absent

<220>
<221>  MISC_FEATURE
<222>  (2)..(2)
<223>  A, C, D, S, or absent

<220>
<221>  MISC_FEATURE
<222>  (3)..(3)
<223>  A, D, N, or absent

<220>
<221>  MISC_FEATURE
<222>  (4)..(4)
<223>  A, L, T, or absent

<220>
<221>  MISC_FEATURE
<222>  (5)..(5)
<223>  A or S

<220>
<221>  MISC_FEATURE
<222>  (6)..(6)
<223>  A, S, T, or V

<220>
<221>  MISC_FEATURE

<222>  (7)..(7)
<223>  A, C, or K

<220>
<221>  MISC_FEATURE
<222>  (8)..(8)
<223>  A, L, M, or V

<220>
<221>  MISC_FEATURE
<222>  (9)..(9)
<223>  L or T

<220>
<221>  MISC_FEATURE
<222>  (10)..(10)
<223>  G, H, or Q

<220>
<221>  MISC_FEATURE
<222>  (11)..(11)
<223>  K, Q, or R

<220>
<221>  MISC_FEATURE
<222>  (12)..(12)
<223>  L, W, or Y

<220>
<221>  MISC_FEATURE
<222>  (13)..(13)
<223>  A, N, Q, S, or T

<220>
<221>  MISC_FEATURE
<222>  (14)..(14)
<223>  A, D, E, G, K, N, Q, or R

<220>
<221>  MISC_FEATURE
<222>  (15)..(15)
<223>  A, D, E, F, L, S, or Y

<220>
<221>  MISC_FEATURE
<222>  (16)..(16)
<223>  F or L

<220>
<221>  MISC_FEATURE
<222>  (17)..(17)
<223>  H, Q, S or V

<220>
<221>  MISC_FEATURE
<222>  (18)..(18)
<223>  K or R

<220>
<221>  MISC_FEATURE
<222>  (19)..(19)
<223>  F, L, S, or absent

<220>
<221>  MISC_FEATURE
<222>  (20)..(20)
<223>  H, K, Q, or absent

<220>
<221> MISC_FEATURE
<222> (21)..(21)
<223> Q, T, or absent

<220>
<221> MISC_FEATURE
<222> (22)..(22)
<223> F, L, or Y

<220>
<221> MISC_FEATURE
<222> (24)..(24)
<223> P or R

<220>
<221> MISC_FEATURE
<222> (27)..(27)
<223> T or V

<220>
<221> MISC_FEATURE
<222> (30)..(30)
<223> E, K or N

<220>
<221> MISC_FEATURE
<222> (31)..(31)
<223> A or T

<220>
<221> MISC_FEATURE
<222> (32)..(32)
<223> F, Y, or absent

<400> 68

Xaa Xaa Xaa Xaa Xaa Xaa Xaa Xaa Xaa Xaa Xaa Xaa Xaa Xaa Xaa Xaa
1               5                   10                  15

Xaa Xaa Xaa Xaa Xaa Xaa Pro Xaa Thr Asn Xaa Gly Ser Xaa Xaa Xaa
            20                  25                  30

<210> 69
<211> 8
<212> PRT
<213> Artificial Sequence

<220>
<223> Synthetic peptide construct

<220>
<221> MISC_FEATURE
<223> residues 1 and 8 are independently selected residues having side
      chains which can be chemically bonded to each other to form an
      intramolecular linkage

<220>
<221> MISC_FEATURE
<222> (1)..(1)
<223> variable amino acid

<220>
<221> MISC_FEATURE
<222> (2)..(2)

<223>  variable amino acid or absent

<220>
<221>  MISC_FEATURE
<222>  (3)..(3)
<223>  Ala, Gly, Ser, Asp or absent

<220>
<221>  MISC_FEATURE
<222>  (4)..(4)
<223>  Asn, Ala, Asp, Gly or absent

<220>
<221>  MISC_FEATURE
<222>  (5)..(5)
<223>  Ala, Leu, Thr, or Ser

<220>
<221>  MISC_FEATURE
<222>  (6)..(6)
<223>  Ala, Ser, or Thr

<220>
<221>  MISC_FEATURE
<222>  (7)..(7)
<223>  Ala, Ser, Val, Hse, Ahb, Ahp, D-Thr, Thr, or a derivative thereof

<220>
<221>  MISC_FEATURE
<222>  (8)..(8)
<223>  variable amino acid

<400>  69

Xaa Xaa Xaa Xaa Xaa Xaa Xaa Xaa
1               5


<210>  70
<211>  28
<212>  PRT
<213>  Artificial Sequence

<220>
<223>  Synthetic peptide construct


<220>
<221>  MISC_FEATURE
<222>  (1)..(4)
<223>  This region may be absent or encompass 1-4 variable amino acids

<220>
<221>  MISC_FEATURE
<222>  (7)..(7)
<223>  Gly or Aib

<220>
<221>  MISC_FEATURE
<222>  (8)..(8)
<223>  Lys, Arg, Orn, hArg, Cit, hLys, or Lys(for)

<220>
<221>  MISC_FEATURE
<222>  (12)..(12)
<223>  Glu or Phe

<220>

<221>  MISC_FEATURE
<222>  (14)..(14)
<223>  His or Aib

<220>
<221>  MISC_FEATURE
<222>  (15)..(15)
<223>  Lys, Arg, Orn, hArg, Cit, hLys,Lys(for), or Lys(PEG 5000)

<220>
<221>  MISC_FEATURE
<222>  (19)..(19)
<223>  Tyr or Leu

<220>
<221>  MISC_FEATURE
<222>  (21)..(21)
<223>  Arg or Pro

<220>
<221>  MISC_FEATURE
<222>  (25)..(28)
<223>  This region may be absent or encompass 1-4 variable amino acids

<400>  70

Xaa Xaa Xaa Xaa Val Leu Xaa Xaa Leu Ser Gln Xaa Leu Xaa Xaa Leu
1               5                   10                  15

Gln Thr Xaa Pro Xaa Thr Asn Thr Xaa Xaa Xaa Xaa
            20                  25

<210>  71
<211>  11
<212>  PRT
<213>  Artificial Sequence

<220>
<223>  Synthetic peptide construct

<220>
<221>  MISC_FEATURE
<222>  (1)..(1)
<223>  Lys, Tyr, or absent

<220>
<221>  MISC_FEATURE
<222>  (2)..(2)
<223>  Ser, Pro, or absent

<220>
<221>  MISC_FEATURE
<222>  (3)..(3)
<223>  Ser, Pro, Arg, or absent

<220>
<221>  MISC_FEATURE
<222>  (4)..(4)
<223>  Thr or absent

<220>
<221>  MISC_FEATURE
<222>  (5)..(5)
<223>  Asn or absent

<220>
<221>  MISC_FEATURE
<222>  (6)..(6)
<223>  Val, Thr, or absent

<220>
<221>  MISC_FEATURE
<222>  (8)..(8)
<223>  Ser or Glu

<220>
<221>  MISC_FEATURE
<222>  (9)..(9)
<223>  Asn, Lys, or Gly

<220>
<221>  MISC_FEATURE
<222>  (10)..(10)
<223>  Thr, Phe, or Ala

<220>
<221>  MISC_FEATURE
<222>  (11)..(11)
<223>  Tyr, Phe, Pro, or absent

<400>  71

Xaa Xaa Xaa Xaa Xaa Xaa Gly Xaa Xaa Xaa Xaa
1               5                   10


<210>  72
<211>  8
<212>  PRT
<213>  Artificial Sequence

<220>
<223>  Synthetic peptide construct


<220>
<221>  MISC_FEATURE
<223>  residues 1 and 8 are independently selected residues having side
       chains which can be chemically bonded to each other to form an
       intramolecular linkage

<220>
<221>  MISC_FEATURE
<222>  (1)..(1)
<223>  variable amino acid

<220>
<221>  MISC_FEATURE
<222>  (2)..(2)
<223>  any amino acid or absent

<220>
<221>  MISC_FEATURE
<222>  (3)..(3)
<223>  Ala, Gly, Ser, Asp or absent

<220>
<221>  MISC_FEATURE
<222>  (4)..(4)
<223>  Asn, Ala, Asp, Gly or absent

<220>
<221>  MISC_FEATURE

<222>  (5)..(5)
<223>  Ala, Leu, Thr, or Ser

<220>
<221>  MISC_FEATURE
<222>  (6)..(6)
<223>  Ala, Ser, or Thr

<220>
<221>  MISC_FEATURE
<222>  (7)..(7)
<223>  Ala, Ser, Val, Hse, Ahb, Ahp, D-Thr, Thr, or a derivative thereof

<220>
<221>  MISC_FEATURE
<222>  (8)..(8)
<223>  variable amino acid

<400>  72

Xaa Xaa Xaa Xaa Xaa Xaa Xaa Xaa
1               5


<210>  73
<211>  28
<212>  PRT
<213>  Artificial Sequence

<220>
<223>  Synthetic peptide construct


<220>
<221>  MISC_FEATURE
<222>  (1)..(4)
<223>  This region may be absent or encompass 1-4 variable amino acids

<220>
<221>  MISC_FEATURE
<222>  (5)..(5)
<223>  Ala or Val

<220>
<221>  MISC_FEATURE
<222>  (6)..(6)
<223>  Thr, Met or Leu

<220>
<221>  MISC_FEATURE
<222>  (7)..(7)
<223>  Gln, Gly, or His

<220>
<221>  MISC_FEATURE
<222>  (9)..(9)
<223>  Leu or Thr

<220>
<221>  MISC_FEATURE
<222>  (10)..(10)
<223>  Ala, Thr, Asn, Phe, Tyr, Ser, or Thr

<220>
<221>  MISC_FEATURE
<222>  (11)..(11)
<223>  Asn, Arg, Ala, Asp, Glu, Gln, Thr, or Gly

<220>
<221> MISC_FEATURE
<222> (12)..(12)
<223> Phe, Leu, Ser, Glu, Ala, Asp, or Tyr

<220>
<221> MISC_FEATURE
<222> (13)..(13)
<223> Leu or Asp

<220>
<221> MISC_FEATURE
<222> (14)..(14)
<223> Val, His, Ser, Phe, or Aib

<220>
<221> MISC_FEATURE
<222> (15)..(15)
<223> His, Arg, Lys, Orn, hArg, Cit, hLys, Lys(for), or Lys(PEG5000)

<220>
<221> MISC_FEATURE
<222> (16)..(16)
<223> Leu, Ser or Phe

<220>
<221> MISC_FEATURE
<222> (17)..(17)
<223> Gln or His

<220>
<221> MISC_FEATURE
<222> (18)..(18)
<223> Thr or Asn

<220>
<221> MISC_FEATURE
<222> (19)..(19)
<223> Tyr, Val, Phe, Leu or Met

<220>
<221> MISC_FEATURE
<222> (21)..(21)
<223> Arg or Pro

<220>
<221> MISC_FEATURE
<222> (25)..(28)
<223> This region may be absent or encompass 1-4 variable amino acids

<400> 73

Xaa Xaa Xaa Xaa Xaa Xaa Xaa Arg Xaa Xaa Xaa Xaa Xaa Xaa Xaa Xaa
1               5                   10                  15

Xaa Xaa Xaa Pro Xaa Thr Asn Thr Xaa Xaa Xaa Xaa
            20                  25

<210> 74
<211> 11
<212> PRT
<213> Artificial Sequence

<220>
<223> Synthetic peptide construct

<220>
<221>  MISC_FEATURE
<222>  (1)..(1)
<223>  Lys, Tyr, or absent

<220>
<221>  MISC_FEATURE
<222>  (2)..(2)
<223>  Ser, Pro, or absent

<220>
<221>  MISC_FEATURE
<222>  (3)..(3)
<223>  Ser, Pro, Arg, or absent

<220>
<221>  MISC_FEATURE
<222>  (4)..(4)
<223>  Thr or absent

<220>
<221>  MISC_FEATURE
<222>  (5)..(5)
<223>  Asn or absent

<220>
<221>  MISC_FEATURE
<222>  (6)..(6)
<223>  Val, Thr, or absent

<220>
<221>  MISC_FEATURE
<222>  (8)..(8)
<223>  Ser or Glu

<220>
<221>  MISC_FEATURE
<222>  (9)..(9)
<223>  Asn, Lys, or Gly

<220>
<221>  MISC_FEATURE
<222>  (10)..(10)
<223>  Thr, Phe, or Ala

<220>
<221>  MISC_FEATURE
<222>  (11)..(11)
<223>  Tyr, Phe, Pro, or absent

<400>  74

Xaa Xaa Xaa Xaa Xaa Xaa Gly Xaa Xaa Xaa Xaa
1               5                   10


<210>  75
<211>  32
<212>  PRT
<213>  Artificial Sequence

<220>
<223>  Synthetic peptide construct

<400>  75

Lys Cys Asn Thr Ala Thr Cys Val Leu Gly Lys Leu Ser Gln Glu Leu

1               5                   10                  15

His Arg Leu Gln Thr Tyr Pro Arg Thr Asn Thr Gly Ser Asn Thr Tyr
            20                  25                  30

<210>  76
<211>  32
<212>  PRT
<213>  Artificial Sequence

<220>
<223>  Synthetic peptide construct

<400>  76

Lys Cys Asn Thr Ala Thr Cys Val Leu Gly Arg Leu Ser Gln Glu Leu
1               5                   10                  15

His Arg Leu Gln Thr Leu Pro Arg Thr Asn Thr Gly Ser Asn Thr Tyr
            20                  25                  30

<210>  77
<211>  32
<212>  PRT
<213>  Artificial Sequence

<220>
<223>  Synthetic peptide construct

<400>  77

Lys Cys Asn Thr Ala Thr Cys Val Leu Gly Arg Leu Ser Gln Glu Leu
1               5                   10                  15

His Arg Leu Gln Thr Tyr Pro Pro Thr Asn Thr Gly Ser Asn Thr Tyr
            20                  25                  30

<210>  78
<211>  32
<212>  PRT
<213>  Artificial Sequence

<220>
<223>  Synthetic peptide construct

<400>  78

Lys Cys Asn Thr Ala Thr Cys Val Leu Gly Arg Leu Ser Gln Glu Leu
1               5                   10                  15

His Arg Leu Gln Thr Tyr Pro Arg Thr Asn Val Gly Ser Asn Thr Tyr
            20                  25                  30

<210>  79
<211>  32
<212>  PRT
<213>  Artificial Sequence

<220>
<223>  Synthetic peptide construct

<400>  79

Lys Cys Asn Thr Ala Thr Cys Val Leu Gly Arg Leu Ser Gln Glu Leu
1               5                   10                  15

His Arg Leu Gln Thr Leu Pro Pro Thr Asn Val Gly Ser Asn Thr Tyr
            20                  25                  30

<210>  80
<211>  32
<212>  PRT
<213>  Artificial Sequence

<220>
<223>  Synthetic peptide construct

<400>  80

Lys Cys Asn Thr Ala Thr Cys Val Leu Gly Arg Leu Ala Asn Phe Leu
1               5                   10                  15

His Arg Leu Gln Thr Tyr Pro Arg Thr Asn Thr Gly Ser Asn Thr Tyr
            20                  25                  30

<210>  81
<211>  32
<212>  PRT
<213>  Artificial Sequence

<220>
<223>  Synthetic peptide construct

<400>  81

Ala Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  82
<211>  32
<212>  PRT
<213>  Artificial Sequence

<220>
<223>  Synthetic peptide construct

<400>  82

Lys Cys Asn Ala Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  83
<211>  32
<212>  PRT

<213>  Artificial Sequence

<220>
<223>  Synthetic peptide construct

<400>  83

Lys Cys Asn Thr Ala Ala Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  84
<211>  32
<212>  PRT
<213>  Artificial Sequence

<220>
<223>  Synthetic peptide construct

<400>  84

Cys Ala Asn Leu Ser Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  85
<211>  28
<212>  PRT
<213>  Artificial Sequence

<220>
<223>  Synthetic peptide construct

<220>
<221>  MOD_RES
<222>  (1)..(1)
<223>  Isocaproyl-Ser

<400>  85

Ser Thr Ala Val Leu Gly Arg Leu Ser Gln Glu Leu His Arg Leu Gln
1               5                   10                  15

Thr Tyr Pro Arg Thr Asn Thr Gly Ser Asn Thr Tyr
            20                  25

<210>  86
<211>  32
<212>  PRT
<213>  Artificial Sequence

<220>
<223>  Synthetic peptide construct

<400>  86

Cys Ser Asn Ala Ser Thr Cys Val Leu Gly Arg Leu Ser Gln Glu Leu

1               5                   10                  15

His Arg Leu Gln Thr Tyr Pro Arg Thr Asn Thr Gly Ser Asn Thr Tyr
            20                  25                  30

<210>  87
<211>  32
<212>  PRT
<213>  Artificial Sequence

<220>
<223>  Synthetic peptide construct

<400>  87

Cys Ser Asn Leu Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  88
<211>  32
<212>  PRT
<213>  Artificial Sequence

<220>
<223>  Synthetic peptide construct

<400>  88

Cys Ser Asn Leu Ser Ala Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  89
<211>  32
<212>  PRT
<213>  Artificial Sequence

<220>
<223>  Synthetic peptide construct

<400>  89

Lys Cys Asn Thr Ala Thr Cys Val Leu Gly Arg Leu Ser Gln Glu Leu
1               5                   10                  15

His Lys Leu Gln Thr Tyr Pro Arg Thr Asn Thr Gly Ser Asn Thr Tyr
            20                  25                  30

<210>  90
<211>  32
<212>  PRT
<213>  Artificial Sequence

<220>
<223>  Synthetic peptide construct

<400>  90

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Gly Thr Pro
            20                  25                  30

<210>  91
<211>  32
<212>  PRT
<213>  Artificial Sequence

<220>
<223>  Synthetic peptide construct

<400>  91

Cys Ser Ala Leu Ser Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  92
<211>  32
<212>  PRT
<213>  Artificial Sequence

<220>
<223>  Synthetic peptide construct

<220>
<221>  MOD_RES
<222>  (1)..(1)
<223>  Ac-Agy

<220>
<221>  MOD_RES
<222>  (7)..(7)
<223>  Agy

<400>  92

Xaa Ser Asn Leu Ser Thr Xaa Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  93
<211>  27
<212>  PRT
<213>  Artificial Sequence

<220>
<223>  Synthetic peptide construct

<220>

<221>  MOD_RES
<222>  (1)..(1)
<223>  Isocaproyl-Ser

<220>
<221>  MOD_RES
<222>  (6)..(6)
<223>  Aib

<400>  93

Ser Thr Ala Val Leu Xaa Arg Leu Ser Gln Glu Leu Arg Leu Gln Thr
1               5                   10                  15

Tyr Pro Arg Thr Asn Thr Gly Ser Gly Thr Pro
            20                  25

<210>  94
<211>  28
<212>  PRT
<213>  Artificial Sequence

<220>
<223>  Synthetic peptide construct

<220>
<221>  MOD_RES
<222>  (1)..(1)
<223>  Isocaproyl-Ser

<220>
<221>  MOD_RES
<222>  (7)..(7)
<223>  Lys(For)

<220>
<221>  MOD_RES
<222>  (14)..(14)
<223>  Lys(For)

<400>  94

Ser Thr Ala Val Leu Gly Lys Leu Ser Gln Glu Leu His Lys Leu Gln
1               5                   10                  15

Thr Tyr Pro Arg Thr Asn Thr Gly Ser Gly Thr Pro
            20                  25

<210>  95
<211>  28
<212>  PRT
<213>  Artificial Sequence

<220>
<223>  Synthetic peptide construct

<220>
<221>  MOD_RES
<222>  (1)..(1)
<223>  Isocaproyl-Ser

<220>

<221>  MOD_RES
<222>  (6)..(6)
<223>  Aib

<220>
<221>  MOD_RES
<222>  (7)..(7)
<223>  Lys(For)

<220>
<221>  MOD_RES
<222>  (13)..(13)
<223>  Aib

<220>
<221>  MOD_RES
<222>  (14)..(14)
<223>  Lys(For)

<400>  95

Ser Thr Ala Val Leu Xaa Lys Leu Ser Gln Glu Leu Xaa Lys Leu Gln
1               5                   10                  15

Thr Tyr Pro Arg Thr Asn Thr Gly Ser Asn Thr Tyr
            20                  25

<210>  96
<211>  28
<212>  PRT
<213>  Artificial Sequence

<220>
<223>  Synthetic peptide construct

<220>
<221>  MOD_RES
<222>  (1)..(1)
<223>  Isocaproyl-Ser

<220>
<221>  MOD_RES
<222>  (6)..(6)
<223>  Aib

<220>
<221>  MOD_RES
<222>  (7)..(7)
<223>  Lys(For)

<220>
<221>  MOD_RES
<222>  (13)..(13)
<223>  Aib

<220>
<221>  MOD_RES
<222>  (14)..(14)
<223>  Lys(For)

<400>  96

Ser Thr Ala Val Leu Xaa Lys Leu Ser Gln Glu Leu Xaa Lys Leu Gln
1               5                   10                  15

Thr Tyr Pro Arg Thr Asn Val Gly Ser Asn Thr Tyr
            20                  25

<210>  97
<211>  32
<212>  PRT
<213>  Artificial Sequence

<220>
<223>  Synthetic peptide construct

<400>  97

Lys Cys Asn Thr Ala Thr Cys Leu Leu Gln Gln Leu Gln Lys Leu Leu
1               5                   10                  15

Gln Lys Leu Lys Gln Tyr Pro Arg Thr Asn Thr Gly Ser Asn Thr Tyr
            20                  25                  30

<210>  98
<211>  32
<212>  PRT
<213>  Artificial Sequence

<220>
<223>  Synthetic peptide construct

<400>  98

Lys Cys Asn Thr Ala Ser Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  99
<211>  32
<212>  PRT
<213>  Artificial Sequence

<220>
<223>  Synthetic peptide construct

<400>  99

Lys Cys Asn Thr Ala Val Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  100
<211>  29
<212>  PRT
<213>  Artificial Sequence

<220>
<223>  Synthetic peptide construct

<400>  100

Lys Cys Asn Thr Ala Thr Cys Val Leu Gly Arg Leu Ser Gln Glu Leu
1               5                   10                  15

His Arg Tyr Pro Arg Thr Asn Thr Gly Ser Asn Thr Tyr
            20                  25

<210>  101
<211>  32
<212>  PRT
<213>  Artificial Sequence

<220>
<223>  Synthetic peptide construct

<220>
<221>  MOD_RES
<222>  (11)..(11)
<223>  Lys(For)

<220>
<221>  MOD_RES
<222>  (18)..(18)
<223>  Lys(For)

<400>  101

Lys Cys Asn Thr Ala Thr Cys Val Leu Gly Lys Leu Ser Gln Glu Leu
1               5                   10                  15

His Lys Leu Gln Thr Tyr Pro Arg Thr Asn Thr Gly Ser Asn Thr Tyr
            20                  25                  30

<210>  102
<211>  32
<212>  PRT
<213>  Artificial Sequence

<220>
<223>  Synthetic peptide construct

<220>
<221>  MISC_FEATURE
<222>  (6)..(6)
<223>  D-Thr

<400>  102

Lys Cys Asn Thr Ala Xaa Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  103
<211>  32
<212>  PRT
<213>  Artificial Sequence

<220>

<223>  Synthetic peptide construct

<220>
<221>  MISC_FEATURE
<222>  (6)..(6)
<223>  dAh

<400>  103

Lys Cys Asn Thr Ala Xaa Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  104
<211>  32
<212>  PRT
<213>  Artificial Sequence

<220>
<223>  Synthetic peptide construct

<220>
<221>  MOD_RES
<222>  (1)..(1)
<223>  Ac-Ala

<220>
<221>  MOD_RES
<222>  (18)..(18)
<223>  Lys(PEG5000)

<400>  104

Ala Cys Asn Thr Ala Thr Cys Val Leu Gly Arg Leu Ser Gln Glu Leu
1               5                   10                  15

His Lys Leu Gln Thr Tyr Pro Arg Thr Asn Thr Gly Ser Asn Thr Tyr
            20                  25                  30

<210>  105
<211>  35
<212>  PRT
<213>  Artificial Sequence

<220>
<223>  Synthetic peptide construct

<400>  105

Lys Cys Asn Thr Ala Thr Cys Val Leu Gly Arg Leu Ser Gln Glu Leu
1               5                   10                  15

His Arg Leu Gln Thr Leu Gln Thr Tyr Pro Arg Thr Asn Thr Gly Ser
            20                  25                  30

Asn Thr Tyr
        35

<210>  106
<211>  36
<212>  PRT
<213>  Artificial Sequence

<220>
<223>  Synthetic peptide construct

<400>  106

Lys Cys Asn Thr Ala Thr Cys Val Leu Gly Arg Leu Ser Gln Glu Leu
1               5                   10                  15

His Arg Leu Gln Thr Leu Leu Gln Thr Tyr Pro Arg Thr Asn Thr Gly
            20                  25                  30

Ser Asn Thr Tyr
        35

<210>  107
<211>  32
<212>  PRT
<213>  Artificial Sequence

<220>
<223>  Synthetic peptide construct

<400>  107

Lys Cys Asn Thr Ala Thr Cys Val Leu Gly Lys Leu Ser Gln Glu Leu
1               5                   10                  15

His Lys Leu Gln Thr Tyr Pro Arg Thr Asn Thr Gly Ser Asn Thr Tyr
            20                  25                  30

<210>  108
<211>  32
<212>  PRT
<213>  Artificial Sequence

<220>
<223>  Synthetic peptide construct

<400>  108

Lys Cys Asn Thr Ser Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  109
<211>  32
<212>  PRT
<213>  Artificial Sequence

<220>
<223>  Synthetic peptide construct

<400>  109

Lys Cys Asn Thr Ala Thr Cys Ala Thr Gln Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  110
<211>  32
<212>  PRT
<213>  Artificial Sequence

<220>
<223>  Synthetic peptide construct

<400>  110

Lys Cys Asn Thr Ala Thr Cys Ala Thr Gln Arg Leu Ser Gln Glu Leu
1               5                   10                  15

His Arg Leu Gln Thr Tyr Pro Arg Thr Asn Val Gly Ser Asn Thr Tyr
            20                  25                  30

<210>  111
<211>  32
<212>  PRT
<213>  Artificial Sequence

<220>
<223>  Synthetic peptide construct

<400>  111

Lys Cys Asn Thr Ser Thr Cys Ala Thr Gln Arg Leu Ala Asn Glu Leu
1               5                   10                  15

Val Arg Leu Gln Thr Tyr Pro Arg Thr Asn Val Gly Ser Asn Thr Tyr
            20                  25                  30

<210>  112
<211>  32
<212>  PRT
<213>  Artificial Sequence

<220>
<223>  Synthetic peptide construct

<220>
<221>  MISC_FEATURE
<222>  (6)..(6)
<223>  Hse

<400>  112

Lys Cys Asn Thr Ala Xaa Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  113
<211>  32
<212>  PRT
<213>  Artificial Sequence

<220>
<223>  Synthetic peptide construct


<220>
<221>  MISC_FEATURE
<222>  (6)..(6)
<223>  Ahb

<400>  113

Lys Cys Asn Thr Ala Xaa Cys Val Leu Gly Arg Leu Ser Gln Glu Leu
1               5                   10                  15


His Arg Leu Gln Thr Tyr Pro Arg Thr Asn Thr Gly Ser Asn Thr Tyr
            20                  25                  30


<210>  114
<211>  32
<212>  PRT
<213>  Artificial Sequence

<220>
<223>  Synthetic peptide construct


<220>
<221>  MISC_FEATURE
<222>  (6)..(6)
<223>  Ahp

<400>  114

Lys Cys Asn Thr Ala Xaa Cys Val Leu Gly Arg Leu Ser Gln Glu Leu
1               5                   10                  15


His Arg Leu Gln Thr Tyr Pro Arg Thr Asn Thr Gly Ser Asn Thr Tyr
            20                  25                  30


<210>  115
<211>  32
<212>  PRT
<213>  Artificial Sequence

<220>
<223>  Synthetic peptide construct


<220>
<221>  MISC_FEATURE
<222>  (6)..(6)
<223>  Thr(OPO3H2)

<400>  115

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  116
<211>  32
<212>  PRT
<213>  Artificial Sequence

<220>
<223>  Synthetic peptide construct

<220>
<221>  MOD_RES
<222>  (11)..(11)
<223>  Orn

<220>
<221>  MOD_RES
<222>  (18)..(18)
<223>  Orn

<400>  116

Lys Cys Asn Thr Ala Thr Cys Val Leu Gly Xaa Leu Ser Gln Glu Leu
1               5                   10                  15

His Xaa Leu Gln Thr Tyr Pro Arg Thr Asn Thr Gly Ser Asn Thr Tyr
            20                  25                  30

<210>  117
<211>  32
<212>  PRT
<213>  Artificial Sequence

<220>
<223>  Synthetic peptide construct

<220>
<221>  MOD_RES
<222>  (11)..(11)
<223>  Cit

<220>
<221>  MOD_RES
<222>  (18)..(18)
<223>  Cit

<400>  117

Lys Cys Asn Thr Ala Thr Cys Val Leu Gly Xaa Leu Ser Gln Glu Leu
1               5                   10                  15

His Xaa Leu Gln Thr Tyr Pro Arg Thr Asn Thr Gly Ser Asn Thr Tyr
            20                  25                  30

<210>  118
<211>  32
<212>  PRT
<213>  Artificial Sequence

<220>
<223>  Synthetic peptide construct


<220>
<221>  MISC_FEATURE
<222>  (11)..(11)
<223>  homoLys

<220>
<221>  MISC_FEATURE
<222>  (18)..(18)
<223>  homoLys

<400>  118

Lys Cys Asn Thr Ala Thr Cys Val Leu Gly Xaa Leu Ser Gln Glu Leu
1               5                   10                  15


His Xaa Leu Gln Thr Tyr Pro Arg Thr Asn Thr Gly Ser Asn Thr Tyr
            20                  25                  30


<210>  119
<211>  33
<212>  PRT
<213>  Artificial Sequence

<220>
<223>  Synthetic peptide construct


<220>
<221>  MOD_RES
<222>  (1)..(1)
<223>  L-Octylglycine

<400>  119

Xaa Lys Cys Asn Thr Ala Thr Cys Val Leu Gly Arg Leu Ser Gln Glu
1               5                   10                  15


Leu His Arg Leu Gln Thr Tyr Pro Arg Thr Asn Thr Gly Ser Asn Thr
            20                  25                  30


Tyr



<210>  120
<211>  31
<212>  PRT
<213>  Artificial Sequence

<220>
<223>  Synthetic peptide construct


<220>
<221>  MOD_RES
<222>  (1)..(1)
<223>  N-3,6-dioxaoctanoyl-Cys

<400>  120

Cys Asn Thr Ala Thr Cys Val Leu Gly Arg Leu Ser Gln Glu Leu His
1               5                   10                  15

Arg Leu Gln Thr Val Pro Arg Thr Asn Thr Gly Ser Asn Thr Tyr
            20                  25                  30

<210>  121
<211>  32
<212>  PRT
<213>  Artificial Sequence

<220>
<223>  Synthetic peptide construct

<400>  121

Lys Cys Asn Thr Ala Thr Cys Met Leu Gly Arg Tyr Thr Gln Asp Phe
1               5                   10                  15

His Arg Leu Gln Thr Tyr Pro Arg Thr Asn Thr Gly Ser Asn Thr Tyr
            20                  25                  30

<210>  122
<211>  32
<212>  PRT
<213>  Artificial Sequence

<220>
<223>  Synthetic peptide construct

<400>  122

Asp Ser Asn Leu Ser Thr Lys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  123
<211>  32
<212>  PRT
<213>  Artificial Sequence

<220>
<223>  Synthetic peptide construct

<400>  123

Lys Asp Asn Thr Ala Thr Lys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  124
<211>  31
<212>  PRT
<213>  Artificial Sequence

<220>

<223>  Synthetic peptide construct

<400>  124

Cys Asn Thr Ala Thr Cys Val Leu Gly Arg Leu Ser Gln Glu Leu His
1               5                   10                  15

Arg Leu Gln Thr Tyr Pro Arg Thr Asn Thr Gly Ser Asn Thr Tyr
            20                  25                  30

<210>  125
<211>  33
<212>  PRT
<213>  Artificial Sequence

<220>
<223>  Synthetic peptide construct

<220>
<221>  MOD_RES
<222>  (33)..(33)
<223>  9Anc

<400>  125

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

Xaa

<210>  126
<211>  33
<212>  PRT
<213>  Artificial Sequence

<220>
<223>  Synthetic peptide construct

<220>
<221>  MOD_RES
<222>  (33)..(33)
<223>  L-octylglycine

<400>  126

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

Xaa

<210>  127
<211>  32
<212>  PRT
<213>  Artificial Sequence

<220>
<223>  Synthetic peptide construct


<220>
<221>  MOD_RES
<222>  (1)..(1)
<223>  N-isocaproyl-Lys

<400>  127

Lys Cys Asn Thr Ala Thr Cys Val Leu Gly Arg Leu Ser Gln Glu Leu
1               5                   10                  15


His Arg Leu Gln Thr Tyr Pro Arg Thr Asn Thr Gly Ser Asn Thr Tyr
            20                  25                  30


<210>  128
<211>  32
<212>  PRT
<213>  Artificial Sequence

<220>
<223>  Synthetic peptide construct


<220>
<221>  MOD_RES
<222>  (11)..(11)
<223>  homo-Arg

<220>
<221>  MOD_RES
<222>  (18)..(18)
<223>  homo-Arg

<400>  128

Lys Cys Asn Thr Ala Thr Cys Val Leu Gly Xaa Leu Ser Gln Glu Leu
1               5                   10                  15


His Xaa Leu Gln Thr Tyr Pro Arg Thr Asn Thr Gly Ser Asn Thr Tyr
            20                  25                  30


<210>  129
<211>  32
<212>  PRT
<213>  Artificial Sequence

<220>
<223>  Synthetic peptide construct

<400>  129

Phe Cys Asn Thr Ala Thr Cys Val Leu Gly Arg Leu Ser Gln Glu Leu
1               5                   10                  15


His Arg Leu Gln Thr Tyr Pro Arg Thr Asn Thr Gly Ser Asn Thr Tyr

20                  25                  30

<210>  130
<211>  32
<212>  PRT
<213>  Artificial Sequence

<220>
<223>  Synthetic peptide construct


<220>
<221>  MISC_FEATURE
<222>  (18)..(18)
<223>  Cit

<400>  130

Lys Cys Asn Thr Ala Thr Cys Val Leu Gly Arg Leu Ser Gln Glu Leu
1               5                   10                  15


His Xaa Leu Gln Thr Tyr Pro Arg Thr Asn Thr Gly Ser Asn Thr Tyr
            20                  25                  30


<210>  131
<211>  32
<212>  PRT
<213>  Artificial Sequence

<220>
<223>  Synthetic peptide construct


<220>
<221>  MOD_RES
<222>  (18)..(18)
<223>  Orn

<400>  131

Lys Cys Asn Thr Ala Thr Cys Val Leu Gly Arg Leu Ser Gln Glu Leu
1               5                   10                  15


His Xaa Leu Gln Thr Tyr Pro Arg Thr Asn Thr Gly Ser Asn Thr Tyr
            20                  25                  30


<210>  132
<211>  32
<212>  PRT
<213>  Artificial Sequence

<220>
<223>  Synthetic peptide construct

<400>  132

Ile Cys Asn Thr Ala Thr Cys Val Leu Gly Arg Leu Ser Gln Glu Leu
1               5                   10                  15


His Arg Leu Gln Thr Tyr Pro Arg Thr Asn Thr Gly Ser Asn Thr Tyr
            20                  25                  30

<210>  133
<211>  32
<212>  PRT
<213>  Artificial Sequence

<220>
<223>  Synthetic peptide construct


<220>
<221>  MOD_RES
<222>  (1)..(1)
<223>  L-Octylglycine

<400>  133

Xaa Cys Asn Thr Ala Thr Cys Val Leu Gly Arg Leu Ser Gln Glu Leu
1               5                   10                  15


His Arg Leu Gln Thr Tyr Pro Arg Thr Asn Thr Gly Ser Asn Thr Tyr
            20                  25                  30


<210>  134
<211>  31
<212>  PRT
<213>  Artificial Sequence

<220>
<223>  Synthetic peptide construct


<220>
<221>  MOD_RES
<222>  (1)..(1)
<223>  Isocaproyl-Cys

<400>  134

Cys Asn Thr Ala Thr Cys Val Leu Gly Arg Leu Ser Gln Glu Leu His
1               5                   10                  15


Arg Leu Gln Thr Tyr Pro Arg Thr Asn Thr Gly Ser Asn Thr Tyr
            20                  25                  30


<210>  135
<211>  32
<212>  PRT
<213>  Artificial Sequence

<220>
<223>  Synthetic peptide construct


<220>
<221>  MOD_RES
<222>  (11)..(11)
<223>  Cit

<400>  135

Lys Cys Asn Thr Ala Thr Cys Val Leu Gly Xaa Leu Ser Gln Glu Leu
1               5                   10                  15

His Arg Leu Gln Thr Tyr Pro Arg Thr Asn Thr Gly Ser Asn Thr Tyr
            20                  25                  30

<210>  136
<211>  33
<212>  PRT
<213>  Artificial Sequence

<220>
<223>  Synthetic peptide construct

<220>
<221>  MOD_RES
<222>  (33)..(33)
<223>  4ABU

<400>  136

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

Xaa

<210>  137
<211>  33
<212>  PRT
<213>  Artificial Sequence

<220>
<223>  Synthetic peptide construct

<220>
<221>  MOD_RES
<222>  (1)..(1)
<223>  Isocaproyl-Lys

<220>
<221>  MOD_RES
<222>  (33)..(33)
<223>  4ABU

<400>  137

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

Xaa

<210>  138
<211>  32

<212>  PRT
<213>  Artificial Sequence

<220>
<223>  Synthetic peptide construct

<400>  138

Lys Cys Asn Thr Ser Thr Cys Ala Thr Gln Arg Leu Ala Asn Glu Leu
1               5                   10                  15

Val Arg Leu Gln Thr Tyr Pro Arg Thr Asn Val Gly Ser Glu Ala Phe
            20                  25                  30

<210>  139
<211>  31
<212>  PRT
<213>  Artificial Sequence

<220>
<223>  Synthetic peptide construct

<400>  139

Lys Cys Asn Thr Ala Thr Cys Val Leu Gly Arg Leu Ser Gln Glu Leu
1               5                   10                  15

His Arg Leu Gln Thr Tyr Pro Thr Asn Val Gly Ser Glu Ala Phe
            20                  25                  30

<210>  140
<211>  32
<212>  PRT
<213>  Artificial Sequence

<220>
<223>  Synthetic peptide construct

<400>  140

Lys Cys Asn Thr Ala Thr Cys Val Leu Gly Arg Leu Ser Arg Ser Leu
1               5                   10                  15

His Arg Leu Gln Thr Tyr Pro Arg Thr Asn Thr Gly Ser Asn Thr Tyr
            20                  25                  30

<210>  141
<211>  32
<212>  PRT
<213>  Artificial Sequence

<220>
<223>  Synthetic peptide construct

<400>  141

Lys Cys Asn Thr Ala Thr Cys Val Thr His Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  142
<211>  32
<212>  PRT
<213>  Artificial Sequence

<220>
<223>  Synthetic peptide construct

<400>  142

Lys Cys Asn Thr Ala Thr Cys Val Leu Gly Arg Leu Ala Asp Phe Leu
1               5                   10                  15

His Arg Leu Gln Thr Tyr Pro Arg Thr Asn Thr Gly Ser Asn Thr Tyr
            20                  25                  30

<210>  143
<211>  30
<212>  PRT
<213>  Artificial Sequence

<220>
<223>  Synthetic peptide construct

<400>  143

Cys Asn Thr Ala Thr Cys Val Leu Gly Arg Leu Ser Gln Glu Leu His
1               5                   10                  15

Arg Leu Gln Thr Tyr Pro Arg Thr Asn Thr Gly Ser Asn Thr
            20                  25                  30

<210>  144
<211>  33
<212>  PRT
<213>  Artificial Sequence

<220>
<223>  Synthetic peptide construct

<400>  144

Lys Cys Asn Thr Ala Thr Cys Val Leu Gly Arg Leu Ser Gln Glu Leu
1               5                   10                  15

His Arg Leu Gln Asn Phe Val Pro Arg Thr Asn Thr Gly Ser Asn Thr
            20                  25                  30

Tyr

<210>  145
<211>  32
<212>  PRT
<213>  Artificial Sequence

<220>
<223>  Synthetic peptide construct

<400>  145

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Glu Thr Phe
            20                  25                  30

<210>  146
<211>  32
<212>  PRT
<213>  Artificial Sequence

<220>
<223>  Synthetic peptide construct

<400>  146

Ala Cys Asp Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  147
<211>  32
<212>  PRT
<213>  Artificial Sequence

<220>
<223>  Synthetic peptide construct

<400>  147

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Lys Ala Phe
            20                  25                  30

<210>  148
<211>  32
<212>  PRT
<213>  Artificial Sequence

<220>
<223>  Synthetic peptide construct

<400>  148

Lys Cys Asp Thr Ala Thr Cys Val Thr His Arg Leu Ala Gly Leu Leu
1               5                   10                  15

Ser Arg Ser Gln Thr Tyr Pro Arg Thr Asn Thr Gly Ser Asn Thr Tyr
            20                  25                  30

<210>  149
<211>  32
<212>  PRT
<213>  Artificial Sequence

<220>
<223>  Synthetic peptide construct

<400>  149

Lys Cys Asn Thr Ala Thr Cys Val Leu Gly Arg Leu Ala Asp Ala Leu
1               5                   10                  15

His Arg Leu Gln Thr Tyr Pro Arg Thr Asn Thr Gly Ser Asn Thr Tyr
            20                  25                  30

<210>  150
<211>  32
<212>  PRT
<213>  Artificial Sequence

<220>
<223>  Synthetic peptide construct

<400>  150

Lys Cys Asn Thr Ala Thr Cys Val Leu Gly Arg Leu Ala Ala Phe Leu
1               5                   10                  15

His Arg Leu Gln Thr Tyr Pro Arg Thr Asn Thr Gly Ser Asn Thr Tyr
            20                  25                  30

<210>  151
<211>  32
<212>  PRT
<213>  Artificial Sequence

<220>
<223>  Synthetic peptide construct

<400>  151

Ser Cys Asn Thr Ala Thr Cys Val Leu Gly Arg Leu Ala Asp Phe Leu
1               5                   10                  15

His Arg Leu Gln Thr Tyr Pro Arg Thr Asn Thr Gly Ser Asn Thr Tyr
            20                  25                  30

<210>  152
<211>  32
<212>  PRT
<213>  Artificial Sequence

<220>
<223>  Synthetic peptide construct

<400>  152

Lys Cys Asn Thr Ala Thr Cys Val Leu Gly Arg Leu Ser Gln Glu Leu
1               5                   10                  15

His Arg Leu Gln Thr Met Pro Arg Thr Asn Thr Gly Ser Asn Thr Tyr
            20                  25                  30

<210>  153
<211>  32
<212>  PRT
<213>  Artificial Sequence

<220>
<223>  Synthetic peptide construct

<400>  153

Lys Cys Asn Thr Ala Thr Cys Val Leu Gly Arg Leu Ser Gln Glu Leu
1               5                   10                  15

His Arg Leu Gln Thr Val Pro Arg Thr Asn Thr Gly Ser Asn Thr Tyr
            20                  25                  30

<210>  154
<211>  32
<212>  PRT
<213>  Artificial Sequence

<220>
<223>  Synthetic peptide construct

<400>  154

Lys Cys Asn Thr Ala Thr Cys Val Leu Gly Arg Leu Asn Glu Tyr Leu
1               5                   10                  15

His Arg Leu Gln Thr Tyr Pro Arg Thr Asn Thr Gly Ser Asn Thr Tyr
            20                  25                  30

<210>  155
<211>  32
<212>  PRT
<213>  Artificial Sequence

<220>
<223>  Synthetic peptide construct

<400>  155

Ser Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  156
<211>  32
<212>  PRT
<213>  Artificial Sequence

<220>
<223>  Synthetic peptide construct

<400>  156

Lys Cys Asn Thr Ala Thr Cys Val Leu Gly Arg Leu Thr Glu Phe Leu
1               5                   10                  15

His Arg Leu Gln Thr Tyr Pro Arg Thr Asn Thr Gly Ser Asn Thr Tyr
            20                  25                  30

<210>  157
<211>  32
<212>  PRT
<213>  Artificial Sequence

<220>
<223>  Synthetic peptide construct

<400>  157

Lys Cys Asn Thr Ala Thr Cys Val Leu Gly Arg Leu Ala Glu Phe Leu
1               5                   10                  15

His Arg Leu Gln Thr Tyr Pro Arg Thr Asn Thr Gly Ser Asn Thr Tyr
            20                  25                  30

<210>  158
<211>  32
<212>  PRT
<213>  Artificial Sequence

<220>
<223>  Synthetic peptide construct

<400>  158

Lys Cys Asn Thr Ala Thr Cys Val Leu Gly Arg Leu Thr Asp Tyr Leu
1               5                   10                  15

His Arg Leu Gln Thr Tyr Pro Arg Thr Asn Thr Gly Ser Asn Thr Tyr
            20                  25                  30

<210>  159
<211>  32
<212>  PRT
<213>  Artificial Sequence

<220>
<223>  Synthetic peptide construct

<400>  159

Lys Cys Asn Thr Ala Thr Cys Val Leu Gly Arg Leu Ala Gln Phe Leu
1               5                   10                  15

His Arg Leu Gln Thr Tyr Pro Arg Thr Asn Thr Gly Ser Asn Thr Tyr
            20                  25                  30

<210>  160
<211>  32
<212>  PRT
<213>  Artificial Sequence

<220>
<223>  Synthetic peptide construct

<400>  160

Lys Cys Asn Thr Ala Thr Cys Val Leu Gly Arg Leu Ala Asp Phe Leu
1               5                   10                  15

His Arg Phe Gln Thr Phe Pro Arg Thr Asn Thr Gly Ser Asn Thr Tyr
            20                  25                  30

<210>  161
<211>  32
<212>  PRT
<213>  Artificial Sequence

<220>
<223>  Synthetic peptide construct

<400>  161

Lys Cys Asn Thr Ala Thr Cys Val Leu Gly Arg Leu Ala Asp Phe Leu
1               5                   10                  15

His Arg Phe His Thr Phe Pro Arg Thr Asn Thr Gly Ser Asn Thr Tyr
            20                  25                  30

<210>  162
<211>  32
<212>  PRT
<213>  Artificial Sequence

<220>
<223>  Synthetic peptide construct

<400>  162

Lys Cys Asn Thr Ala Thr Cys Val Leu Gly Arg Leu Ala Asp Phe Leu
1               5                   10                  15

His Arg Phe Gln Thr Phe Pro Arg Thr Asn Thr Gly Ser Gly Thr Pro
            20                  25                  30

<210>  163
<211>  31
<212>  PRT
<213>  Artificial Sequence

<220>
<223>  Synthetic peptide construct

<400>  163

Cys Asn Thr Ala Thr Cys Val Leu Gly Arg Leu Ala Asp Phe Leu His
1               5                   10                  15

Arg Leu Gln Thr Tyr Pro Arg Thr Asn Thr Gly Ser Asn Thr Tyr
            20                  25                  30

<210>  164
<211>  32
<212>  PRT
<213>  Artificial Sequence

<220>

<223>  Synthetic peptide construct

<400>  164

Lys Cys Asp Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  165
<211>  32
<212>  PRT
<213>  Artificial Sequence

<220>
<223>  Synthetic peptide construct

<400>  165

Lys Cys Asn Thr Ala Thr Cys Val Leu Gly Arg Leu Phe Asp Phe Leu
1               5                   10                  15

His Arg Leu Gln Thr Tyr Pro Arg Thr Asn Thr Gly Ser Asn Thr Tyr
            20                  25                  30

<210>  166
<211>  32
<212>  PRT
<213>  Artificial Sequence

<220>
<223>  Synthetic peptide construct

<400>  166

Lys Cys Asn Thr Ala Thr Cys Val Leu Gly Arg Leu Ala Ala Ala Leu
1               5                   10                  15

His Arg Leu Gln Thr Tyr Pro Arg Thr Asn Thr Gly Ser Asn Thr Tyr
            20                  25                  30

<210>  167
<211>  32
<212>  PRT
<213>  Artificial Sequence

<220>
<223>  Synthetic peptide construct

<400>  167

Thr Cys Asp Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  168
<211>  32

<212>  PRT
<213>  Artificial Sequence

<220>
<223>  Synthetic peptide construct

<400>  168

Cys Ser Asn Leu Ser Thr Cys Ala Thr Gln Arg Leu Ala Asn Glu Leu
1               5                   10                  15

Val Arg Leu Gln Thr Tyr Pro Arg Thr Asn Val Gly Ser Asn Thr Tyr
            20                  25                  30

<210>  169
<211>  32
<212>  PRT
<213>  Artificial Sequence

<220>
<223>  Synthetic peptide construct

<400>  169

Lys Cys Asn Thr Ala Thr Cys Ala Thr Gln Arg Leu Ala Asn Glu Leu
1               5                   10                  15

Val Arg Leu Gln Thr Tyr Pro Arg Thr Asn Val Gly Ser Asn Thr Tyr
            20                  25                  30

<210>  170
<211>  32
<212>  PRT
<213>  Artificial Sequence

<220>
<223>  Synthetic peptide construct

<400>  170

Cys Ser Asn Leu Ser Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  171
<211>  32
<212>  PRT
<213>  Artificial Sequence

<220>
<223>  Synthetic peptide construct

<400>  171

Lys Cys Asn Thr Ala Thr Cys Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

<210>  172
<211>  25
<212>  PRT
<213>  Artificial Sequence

<220>
<223>  Synthetic peptide construct

<400>  172

Val Leu Gly Lys Leu Ser Gln Glu Leu His Lys Leu Gln Thr Tyr Pro
1               5                   10                  15

Arg Thr Asn Thr Gly Ser Gly Thr Pro
            20                  25

<210>  173
<211>  25
<212>  PRT
<213>  Artificial Sequence

<220>
<223>  Synthetic peptide construct

<400>  173

Val Leu Gly Lys Leu Ser Gln Glu Leu His Lys Leu Gln Thr Tyr Pro
1               5                   10                  15

Arg Thr Asn Thr Gly Ser Asn Thr Tyr
            20                  25

<210>  174
<211>  25
<212>  PRT
<213>  Artificial Sequence

<220>
<223>  Synthetic peptide construct

<220>
<221>  MOD_RES
<222>  (1)..(1)
<223>  hydrogen or acetyl group on N-terminal amino acid residue

<220>
<221>  MISC_FEATURE
<222>  (1)..(1)
<223>  Ala or absent

<220>
<221>  MISC_FEATURE
<222>  (4)..(4)
<223>  Arg, Gln, Lys, Asn or Leu

<220>
<221>  MISC_FEATURE
<222>  (8)..(8)
<223>  Gln, Glu, Asn, Asp or Phe

<220>

<221>  MISC_FEATURE
<222>  (22)..(22)
<223>  Ala or Ser

<400>  174

Xaa Thr Gln Xaa Leu Ala Asn Xaa Leu Val Arg Leu Gln Thr Tyr Pro
1               5                   10                  15

Arg Thr Asn Val Gly Xaa Asn Thr Tyr
            20                  25

<210>  175
<211>  25
<212>  PRT
<213>  Artificial Sequence

<220>
<223>  Synthetic peptide construct

<220>
<221>  MOD_RES
<222>  (1)..(1)
<223>  acetyl-Ala

<400>  175

Ala Thr Gln Arg Leu Ala Asn Glu Leu Val Arg Leu Gln Thr Tyr Pro
1               5                   10                  15

Arg Thr Asn Val Gly Ser Asn Thr Tyr
            20                  25

<210>  176
<211>  25
<212>  PRT
<213>  Artificial Sequence

<220>
<223>  Synthetic peptide construct

<400>  176

Ala Thr Gln Gln Leu Ala Asn Gln Leu Val Arg Leu Gln Thr Tyr Pro
1               5                   10                  15

Arg Thr Asn Val Gly Ser Asn Thr Tyr
            20                  25

<210>  177
<211>  25
<212>  PRT
<213>  Artificial Sequence

<220>
<223>  Synthetic peptide construct

<400>  177

Ala Thr Gln Leu Leu Ala Asn Gln Leu Val Arg Leu Gln Thr Tyr Pro
1               5                   10                  15

Arg Thr Asn Val Gly Ser Asn Thr Tyr
            20                  25


<210>  178
<211>  25
<212>  PRT
<213>  Artificial Sequence

<220>
<223>  Synthetic peptide construct

<400>  178

Ala Thr Gln Arg Leu Ala Asn Gln Leu Val Arg Leu Gln Thr Tyr Pro
1               5                   10                  15


Arg Thr Asn Val Gly Ser Asn Thr Tyr
            20                  25


<210>  179
<211>  25
<212>  PRT
<213>  Artificial Sequence

<220>
<223>  Synthetic peptide construct

<400>  179

Ala Thr Gln Leu Leu Ala Asn Glu Leu Val Arg Leu Gln Thr Tyr Pro
1               5                   10                  15


Arg Thr Asn Val Gly Ser Asn Thr Tyr
            20                  25


<210>  180
<211>  25
<212>  PRT
<213>  Artificial Sequence

<220>
<223>  Synthetic peptide construct

<400>  180

Ala Thr Gln Gln Leu Ala Asn Glu Leu Val Arg Leu Gln Thr Tyr Pro
1               5                   10                  15


Arg Thr Asn Val Gly Ser Asn Thr Tyr
            20                  25


<210>  181
<211>  39
<212>  PRT
<213>  Heloderma horridum

<400>  181

His Ser Asp Gly Thr Phe Thr Ser Asp Leu Ser Lys Gln Met Glu Glu

1                   5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  182
<211>  39
<212>  PRT
<213>  Heloderma spectum

<400>  182

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  183
<211>  39
<212>  PRT
<213>  Artificial Sequence

<220>
<223>  Synthetic peptide construct

<400>  183

His Gly Glu Gly Thr Phe Thr Ser Asp Leu Ser Lys Gln Leu Glu Glu
1               5                   10                  15

Glu Ala Val Arg Leu Phe Ile Glu Phe Leu Lys Asn Gly Gly Pro Ser
            20                  25                  30

Ser Gly Ala Pro Pro Pro Ser
        35

<210>  184
<211>  39
<212>  PRT
<213>  Artificial Sequence

<220>
<223>  Synthetic peptide construct

<400>  184

His Gly Glu Gly Ala Phe Thr Ser Asp Leu Ser Lys Gln Leu Glu Glu
1               5                   10                  15

Glu Ala Val Arg Leu Phe Ile Glu Phe Leu Lys Asn Gly Gly Pro Ser
            20                  25                  30

Ser Gly Ala Pro Pro Pro Ser
        35


<210>  185
<211>  39
<212>  PRT
<213>  Artificial Sequence

<220>
<223>  Synthetic peptide construct

<400>  185

His Gly Glu Gly Thr Phe Thr Ser Asp Leu Ser Lys Gln Leu Glu Glu
1               5                   10                  15


Glu Ala Val Arg Leu Ala Ile Glu Phe Leu Lys Asn Gly Gly Pro Ser
            20                  25                  30


Ser Gly Ala Pro Pro Pro Ser
        35


<210>  186
<211>  30
<212>  PRT
<213>  Artificial Sequence

<220>
<223>  Synthetic peptide construct

<400>  186

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly
            20                  25                  30


<210>  187
<211>  28
<212>  PRT
<213>  Artificial Sequence

<220>
<223>  Synthetic peptide construct

<400>  187

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn
            20                  25


<210>  188
<211>  28
<212>  PRT
<213>  Artificial Sequence

<220>
<223>  Synthetic peptide construct

<400>  188

His Gly Glu Gly Thr Phe Thr Ser Asp Leu Ser Lys Gln Leu Glu Glu
1               5                   10                  15

Glu Ala Val Arg Leu Phe Ile Glu Phe Leu Lys Asn
            20                  25

<210>  189
<211>  28
<212>  PRT
<213>  Artificial Sequence

<220>
<223>  Synthetic peptide construct

<400>  189

His Gly Glu Gly Thr Phe Thr Ser Asp Leu Ser Lys Gln Leu Glu Glu
1               5                   10                  15

Glu Ala Val Arg Leu Ala Ile Glu Phe Leu Lys Asn
            20                  25

<210>  190
<211>  39
<212>  PRT
<213>  Artificial Sequence

<220>
<223>  Synthetic peptide construct

<400>  190

His Gly Glu Gly Thr Phe Thr Ser Asp Leu Ser Lys Gln Leu Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  191
<211>  32
<212>  PRT
<213>  Artificial Sequence

<220>
<223>  Synthetic peptide construct

<220>
<221>  MOD_RES
<222>  (1)..(1)
<223>  Ac-Lys

<220>
<221>  MOD_RES
<222>  (2)..(2)
<223>  Agy

<220>
<221>  MOD_RES
<222>  (7)..(7)
<223>  Agy

<400>  191

Lys Xaa Asn Thr Ala Thr Xaa Val Leu Gly Arg Leu Ser Gln Glu Leu
1               5                   10                  15

His Arg Leu Gln Thr Tyr Pro Arg Thr Asn Thr Gly Ser Asn Thr Tyr
            20                  25                  30

## Description

### CROSS-REFERENCE TO RELATED APPLICATIONS

This application claims benefit of U.S. provisional patent application number 60/624,357, filed November 1, 2004, the entire contents of which are incorporated herein for all purposes.

### FIELD OF THE INVENTION

The present invention relates to the medical field and in particular to the field of health, diet and nutrition. The invention relates to the use of anti-obesity agents.

### BACKGROUND

Obesity and its associated disorders are common and very serious public health problems in the United States and throughout the world. Upper body obesity is the strongest risk factor known for type 2 diabetes mellitus and is a strong risk factor for cardiovascular disease. Obesity is a recognized risk factor for hypertension, atherosclerosis, congestive heart failure, stroke, gallbladder disease, osteoarthritis, sleep apnea, reproductive disorders such as polycystic ovarian syndrome, cancers of the breast, prostate, and colon, and increased incidence of complications of general anesthesia (*see, e*.*g*., Kopelman, Nature 404: 635-43 (2000)).

Obesity reduces life-span and carries a serious risk of the co-morbidities listed above, as well disorders such as infections, varicose veins, acanthosis nigricans, eczema, exercise intolerance, insulin resistance, hypertension hypercholesterolemia, cholelithiasis, orthopedic injury, and thromboembolic disease (Rissanen et al., Br. Med. J. 301: 835-7 (1990)). Obesity is also a risk factor for the group of conditions called insulin resistance syndrome, or "Syndrome X" and metabolic syndrome. The worldwide medical cost of obesity and associated disorders is enormous.

The pathogenesis of obesity is believed to be multifactorial. A problem is that, in obese subjects, nutrient availability and energy expenditure do not come into balance until there is excess adipose tissue. The central nervous system (CNS) controls energy balance and coordinates a variety of behavioral, autonomic and endocrine activities appropriate to the metabolic status of the animal. The mechanisms or systems that control these activities are broadly distributed across the forebrain (e.g., hypothalamus), hindbrain (e.g., brainstem), and spinal cord. Ultimately, metabolic (i.e., fuel availability) and cognitive (i.e., learned preferences) information from these systems is integrated and the decision to engage in appetitive (food seeking) and consummatory (ingestion) behaviors is either turned on (meal procurement and initiation) or turned off (meal termination). The hypothalamus is thought to be principally responsible for integrating these signals and then issuing commands to the brainstem. Brainstem nuclei that control the elements of the consummatory motor control system (e.g., muscles responsible for chewing and swallowing). As such, these CNS nuclei have literally been referred to as constituting the "final common pathway" for ingestive behavior.

Neuroanatomical and pharmacological evidence support that signals of energy and nutritional homeostatis integrate in forebrain nuclei and that the consummatory motor control system resides in brainstem nuclei, probably in regions surrounding the trigeminal motor nucleus. There are extensive reciprocal connection between the hypothalamus and brainstem. A variety of CNS-directed anti-obesity therapeutics (e.g., small molecules and peptides) focus predominantly upon forebrain substrates residing in the hypothalamus and/or upon hindbrain substrates residing in the brainstem.

Obesity remains a poorly treatable, chronic, essentially intractable metabolic disorder. Accordingly, a need exists for new therapies useful in weight reduction and/or weight maintenance in a subject. Such therapies would lead to a profound beneficial effect on the subject's health.

All patents, patent applications, and publications cited herein are hereby incorporated by reference in their entirety.

### SUMMARY

The present invention provides methods and compositions useful in the control, treatment and prevention of obesity and obesity-related conditions, disorders, and diseases. The methods of the invention involve administration of at least two anti-obesity agents to a subject in amounts effective to control, treat and prevent obesity and obesity-related conditions, disorders and diseases.

In one aspect, the present invention provides methods for reducing nutrient availability in a subject. In another aspect, the invention provides methods for reducing weight of a subject. In one aspect, the present invention provides methods for inducing a synergistic anti-obesity effect among compounds.

In one aspect, the present invention provides methods for treating obesity in a subject comprising peripherally administering therapeutically effective amounts of at least two different anti-obesity agents, wherein at least one anti-obesity agent acts upon structures in the forebrain involved in food intake or body weight modulation and at least one anti-obesity agent that acts upon structures in the hindbrain involved in food intake or body weight modulation, wherein when one anti-obesity agent is a PYY(3-36) a PYY(3-36) analog, or a PYY(3-36) agonist, then another anti-obesity agent is not an amylin, an amylin agonist, an amylin analog, a CCK, a CCK analog, or a CCK agonist, and wherein when one anti-obesity agent is an exendin, an exendin derivative or an exendin agonist, then another anti-obesity agent is not an amylin, an amylin agonist, an amylin analog.

In certain embodiments, the methods of the invention include administration to a subject at least two different anti-obesity agents where at least one of the anti-obesity agents is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate, a CCK, a CCK analog, a CCK agonist, an amylin, an amylin analog, and an amylin agonist. In certain embodiments, the anti-obesity agent administered is phentermine, rimonabant, sibutramine or pramlintide

In one embodiment, the invention provides a method of treating obesity in a subject comprising peripherally administering therapeutically effective amounts of at least two anti-obesity agents, wherein the first anti-obesity agent is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate, wherein the second anti-obesity agent is selected from the group consisting of a CCK, a CCK analog, a CCK agonist, an amylin, an amylin analog, and an amylin agonist, wherein when the first anti-obesity agent is not a PYY(3-36) a PYY(3-36) analog, or a PYY(3-36) agonist, and wherein when the first anti-obesity agent is an exendin, an exendin derivative or an exendin agonist, then the second anti-obesity agent is not an amylin, an amylin agonist, an amylin analog.

In one embodiment, the invention provides methods of treating obesity comprising administration of a first anti-obesity agent selected from an amylin, an amylin analog or an amylin agonist in combination with a second anti-obesity agent selected from a leptin, a leptin derivative or a leptin agonist, wherein the administration of the agents result in a synergistic effect as compared to administration of either agent alone.

In certain embodiments, the invention provides methods whereby the subject reduces body weight by least 10%, the subject reduces body fat mass, the subject loses ectopic fat, or any combination thereof.

In some embodiments, the methods are direct to a subject which suffers from obesity, an obesity-related disorder, an obesity related disease, an obesity-related condition, diabetes, insulin-resistance syndrome, lypodystrpohy, nonalcoholic steatohepatitis, a cardiovascular disease, polycystic ovary syndrome, metabolic syndrome or a desire to lose body weight.

In one aspect, administration of the anti-obesity agents in combination may be simultaneous, concurrent, or sequential administration.

The present invention is also concerned with treatment of obesity and obesity-related conditions, disorders and diseases, and the use of the anti-obesity agents and compositions of the present invention for manufacture of a medicament useful for treating these conditions.

### BRIEF DESCRIPTION OF THE DRAWINGS

Fig. 1 is a graph depicting an effect of administration of leptin and amylin on food intake.

Fig. 2 is a graph depicting an effect of administration of leptin and amylin on body weight.

Fig. 3 is a graph depicting an effect of administration of leptin and amylin on body composition.

Fig. 4 is a graph depicting an effect of administration of leptin and amylin on body composition.

Fig. 5 is a graph depicting an effect of administration of leptin and amylin on energy expenditure.

Fig. 6A is a graph depicting an effect on body weight of administration of leptin (500 µg/kg/day) and amylin (100 µg/kg/day), either alone or in combination over two weeks. Fig. 6B is a graph depicting an effect on body fat of a two-week administration of leptin (504 µg/kg/day) and amylin (100 µg/kg/day), either alone or in combination. Fig. 6C is a graph depicting an effect on body protein of a two-week administration of leptin (500 µg/kg/day) and amylin (100 µg/kg/day), either alone or in combination.

Fig. 7 is a graph depicting an effect on body weight of administration of leptin alone (500 µg/kg/day), pair-fed leptin alone (500 µg/kg/day), and leptin (500 µg/kg/day and amylin (100 µg/kg/day) in combination over two weeks.

Fig. 8 shows graphs depicting serum leptin concentrations in normal HSD and in DIO prone animals that either received vehicle, were pair-fed to the amylin-treated group, or received amylin (100 µg/kg/day) for two weeks.

Fig. 9A is a graph depicting an effect on body weight of administration of vehicle or leptin (500 µg/kg/day) in normal animals. Fig. 9B is a graph depicting an effect on body weight of administration of vehicle or leptin (500 µg/kg/day) in DIO prone animals.

Fig. 10A is a graph depicting an effect on body weight of administration of sibutramine (3 mg/kg/day) and amylin (100 µg/kg/day), either alone or in combination over two weeks. Fig. 6B is a graph depicting an effect on body fat of a two-week administration of sibutramine (3 mg/kg/day) and amylin (100 µg/kg/day), either alone or in combination. Fig. 6C is a graph depicting an effect on body protein of a two-week administration of sibutramine (3 mg/kg/day) and amylin (100 µg/kg/day), either alone or in combination.

Fig. 11A is a graph depicting an effect on body weight of administration of phentermine (10 mg/kg/day) and amylin (100 µg/kg/day), either alone or in combination over two weeks. Fig. 6B is a graph depicting an effect on body fat of a two-week administration of phentermine (10 mg/kg/day) and amylin (100 µg/kg/day), either alone or in combination. Fig. 6C is a graph depicting an effect on body protein of a two-week administration of phentermine (10 mg/kg/day) and amylin (100 µg/kg/day), either alone or in combination.

Fig. 12A is a graph depicting an effect on body weight of administration of rimonabant (3 mg/kg/day) and amylin (100 µg/kg/day), either alone or in combination over two weeks. Fig. 6B is a graph depicting an effect on body fat of a two-week administration of rimonabant (3 mg/kg/day) and amylin (100 µg/kg/day), either alone or in combination. Fig. 6C is a graph depicting an effect on body protein of a two-week administration of rimonabant (3 mg/kg/day) and amylin (100 µg/kg/day), either alone or in combination.

Figure 13 is a graph depicting the effect of administration of a range of doses of a CB-1 antagonist, either alone or in combination with amylin (100 µg/kg/day), on body weight. The time course of the combinations in the circled area are depicted in Figs. 14A and B.

Fig. 14A is a graph depicting the effect of administration of a CB-1 antagonist (1 mg/kg/day) and amylin (100 µg/kg/day), either alone or in combination, on body weight. Fig. 14B is a graph depicting the effect of administration of a CB-1 antagonist (3 mg/kg/day) and amylin (100 µg/kg/day), either alone or in combination, on body weight.

Figure 15A is a graph depicting an effect on body weight of administration of an exendin-4 analog (10 µg/kg/day) and amylin (100 µg/kg/day), either alone or in combination over two weeks. Fig. 6B is a graph depicting an effect on body fat of a two-week administration of an exendin-4 analog (10 µg/kg/day) and amylin (100 µg/kg/day), either alone or in combination. Fig. 6C is a graph depicting an effect on body protein of a two-week administration of exendin-4 (10 µg/kg/day) and amylin (100 µg/kg/day), either alone or in combination.

Figure 16A is a graph depicting an effect on body weight of administration of PYY(3-36) (1 mg/kg/day) and amylin (100 µg/kg/day), either alone or in combination over two weeks. Fig. 6B is a graph depicting an effect on body fat of a two-week administration of PYY(3-36) (1 mg/kg/day) and amylin (100 µg/kg/day), either alone or in combination. Fig. 6C is a graph depicting an effect on body protein of a two-week administration of PYY(3-36) (1 mg/kg/day) and amylin (100 µg/kg/day), either alone or in combination.

### DETAILED DESCRIPTION

We have discovered that administration of an anti-obesity agent that acts upon structures in the forebrain involved in food intake and/or body weight modulation in combination with an anti-obesity agent that acts upon structures in the hindbrain involved in food intake and/or body weight modulation is surprisingly effective in reducing nutrient availability and in treating obesity and obesity related conditions, disorders, and diseases. It has been discovered that when administration of such anti-obesity agents is combined in this manner, the anti-obesity agents are more effective in reducing nutrient availability in the recipient than use of one of the agents alone. As shown herein, for example, a combination of anti-obesity agents can act synergistically to reduce nutrient availability, e.g., to reduce weight, reduce fat, reduce food intake, or any combination of these three.

Particular areas of the forebrain (telencephalonic- and diencephalonic-derived constituents of the brain) and hindbrain or brainstem (including the midbrain, pons and medulla) have been identified as being involved in controlling energy balance. Forebrain structures or nuclei residing in the hypothalamus involved in food intake and/or body weight modulation include, for example, the arcuate nucleus (ARC), the paraventricular nucleus (PVN), the dorsomedial hypothalamus (DMH), the ventromedial nucleus (VMH), and the lateral hypothalamus nucleus (LHA). Hindbrain structures or nuclei residing in the brainstem involved in food intake and/or body weight modulation include, for example, the nucleus of the solitary tract (NST), the area postrema (AP), and the lateral parabrachial nucleus (IPBN). Brainstem nuclei that control the elements of the consummatory motor control system are likely controlled by primary or second order projections from brainstem regions like the NST, AP, and IPBN. It is noteworthy that the AP, NST and IPBN have all been shown to (collectively and independently) possess their own integrative abilities.

A variety of CNS-directed anti-obesity agents act upon these forebrain structures residing in the hypothalamus involved in food intake and/or body weight modulation. In addition, CNS-directed anti-obesity agents act upon hindbrain structures residing in the brainstem involved in food intake and/or body weight modulation. Examples of such anti-obesity agents are described herein. See Table 1 for examples. Such agents include, for example, neuropeptide Y1 (NPY1) receptor antagonists, NPY5 receptor antagonists, leptin and leptin agonists, ciliary neurotrophic factor (CNTF) and CNTF agonists, melanin-concentrating hormone (MHC) and MCH antagonists, melacortins (MC) and MC agonists, cannabinoid receptor (CB-1) antagonists, serotonin (5-HT) and 5-HT agonists, peptide YY (PYY) and PYY agonists, exendin and exendin agonists, GLP-1 and GLP-1 agonist, DPP-IV inhibitors, ghrelin and ghrelin antagonists, cholecystokinin (CCK) and CCK agonists, and amylin and amylin agonists.

**Table 1. Individual anti-obesity targets and location**

| Signaling System | CNS Region | Food Intake Role | Anti-obesity agents |
|---|---|---|---|
| Neuropeptide Y (NPY) | Forebrain (ARC/PVN) | Increases intake | NAY1 and NPY5 receptor antagonists |
| Leptin | Forebrain (ARC) | Decreases intake | Leptin, or agonists |
| Ciliary neurotrophic factor (CNTF) | Forebrain (ARC) | Decreases intake | CNTF (Axokine®) |
| Melanin-concentrating hormone (MCH) | Forebrain (ARC/PVN) | Increases intake | MCH antagonists |
| Melanocortins (MC) | Forebrain (PVN/ARC) | Agonists decrease intake | MC4 agonists |
| Cannabinoids (CB) | Forebrain (widespread) | Increase intake | Cannabinoid receptor antagonists |
| Serotonin (5-HT) | Forebrain (VMH) | Decrease intake | 5-HT2C agonists |
| Peptide YY (PYY) | Forebrain (ARC) | Decrease intake | PYY(3-36) agonists |
| Glucagon-like peptide-1 (GLP-1) | Forebrain (PVN) | Decrease intake | Exenatide and other GLP-1 ligands, DPP-IV inhibitors |
| Ghrelin | Forebrain (ARC) | Increase intake | Ghrelin antagonists |
| Cholecystokinin (CCK) | Hindbrain (AP) | Decrease intake | CCK agonists |
| Amylin | Hindbrain (AP) | Decrease intake | Amylin agonists, Pramlintide, amylin analogs |

In certain embodiments, the methods include a first compound that predominantly targets the energy balance centers of the hypothalamus, such as the ARC, PVN, VM, and LH. In certain embodiments, the methods include a second compound that predominantly targets the energy balance centers of the hindbrain such as the NST, the AP and the IPBN.

In certain embodiments, these compounds are anti-obesity agents. In certain embodiments, the methods may include use of one or more predominantly forebrain acting anti-obesity agents. In other embodiments, the methods may include use of one or more predominantly hindbrain acting anti-obesity agents. Exemplary anti-obesity agents include an NPY1 receptor antagonist, an NPY5 receptor antagonist, a leptin or a leptin agonist or analog, a CNTF (*e*.*g*., AXOKINE®), an MCH antagonist, a MC4 agonist, a CB-1 antagonist (*e*.*g*., rimonabant), a 5-HT2C agonist, an NPY2 receptor agonist (e.g., a PYY(3-36) or a PYY(3-36) agonist), an exendin or an exendin agonist or analog, a GLP-1 or a GLP-1 agonist or analog, a DPP-IV inhibitor, a ghrelin antagonist, a CCK or a CCK agonist or analog, and an amylin or an amylin agonist or analog.

As exemplified herein, agonists and antagonists that are anti-obesity agents in the invention include, for example, molecules such peptides, polypeptides and small molecules agents.

The details of one or more embodiments of the invention are set forth in the accompanying drawings and the description below. Other features, objects, and advantages of the invention will be apparent from the description and drawings, and from the claims.

### Definitions

An "anti-obesity agent" is a compound that is able to reduce nutrient availability to the body upon administration. A "weight-inducing agent" is a compound that would increase nutrient availability to the body. In one aspect, a weight-inducing agent is an antagonist of an anti-obesity agent.

As used herein, an anti-obesity agent that "acts on a forebrain structure involved in food intake and/or body weight modulation" stimulates or suppresses activity of a particular region, e.g., particular nuclei and/or neuronal circuits, in the forebrain. This forebrain stimulation or suppression leads to a reduction in nutrient availability to the body. An anti-obesity agent that "acts on a hindbrain structure involved in food intake and/or body weight modulation" stimulates or suppresses activity of a particular region, e.g., particular nuclei and/or neuronal circuits, in the hindbrain. This hindbrain stimulation or suppression results in a reduction in nutrient availability to the body.

"Reduced nutrient availability" is meant to include any means by which the body reduces the nutrients available to the body to store as fat. In other words, reducing nutrient availability may be by means that include, but are not limited to, reducing appetite, increasing satiety, affecting food choice/taste aversion, increasing metabolism, and/or decreasing or inhibiting food absorption. Exemplary mechanisms that may be affected include delayed gastric emptying or decreased absorption of food in the intestines.

"Increased nutrient availability" is meant to include any means by which the body increases the nutrients available to the body to store as fat. In other words, increasing nutrient availability may be by means that include, but are not limited to, increasing appetite, decreasing satiety, affecting food choice, decreasing taste aversion, decreasing metabolism, and/or increasing food absorption. Exemplary mechanisms that may be affected include decreasing gastric hypomotility or increasing absorption of food in the intestines.

While "obesity" is generally defined as a body mass index (BMI) over 30, for purposes of this disclosure, any subject, including those with a body mass index of less than 30, who needs or wishes to reduce body weight or prevent body weight gain is included in the scope of "obese." Thus, subjects with a BMI of less than 30 and 25 and above (considered overweight) or below 25 are also included in the subjects of the invention. Morbid obesity refers to a BMI of 40 or greater.

With regard to the methods to reduce nutrient availability, as used herein, a "subject in need thereof" includes subjects who are overweight or obese or morbidly obese, or desirous of losing weight. In addition, subjects who are insulin resistant, glucose intolerant, or have any form of diabetes mellitus (e.g., type 1, 2 or gestational diabetes) can benefit from these methods to reduce nutrient availability.

With regard to the methods to increase nutrient availability, as used herein, a "subject in need thereof" includes subjects who are underweight or desirous of gaining weight.

A "subject" is meant to include any animal, including humans, primates, and other mammals including rats, mice, pets such as cats, dogs, livestock such as horses, cattle, sheep and goats, as well as chicken, turkey and any other animal for which body weight or altering body composition may be an issue.

By "metabolic rate" is meant the amount of energy liberated/expended per unit of time. Metabolism per unit time can be estimated by food consumption, energy released as heat, or oxygen used in metabolic processes. It is generally desirable to have a higher metabolic rate when one wants to loose weight. For example, a person with a high metabolic rate may be able to expend more energy (e.g., the body burns more calories) to perform an activity than a person with a low metabolic rate for that activity.

As used herein, "lean mass" or "lean body mass" refers to muscle and bone. Lean body mass does not necessarily indicate fat free mass. Lean body mass contains a small percentage of fat (roughly 3%) within the central nervous system (brain and spinal cord), marrow of bones, and internal organs. Lean body mass is measured in terms of density. Methods of measuring fat mass and lean mass include, but are not limited to, underwater weighing, air displacement plethysmograph, x-ray, DEXA scans, MRIs and CT scans. In one embodiment, fat mass and lean mass is measured using underwater weighing.

By "fat distribution" is meant the location of fat deposits in the body. Such locations of fat deposition include, for example, subcutaneous, visceral and ectopic fat depots.

By "subcutaneous fat" is meant the deposit of lipids just below the skin's surface. The amount of subcutaneous fat in a subject can be measured using any method available for the measurement of subcutaneous fat Methods of measuring subcutaneous fat are known in the art, for example, those described in U.S. Patent No. 6,530,886, the entirety of which is incorporated herein by reference.

By "visceral fat" is meant the deposit of fat as intra-abdominal adipose tissue. Visceral fat surrounds vital organs and can be metabolized by the liver to produce blood cholesterol. Visceral fat has been associated with increased risks of conditions such as polycystic ovary syndrome, metabolic syndrome and cardiovascular diseases.

By "ectopic fat storage" is meant lipid deposits within and around tissues and organs that constitute the lean body mass (e.g., skeletal muscle, heart, liver, pancreas, kidneys, blood vessels). Generally, ectopic fat storage is an accumulation of lipids outside classical adipose tissue depots in the body.

As used herein, and as well-understood in the art, "treatment" is an approach for obtaining beneficial or desired results, including clinical results. "Treating" or "palliating" a disease, disorder, or condition means that the extent and/or undesirable clinical manifestations of a condition, disorder, or a disease state are lessened and/or time course of the progression is slowed or lengthened, as compared to not treating the disorder. For example, in treating obesity, a decrease in body weight, e.g., at least a 5% decrease in body weight, is an example of a desirable treatment result. For purposes of this invention, beneficial or desired clinical results include, but are not limited to, alleviation or amelioration of one or more symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Further, treating does not necessarily occur by administration of one dose, but often occurs upon administration of a series of doses. Thus, a therapeutically effective amount, an amount sufficient to palliate, or an amount sufficient to treat a disease, disorder, or condition may be administered in one or more administrations.

As used herein, the term "therapeutically effective amount" means the amount of the active compounds in the composition that will elicit the biological or medical response in a tissue, system, subject, or human that is being sought by the researcher, veterinarian, medical doctor or other clinician, which includes alleviation of the symptoms of the disorder being treated. The novel methods of treatment of this invention are for disorders known to those skilled in the art.

As used herein, the term "prophylactically effective amount" means the amount of the active compounds in the composition that will elicit the biological or medical response in a tissue, system, subject, or human that is being sought by the researcher, veterinarian, medical doctor or other clinician, to prevent the onset of obesity or an obesity-related disorder, condition or disease in subjects as risk for obesity or the obesity-related disorder, condition or disease.

As used herein, the singular form "a", "an", and "the" includes plural references unless otherwise indicated or clear from context. For example, as will be apparent from context, "an" amylin agonist can include one or more amylin agonists.

As used herein, an "analog" refers to a peptide whose sequence was derived from that of a base reference peptide, *e*.*g*., amylin and calcitonin, and includes insertions, substitutions, extensions, and/or deletions of the reference amino acid sequence, for example having at least 50 or 55% amino acid sequence identity with the base peptide, in other cases, for example, having at least 70%, 80%, 90%, or 95% amino acid sequence identity with the base peptide. Such analogs may comprise conservative or non-conservative amino acid substitutions (including non-natural amino acids and L and D forms). Analogs include compounds having agonist and compounds having antagonist activity. Analogs, as herein defined, also include derivatives.

A "derivative" is defined as a reference peptide or analogs, described above, having a chemical modification of one or more of its amino acid side groups, α-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the desamino, N-lower alkyl, N-di-lower alkyl, and N-acyl modifications. Modifications of the terminal amino include, without limitation, the desamino, N-lower alkyl, N-di-lower alkyl, and N-acyl modifications, such as alkyl acyls, branched alkylacyls, akylaryl-acyls. Modifications of the terminal carboy group include, without limitation, the amide, lower alkyl amide, dialkyl amide, arylamide, alkylarylamide and lower alkyl ester modifications. Lower alkyl is C1-C4 alkyl. Furthermore, one or more side groups, or terminal groups, may be protected by protective groups known to the ordinarily-skilled synthetic chemist. The α-carbon of an amino acid may be mono- or dimethylated.

In general, with respect to an amino acid sequence, the term "modification" includes substitutions, insertions, elongations, deletions, and derivatizations alone or in combination. The polypeptides of the invention may include one or more modifications of a "non-essential" amino acid residue. In the context of the invention, a "non-essential" amino acid residue is a residue that can be altered, *e.g*., deleted or substituted, in the novel amino acid sequence without abolishing or substantially reducing the activity (*e*.*g*., the agonist activity) of the polypeptide (*e.g*., the analog polypeptide). The polypeptides of the invention may include deletions of 1, 2, 3, 4, 5, 6, 7, 8, 9, 10 or more non-essential amino acid residues. The polypeptides of the invention may include additions of at least of 1, 2, 3, 4, 5, 6, 7, 8, 9, 10 or more amino acids without abolishing or substantially reducing the activity of the polypeptide.

Substitutions include conservative amino acid substitutions. A "conservative amino acid substitution" is one in which the amino acid residue is replaced with an amino acid residue having a similar side chain, or physicochemical characteristics (*e*.*g*., electrostatic, hydrogen bonding, isosteric, hydrophobic features). The amino acids may be naturally occurring or nonnatural (unnatural). Families of amino acid residues having similar side chains are known in the art. These families include amino acids with basic side chains (*e.g*., lysine, arginine, histidine), acidic side chains (*e.g*., aspartic acid, glutamic acid), uncharged polar side chains (*e.g.,* glycine, asparagine, glutamine, serine, threonine, tyrosine, metbionine, cysteine), nonpolar side chains (*e.g.,* alanine, valine, leucine, isoleucine, proline, phenylalanine, tryptophan), β-branched side chains (*e.g.,* threonine, valine, isoleucine) and aromatic side chains (*e.g*., tyrosine, phenylalanine, tryptophan, histidine). Substitutions may also include non-conservative changes.

By "amino acid" or "amino acid residue" is meant natural amino acids, unnatural amino acids, and modified amino acid. Unless stated to the contrary, any reference to an amino acid, generally or specifically by name, includes reference to both the D and the L stereoisomers if their structure allow such stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), seine (Ser), threonine (Thr), tryptophan (Trp), tyrosine (Tyr) and valine (Val), Unnatural amino acids include, but are not limited to hommlysine, homoarginine, homoserine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-amineisbutyric acid, 2-ammopimelic acid, teztiary-butylglycine, 2,4-diaminoisobutyric acid, desmosine, 2,2'-diammopimelic acid, 2,3-diaminopropionic acid, N-ethylglycine, N-ethylasparagine, homoproline, hydroxylysine, allo-hydroxylysine, 3-hydroxyprolrue, 4-hydroxyproline, isodesmosine, allo-isoleucine, N-methylalanine, N-methylglycine, N-methylisolsucine, N-methylpentylglycine, N-msthylvaline, naphthalanine, norvaline, norleucine, ornithine, pentylglycine, pipecolic acid and thioproline. Additional unnatural amino acids include modified amino acid residues which are chemically blocked, reversibly or irreversibly, or chemically modified on their N-terminal amino group or their side chain groups, as for example, N-methylated D and L amino acids or residues wherein the side chain functional groups are chemically modified to another functional group. For example, modified amino acids include methionine sulfoxide; methionine sulfone; aspartic acid- (beta-methyl ester), a modified amino acid of aspartic acid; N-etlaylglycine, a modified amino acid of glycine; or alanine carboxamide, a modified amino acid of alanine. Additional residues that can be incorporated are described in Sandberg et al., J. Med. Chem. 41: 2481-91, 1998.

It should be noted that throughout the application that alternatives are written in Markush groups, for example, each amino acid position that contains more than one possible amino acid. It is specifically contemplated that each member of the Markush group should be considered separately, thereby comprising another embodiment of the invention, and the Markush group is not to be read as a single unit.

### Methods of the Invention

In a general aspect, the invention provides methods for reducing nutrient availability through administration of a combination of anti-obesity agents. Thus, the invention provides methods for treating obesity and obesity-related diseases, disorders, and/or conditions that would benefit from a reduction in nutrient availability. Given the increase in effectiveness when used in combinations, methods of the invention may allow for administration of lower dosages of one or more of the anti-obesity agents used in combination as compared to the use of the agent alone, such as in monotherapy.

The methods of the invention provide administration of a combination of anti-obesity agents. Administration of the agents "in combination" should be understood to mean providing each of the agents to a subject in need of treatment. Administration of the agents could occur as a single pharmaceutical dosage formulation containing all of the intended anti-obesity agents or in separately with each intended agent in its own dosage formulation.

Where separate dosage formulations are used, the individual anti-obesity agents can be administered at essentially the same time, *i.e.,* concurrently, or at separately staggered times, *i.e.,* sequentially prior to or subsequent to the administration of the other anti-obesity agent of the method. In some embodiments, administration in combination involves administration of separate dosage formulations during overlapping intervals. For example, anti-obesity agent 1 is administered from day 1 through day 30 and anti-obesity agent 2 is administered from day 20 through day 50. In other embodiments, administration in combination involves administration of separate dosage formulations in sequential, nonoverlapping intervals. For example, anti-obesity agent 1 is administered from day 1 through day 30 and anti-obesity agent 2 is administered from day 35 through day 50. The instant invention is therefore to be understood to include all such regimes of simultaneous, alternating, or completely separate treatments over the total treatment course, and the terms "administration," "administering," "administration in combination" and "administering in combination" are to be interpreted accordingly.

In one embodiment, the invention provides methods for reducing nutrient availability through administration of at least one anti-obesity agent that acts upon forebrain structures involved in food intake and/or body weight modulation in combination with administration of at least one anti-obesity agent that acts upon hindbrain structures involved in food intake and/or body weight modulation. In some cases, the methods of the invention increase or enhance the effectiveness an anti-obesity agent that has limited effectiveness, if any, when used alone (monotherapy). In such cases, the methods of the invention increase or enhance the effectiveness an anti-obesity agent by, for example, preventing or delaying loss of effectiveness by continued use or increasing potency. Methods of the invention may allow for administration of lower dosages of one or more of the anti-obesity agents used in combination as compared to the use of either agent alone.

In one aspect, methods of the invention provide a synergistic anti-obesity effect among the administered agents. Accordingly, in one embodiment, administration of a combination of anti-obesity agents results in an effect, e.g., a reduction in nutrient availability, reduction in body weight, reduction in food intake, increase in metabolism, which is greater than the combination of the results of administration of the anti-obesity agent alone (monotherapy).

In another aspect of the invention, methods are provided which reduce or eliminate a subject's resistance to an anti-obesity agent so that when the agent is administered, it will be able to elicit an anti-obesity response (e.g., reduce nutrient availability, reduce weight, reduce fat mass). For example, and without wishing to be bound by this or any other theory, it is theorized that leptin resistance may be due to the high levels of leptin found in obese subjects (i.e., the body has become desensitized to leptin). Therefore, one method of the invention comprises administering an anti-obesity agent other than leptin (e.g., amylin or an amylin agonist) to reduce weight of the subject so as to reduce or remove the leptin resistance. Once this has been achieved, leptin is then be administered, either alone or in combination with an anti-obesity agent (e.g., amylin or an amylin agonist), for a further anti-obesity effect. Other means of reducing weight to ameliorate leptin resistance are contemplated, such as diet, exercise, other diet drugs, and surgical devices.

In one embodiment, the invention is directed to the delivery of a first anti-obesity agent that acts upon hindbrain structures involved in food intake and/or body weight modulation to prime the body before administration of a second anti-obesity agent that acts upon forebrain structures involved in food intake and/or body weight modulation. In certain embodiments, the administration of the first agent is for a number of days, weeks or even months before the administration of the second agent. At this point, the second agent may be administered alone or in combination with the first agent. In certain embodiments, the first anti-obesity agent is amylin or an amylin agonist and the second agent is a leptin or a leptin agonist. In some embodiments, the serum leptin concentration of a subject prior to administration of the anti-obesity agents is greater than 10 ng/ml, in other embodiments, it is greater than 20 ng/ml.

In one embodiment, an amylin or an amylin agonist is administered to the subject at least 1 day, 2 days, 3 days, 5 days, 7 days, 10 days, 14 days, 21 days, 28 days or more prior to administration of a leptin or leptin agonist. In some embodiments, prior to administration of a leptin or a leptin agonist, an amylin or an amylin agonist is administered to the subject until the serum leptin concentration in the subject is about 4 ng/ml, less than 4 ng/ml, less than 2 ng/ml, less than 1 ng/ml, or less than about 0.5 ng/ml. In some embodiments, a leptin or a leptin agonist is administered in a replacement therapy amount to achieve near physiological concentrations of leptin in the plasma.

In another aspect of the present invention, methods for reducing the risk of developing metabolic disorders are provided, where the method comprises administering to the subject a combination of anti-obesity agents in effective amounts to reduce the weight of a subject.

In some embodiments of the invention methods of the invention are used to increase the metabolic rate in a subject, decrease a reduction in the metabolic rate in a subject, or preserve the metabolic rate in a subject. In one embodiment, the metabolic rate may involve the preferential use of the body's fat as an energy source over lean body tissue. In one aspect, lean body mass is not decreased following administration of the combination of anti-obesity agents. In another aspect, a reduction in the lean body mass is lessened or prevented following administration of the combination of anti-obesity agents. In still another aspect, lean body mass is increased following administration of the combination of anti-obesity agents. Such preference for fat as the energy source may be determined by comparing the amount of fatty tissue to lean body tissue, ascertained by measuring total body weight and fat content at the beginning and end of the treatment period. An increase in metabolic rate is a higher level of the use of calories or another energy source by a subject over a period of time compared with the level of use of calories or other energy source by the subject over another period of time under substantially similar or identical conditions without administration of the combination of anti-obesity agents. In one embodiment, the metabolic rate is increased at least about 5% in a subject, in other embodiments, the metabolic rate is increased at least about 10%, 15%, 20% 25%, 30%, or 35% in a subject compared with the level of use of calories or other energy source by the subject over another period of time under substantially similar or identical conditions without administration of the combination of anti-obesity agents. The increase in metabolic rate can be measured using a respiratory calorimeter, for example. An effective amount of the anti-obesity agents of as used in this embodiment is an amount of each agent effective to increase the metabolic rate in a subject when administered in combination compared to a subject not receiving the agents or only one of the agents.

In another embodiment, a method is provided to reduce a decrease in metabolic rate in a subject Such a decrease in metabolic rate can be the result of any condition or nutritional or physical regimen that leads to a reduction in metabolic rate, for example, due to a reduced calorie diet, a restricted diet, or weight loss. A restricted diet includes allowances or prohibitions, or both on the types of food or the amounts of food or both permitted in a diet, not necessarily based on calories. For example, as in individual diets, the body compensates with a reduced metabolic rate based on the lower caloric intake. In essence, the body down-regulates the requirement for food, thereby subsisting on less food. As dieting continues, the threshold for caloric intake is reduced. When dieting has ended, the individual typically gains weight while eating a normal diet because of the lowered caloric intake threshold and lower-basal metabolic rate (NIH Technology Assessment Conference Panel (1992) Ann. Intern. Med. 116:942-949; Wadden (1993) Ann. Intern. Med. 119:688-693). In one aspect, a method is provided to reduce the loss of metabolic rate in a subject, where the loss of metabolic rate is the result of a reduced calorie diet or weight loss. By using such a method, the subject's reduction in metabolic rate is decreased by at least about 10%, 15%, 20% 25%, 30%, 35%, 40%, 50%, 60%, 70%, 80%, 90%, or 95% in a subject. For such methods, it may be desirable to administer the combination of anti-obesity agents at the time the condition or nutritional or physical regimen is initiated which leads to a loss or reduction in metabolic rate. However, it is also contemplated that administration of the agents is commenced before the condition or nutritional or physical regimen is initiated. In one instance, metabolic rate is measured using a respiratory calorimeter. An effective amount of the anti-obesity agents of as used in this embodiment is an amount of each agent effective to decrease the reduction of the metabolic rate in a subject when administered in combination.

In another aspect, methods for reducing metabolic plateaus are provided, where a method comprises administering effective amounts of anti-obesity agents in combination to a subject. In one embodiment, the subject is losing weight, or has lost weight, for example, due to a reduced calorie diet, increased exercise or a combination thereof. By "metabolic plateau" is meant time intervals of steady metabolic rate while the body adjusts to changes in caloric or energy input. Changes in caloric input or expenditure can be the result of, for example, reduced calorie diets or increased physical activity. Such plateaus can be observed, for example, during a weight loss regimen when weight loss slows or stops. In one embodiment, a method of the present invention reduces the duration of a metabolic plateau in a subject compared with the duration of metabolic plateaus in an otherwise identical subject over the same period of time under substantially similar or identical conditions without administration of the combination of anti-obesity agents. In another embodiment, a method of the present invention reduces the frequency of metabolic plateaus compared with the frequency of metabolic plateaus in an otherwise identical subject over the same period of time under substantially similar or identical conditions without administration of the combination of anti-obesity agents. In still another embodiment, a method of the present invention delays the onset of a metabolic plateau compared with the onset of a metabolic plateau in an otherwise identical subject over the same period of time under substantially similar or identical conditions without administration of the combination of anti-obesity agents. In one embodiment, metabolic plateaus are identified by charting periods of reduced or no weight loss. In one embodiment, at least one metabolic plateau is reduced. In other embodiments, at least two, three, four, five, six, seven, eight, nine, or ten metabolic plateaus are reduced. In another aspect, metabolic plateaus are delayed one day as compared to a subject not administered the combination of anti-obesity agents under identical or similar conditions. In other aspects, metabolic plateaus are delayed 2 days, 3 days, 4 days, 5 days, 6 days, 1 week, 10 days, 2 weeks or 3 weeks in a subject.

In yet another embodiment, a method is provided to preserve the metabolic rate in a subject. In one embodiment, the subject may be at risk of losing metabolic rate, for example, due to the initiation of a reduced calorie diet, restricted diet, or anticipated weight loss. A preservation of metabolic rate is a maintenance of the level of the use of calories or another energy source by a subject over a period of time compared with the level of use of calories or other energy source by an otherwise identical subject over the same period of time under substantially similar or identical conditions without administration of the combination of anti-obesity agents. In one aspect, the metabolic rate is maintained within 15% of the subject's metabolic rate prior to the initiation of the event that results in the decrease in metabolic rate. In other aspects, the metabolic rate is maintained within 10%, within 7%, within 5%, within 3% or less of the subject's metabolic rate. In one aspect, the combination of anti-obesity agents is administered at the initiation of a reduced calorie diet, restricted diet, or exercise regimen.

Metabolic rates can be assessed using any method available for determining such rates, for example by using a respiratory calorimeter. Such methods and devices for assaying metabolic rates are known in the art and are described, for example, in U.S. Patent Nos. 4,572,208, 4,856,531, 6,468,222, 6,616,615, 6,013,009. and 6,475,158. Alternatively, the metabolic rate of an animal can be assessed by measuring the amount of lean tissue versus fatty tissue catabolized by the animal following the diet period. Thus, total body weight and fat content can be measured at the end of the dietary period. In rats, a frequently used method to determine total body fat is to surgically remove and weigh the retroperitoneal fat pad, a body of fat located in the retroperitoneum, the area between the posterior abdominal wall and the posterior parietal peritoneum. The pad weight is considered to be directly related to percent body fat of the animal. Since the relationship between body weight and body fat in rats is linear, obese animals have a correspondingly higher percent of body fat and retroperitoneal fat pad weight.

In another aspect of the present invention, methods for reducing fat mass by increasing the metabolic rate in a subject are provided, where the methods comprise administering a combination of anti-obesity agents in amounts effective to reduce fat mass by increasing the subject's metabolic rate. Fat mass can be expressed as a percentage of the total body mass. In some aspects, the fat mass is reduced by at least 1%, at least 5%, at least 10%, at least 15%, at least 20%, or at least 25% oyer the course of treatment. In one aspect, the subject's lean mass is not decreased over the course of the treatment. In another aspect, the subject's lean mass is maintained or increased over the course of the treatment. In another aspect, the subject is on a reduced calorie diet or restricted diet. By "reduced calorie diet" is meant that the subject is ingesting fewer calories per day than compared to the same subject's normal diet. In one instance, the subject is consuming at least 50 fewer calories per day. In other instances, the subject is consuming at least 100, 150, 200, 250, 300, 400, 500, 600, 700, 800, 900, or 1000 fewer calories per day.

In one embodiment of the present invention, a method for altering the fat distribution in a subject is provided where the method comprises administering a combination of anti-obesity agents in amounts effective to alter fat distribution in the subject. In one aspect, the alteration results from an increased metabolism of visceral or ectopic fat, or both in the subject. In some embodiments, the method involves the metabolism of visceral or ectopic fat or both at a rate of at least about 5%, 10%, 15%, 20%, 25%, 30%, 40%, or 50% greater than for subcutaneous fat. In one aspect, the methods result in a favorable fat distribution. In one embodiment, favorable fat distribution is an increased ratio of subcutaneous fat to visceral fat, ectopic fat, or both. In one aspect, the method involves an increase in lean body mass, for example, as a result of an increase in muscle cell mass.

In another embodiment, methods for reducing the amount of subcutaneous fat in a subject are provided, wherein the method comprises administering, to a subject in need thereof, a combination of anti-obesity agents in amounts effective to reduce the amount of subcutaneous fat in the subject. In one instance, the amount of subcutaneous fat is reduced in a subject by at least about 5%. In other instances, the amount of subcutaneous fat is reduced by at least about 10%, 15%, 20%, 25%, 30% 40%, or 50% compared to the subject prior to administration of the anti-obesity agents.

The methods described herein can be used to reduce the amount of visceral fat in a subject. In one instance, the visceral fat is reduced in a subject by at least about 5%. In other instances, the visceral fat is reduced in the subject by at least about 10%, 15%, 20%, 25%, 30% 40%, or 50% compared to the subject prior to administration of the combination of anti-obesity agents. Visceral fat can be measured through any means available to determine the amount of visceral fat in a subject. Such methods include, for example, abdominal tomography by means of CT scanning and MRI. Other methods for determining visceral fat are described, for example, in U.S. Patent Nos. 6,864,415, 6,850,797, and 6,487,445.

In one embodiment, a method for preventing the accumulation of ectopic fat or reducing the amount of ectopic fat in a subject is provided, wherein the method comprises administering, to a subject in need thereof, a combination of anti-obesity agents in amounts effective to prevent accumulation of ectopic fat or to reduce the amount of ectopic fat in the subject. In one instance, the amount of ectopic fat is reduced in a subject by at least about 5% compared to the subject prior to administration of the combination of anti-obesity agents. In other instances, the amount of ectopic fat is reduced in a subject by at least about 10%, or by at least about 15%, 20%, 25%, 30% 40%, or 50%. Alternatively, the amount of ectopic fat is proportionally reduced 5%, 10%, 15%, 20%, 25%, 30%, 40%, 50%, 60%, 70%, 80%, 90%, or 100% in comparison to subcutaneous fat in a subject. Ectopic fat can be measured in a subject using any method available for measuring ectopic fat.

In another embodiment, methods are provided for producing a more favorable fat distribution in a subject, where the method comprises administering to a subject a combination of anti-obesity agents in amounts effective to produce a favorable fat distribution. In one embodiment, administration of a combination of anti-obesity agents reduces the amount of visceral fat or ectopic fat, or both, in a subject For example, administration of a combination of anti-obesity agents, where at least one anti-obesity agent that acts upon forebrain structures involved in food intake or body weight modulation or both in combination with administration of at least one anti-obesity agent that acts upon hindbrain structures involved in food intake or body weight modulation or both. In one embodiment, the methods preferentially reduce the amount of visceral or ectopic fat, or a combination of both, over the reduction in subcutaneous fat. Such methods result in a higher ratio of subcutaneous fat to visceral fat or ectopic fat. Such improved ratios may result in a reduced risk of the development of cardiovascular diseases, polycystic ovary syndrome, metabolic syndrome, or any combinations thereof. In one embodiment, ectopic or visceral fat is metabolized at a rate 5% greater than subcutaneous fat. In other embodiments, ectopic or visceral fat is metabolized at a rate at least 10% 15%, 20%, 25%, 30% 50%, 60%, 70%, 80%, 90%, or 100% greater than subcutaneous fat.

In still another aspect, methods of the invention include the use of a therapeutically effective amount of a combination of anti-obesity agents administered in combination with glucocortico steroids. Glucocortico steroids have the adverse effect of increasing fat mass and decreasing lean mass. Accordingly, it is contemplated that the anti-obesity agent combination can be used in conjunction with glucocortico steroids under conditions where glucocortico steroid use is beneficial.

Also provided are methods to reduce weight in a morbidly obese subject by first reducing the subject's weight to a level below that of being morbidly obese, then administering to the subject a combination of anti-obesity agents in effective amounts to further reduce the subject's weight. Methods for reducing a subject's weight to below that of morbid obesity include reducing caloric intake, increasing physical activity, drug therapy, bariatric surgery, such as gastric bypass surgery, or any combinations of the preceeding methods. In one aspect, administering the combination of anti-obesity agents further reduces the weight of the subject. In another embodiment, methods are provided for reducing the body mass index in a subject having a body mass index of 40 or less by administering a combination of anti-obesity agents in effective amounts to further reduce the subject's weight.

By reducing weight it is meant that the subject loses a portion of bis/her total body weight over the course of treatment, whether the course of treatment be days, weeks, months or years. Alternatively, reducing weight can be defined as a decrease in proportion of fat mass to lean mass (in other words, the subject has lost fat mass, but maintained or gained lean mass, without necessarily a corresponding loss in total body weight). An effective amount of the anti-obesity agents administered in combination in this embodiment is an amount effective to reduce a subject's body weight over the course of the treatment, or alternatively an amount effective to reduce the subject's percentage of fat mass over the course of the treatment. In certain embodiments, the subject's body weight is reduced, over the course of treatment, by at least about 1%, by at least about 5%, by at least about 10%, by at least about 15%, or by at least about 20%. Alternatively, the subject's percentage of fat mass is reduced, over the course of treatment, by at least 1%, at least 5%, at least 10%, at least 15%, at least 20%, or at least 25%.

In certain embodiments, methods of reducing nutrient availability, e.g., reducing weight, in a subject comprise administering to the subject an effective amount of the anti-obesity agents in a bolus dose one or more times a day. A bolus dose is an intermittent dosage of medicine (as opposed to a continuous infusion). A subject can be administered one or more bolus doses per day. The bolus dose can be the same no matter when it is administered to the subject, or can be adjusted such that the subject is administered a larger bolus dose at certain times of the day as compared to others. Administration of an agent in certain formulations, e.g., sustained-release formulations, a bolus dose can be administered less frequently, for example, once every three days, once per week, twice a month, once every month. Furthermore, the time between bolus doses is preferably long enough to allow the drug administered in the previous bolus dose to clear the subject's blood stream.

In other embodiments, methods of reducing nutrient availability, e.g., reducing weight, in a subject comprise administering to the subject an effective amount of the anti-obesity agents in continuous doses. By continuous dose it is intended to mean the continuous infusion of the drug by, for example, intravenous injection or a transdermal patch. Alternatively, a continuous dose can be administered orally in the form of a controlled release capsule or tablet which releases the drug into the subject's system over a period of time. When administered by a continuous dose, the drug is released over a period of about 1 hour, in some cases the drug is released over a period of about 2, 3, 4, 5, 6, 7, 8, 9, 10, 11, 12, 18, or 24 hours.

By "administered in combination" is meant that the anti-obesity agents are administered as a single administration, simultaneously as separate doses, or as sequentially administered. Sequential administration refers to administering one of the anti-obesity agents either before or after an anti-obesity agent. In one embodiment, the first anti-obesity agent is administered about 30 minutes before or after the at least one other anti-obesity agent, in other embodiments about 1, 2, 3, 4, 5, 6, 7, 8, 9, 10, 11, or 12 hours before or after the at least one other anti-obesity agents. Any of the administered anti-obesity agents can be administered as a bolus dose or as a continuous dose.

The present invention is further directed to methods of increasing thermogenesis in a subject, the method comprising administering to a subject in need thereof an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both. Thermogenesis is the process of liberating calories as heat by increasing the body's metabolic rate. Thermogenesis is activated by mechanisms, including supplements, nutrition, exercise, and exposure to cold.

The present invention is yet further directed to methods of increasing oxidative metabolism in a subject, the method comprising administering to a subject in need thereof an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both. Oxidative metabolism is the process by which oxygen is used to make energy from carbohydrates (sugars).

In another aspect, a method of inducing a feeling of fullness in a subject is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In yet another aspect, a method of controlling hunger in a subject is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In yet a further aspect, a method of prolonging a feeling of satiation in a subject is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In yet a further aspect, a method of reducing caloric intake by reducing the size of a meal is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In another aspect, a method of controlling food intake is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In yet another aspect, a method for ensuring or assisting in compliance with a reduced calorie or restrictive diet is provided, wherein the method comprises administering an effective amount of at least one and-obesity agent that acts upon forebrain structures involved in food intake, body weight modulation, or both in combination with administration of at least one anti-obesity agent that acts upon hindbrain structures involved in food intake, body weight modulation, or both to said subject.

In a further aspect, a method of adjusting a subject's set point so that the body's propensity for homeostasis is adjusted to a healthier set point is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject.

In yet a further aspect, a method of maintaining weight loss or maintaining the weight lost is provided, wherein the method comprises administering an effective amount of at least one anti-obesity agent that acts upon forebrain structures involved in food intake, body weight modulation, or both in combination with administration of at least one anti-obesity agent that acts upon hindbrain structures involved in food intake, body weight modulation, or both to said subject In one embodiment of this aspect of the invention, the weight loss is maintained by re-setting the subject's set point.

Furthermore, in certain embodiments, administration of the anti-obesity agents in combination results in a synergistic effect in any of the methods described herein. In addition, in certain embodiments, administration of the anti-obesity agents in combination results in a lower dosage requirement for at least one of the agents, with the same effect.

In one embodiment, methods of the invention are of use in treating and/or preventing metabolic conditions or disorders that benefit from a reduction in nutrient availability. Accordingly, these methods may be useful in treating and/or preventing of obesity, diabetes (e.g., type 2 or non-insulin dependent diabetes, type 1 diabetes, and gestational diabetes), eating disorders, insulin-resistance syndrome, and cardiovascular disease.

In one embodiment, methods of use in altering fat distribution, reducing fat mass, or both in a subject are provided. Accordingly, subjects for whom altering body composition is of benefit can also benefit from the present methods. Altered body composition, as intended herein, includes loss or maintenance of body fat, with minimization of loss, maintenance, or gain of lean body mass. In such situations, weight may increase as well as decrease. Accordingly, subjects may be lean, overweight, or obese as these terms are generally used in the art. Methods of the invention may also include reducing fat in non-adipose tissue while sparing lean mass. Uses for this method include treating diseases such as nonalcoholic steatohepatitis (NASH) or lipodystrophy.

Methods described herein use the administration of at least one anti-obesity agent that acts upon forebrain structures involved in food intake, body weight modulation, or both in combination with administration of at least one anti-obesity agent that acts upon hindbrain structures involved in food intake, body weight modulation, or both for the control, prevention and/or treatment of such conditions or disorders.

In another aspect, is provided methods that stimulate food intake, promote body weight gain, or both through administration of agents that act on the forebrain and hindbrain. In such methods, weight-inducing agents are administered to a subject in combination and in amounts effective to stimulate food intake, promote weight gain or both in the subject. These methods are particularly beneficial for diseases and disorders like cachexia and anorexia, and other wasting diseases characterized by loss of appetite, diminished food intake, and body weight loss in a subject. Exemplary weight-inducing agents include NPY1 receptor agonists, NPY5 receptor agonists, leptin antagonists, MCH agonists, MC4 antagonists, cannabinoid receptor agonists, 5-HT2C antagonists, exendin antagonists, GLP-1 antagonists, ghrelin agonists, CCK antagonists, and amylin antagonists. Accordingly, one embodiment provides methods for stimulating food intake, promoting body weight gain or both in a subject in need thereof comprising administering to the subject at least two or more weight-inducing agents.

With regard to the administration of weight-inducing agents, the weight-inducing agents are administered as a single administration, simultaneously as separate doses, or as sequentially administered. Where separate dosage formulations are used, the individual weight-inducing agents can be administered at essentially the same time, *i.e*., concurrently, or at separately staggered times, *e.g*., sequentially prior to or subsequent to the administration of the other weight-inducing agent of the method. In one embodiment, the first weight-inducing agent is administered about 30 minutes before or after the at least one other weight-inducing agent, in other embodiments about 1, 2, 3, 4, 5, 6, 7, 8, 9, 10, 11, or 12 hours before or after the at least one other weight-inducing agents. In some embodiments, administration in combination involves administration of separate dosage formulations during overlapping intervals. For example, weight-inducing agent 1 is administered from day 1 through day 30 and weight-inducing agent 2 is administered from day 20 through day 50. In other embodiments, administration in combination involves administration of separate dosage formulations in sequential, nonoverlapping intervals. For example, weight-inducing agent 1 is administered from day 1 through day 30 and weight-inducing agent 2 is administered from day 35 through day 50. The instant invention is therefore to be understood to include all such regimes of simultaneous, alternating, or completely separate treatments over the total treatment course. Any of the administered weight-inducing agents can be administered as a bolus dose or as a continuous dose.

Furthermore, in certain embodiments, administration of the weight-inducing agents in combination results in a synergistic effect in any of the aspects of the invention. In addition, in certain embodiments, administration of the weight-inducing agents in combination results in a lower dosage requirement for at least one of the agents, with the same effect.

Anti-obesity agents for use in the present invention include leptin, leptin derivatives, recombinant leptin, and leptin agonists. Leptin (derived from Greek *leptos,* meaning thin) is a hormone produced predominantly by fat cells. In obese humans, leptin blood levels generally correlate with the amount of fat stored in the body. Generally, the greater the amount of fat, the greater the amount of leptin. Serum leptin levels concentrations in the majority of humans with obesity are high, and a state of leptin resistance is thought to exist (Mantzoros et al. (2000) J. Clin. Endocrinol. Metab. 85:4000-4002). Despite therapeutic attempts at using leptin to treat obesity, the effect of recombinant human leptin has been limited, if any, in causing weight loss in obese individuals. Exceptions to this include the treatment of individuals with congentital leptin deficiency and the treatment of individuals with lipoatrophy. See, for example, Heymsfield et al. (1999) JAMA 282:1568-1575, Farooqi et al. (1999) N. Engl. J. Med. 341:879-884, and U.S. Pat. Publication No. 2005/0020496.

In certain embodiments, leptin is administered in the form of replacement therapy so as to achieve near physiological concentrations of leptin in the plasma. It is estimated that the physiological replacement dose ofleptin is about 0.02 mg/kg of body weight per day for males of all ages, about 0.03 mg/kg of body weight per day for females under 18 years and about 0.04 mg/kg of body weight per day for adult females. When attempting to achieve near physiological concentrations of leptin, one may, for example, treat a subject with 50 percent of the estimated replacement dose for the first month of treatment, 100 percent of the replacement dose for the second month of treatment, 200 percent of the replacement dose for the third month of treatment, etc. Serum leptin levels can be measured by methods known in the art, including, for example, using commercially available immunoassays.

It is one aspect of the invention that fat is reduced by means such as administration of amylin to treat leptin resistance. Once leptin resistance is ameliorated (lessened), leptin can be administered to further treat obesity.

Leptin proteins and leptin protein containing compositions appropriate for use in the methods described herein are known in the art and include, but are not limited to recombinant human leptin (PEG-OB, Hoffman La Roche) and recombinant methiony human leptin (Amgen). Leptin proteins, analogs, derivatives, preparations, formulations, pharmaceutical compositions, doses, and administration routes have previously been described in the following patent publications and are hereby incorporated by reference in their entirety: U.S. Fat. Nos. 5,552,524; 5,552,523; 5,552,522; 5,521,283; and PCT Application Publication Nos. WO 96/05309, WO 96/40912; WO 977/06816, WO 00/20872, WO 97/18833, WO 97/38014, WO 98/08512, and WO 98/284427.

Leptin agonists and antagonists are known in the art and can also be found through a search of the patent databases. For example, leptin agonists are described in U.S. Pat. Publication Nos. 2004/0072219, 2003/049693, 2003/0166847, 2003/0092126, and U.S. Pat. Nos. 6,777,388 and 6,936,439. Leptin antagonists are described in U.S. Pat. PublicationNos. 2004/0048773, 2002/0160935 and U.S. Pat. No. 6,399,745. Means for testing for leptin aganism or antagonism are described in U.S. Pat. Nos. 6,007,998 and 5,855,098. These patent are exemplary and are incorporated herein by reference in their entirety.

Anti-obesity agents for use in the present invention also include amylin and amylin agonists. Amylin is a 37 amino acid peptide hormone that is co-secreted with insulin from pancreatic beta-cells in response to nutrient stimuli. Human amylin (hAmylin) has the following amino acid sequence:
Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr (SEQ ID NO:1). Rat amylin (rAmylin) has the following sequence:
KCNTATCATQRLANFLVRSSNNLGPVLPPTNVGSNTY (SEQ ID NO:2). The use of amylins from any species is contemplated.

It has surprisingly been found that modulation of effective amylin levels in vivo such as through the use of amylin, amylin agonists, and amylin antagonists, can modulate the effective levels of ghrelin in vivo.

Amylin agonists contemplated in the use of the invention include those described in U.S. Patent Nos. 5,686,411, 6,114,304, and 6,410,511, which are herein incorporated by reference in their entirety. Such compounds include those having the formula I:
¹Aₜ-X-Asn-Thr-⁹Ala-Thr-Y-Ala-Thr-¹⁰Gln-Arg-Leu-Bₜ-Asn-¹⁵Phe-Leu-Cₜ-Dₜ-Eₜ-²⁰Fₜ-Gₜ-Asn-Hₜ-Gly-²⁵Iₜ-Jₜ-Leu-Kₜ₋Lₜ-³⁰Thr-Mₜ-Val-Gly-Ser-³⁵Asn-Thr-Tyr-Z (SEQ ID NO:3)
   wherein Aₜ is Lys, Ala, Ser or hydrogen;
   Bₜ is Ala, Ser or Thr;
   Cₜ is Val, Leu or Ile;
   Dₜ is His or Arg;
   Eₜ is Ser or Thr;
   Fₜ is Ser, Thr, Gln or Asn;
   Gₜ is Asn, Glu or His;
   Hₜ is Phe, Leu or Tyr;
   Iₜ is Ala or Pro;
   Jₜ is Ile, Val, Ala or Leu;
   Kₜ is Ser, Pro, Leu, Ile or The
   Lₜ is Ser, Pro or Thr;
   Mₜ is Asn, Asp, or Gln;
   X and Y are independently selected amino acid residues having side chains which are chemically bonded to each other to form an intramolecular linkage; and
   Z is amino, alkylamino, dialkylamino, cycloalkylamino, arylamino, aralkylamino, alkyloxy, aryloxy ar aralkyloxy.

Suitable side chains for X and Y include groups derived from alkyl sulfhydryls which may form disulfide bonds; alkyl acids and alkyl amines which may form cyclic lactams; alkyl aldehydes or alkyl halides and alkylamines which may condense and be reduced to form an alkyl amine bridge; or side chains which may be connected to form an alkyl, alkenyl, alkynyl, ether or thioether bond. Preferred alkyl chains include lower alkyl groups having from about 1 to about 6 carbon atoms.

An additional aspect of the present invention is directed to agonist analogues of SEQ ID NO:3 which are not bridged, and wherein X and Y are independently selected from Ala, Ser, Cys, Val, Leu and Ile or alkyl, aryl, or aralkyl esters and ethers of Ser or Cys.

Biologically active derivatives of the above agonist analogs are also included within the scope of this invention in which the stereochemistry of individual amino acids may be inverted from (L)/S to (D)/R at one or more specific sites.

Also included within the scope of this invention are the agonist analogs modified by glycosylation of Asn, Ser and/or Thr residues.

Biologically active agonist analogs of amylin are included within the scope of this invention which contain less peptide character. Such peptide mimetics may include, for example, one or more of the following substitutions for --CO--NH-- amide bonds: depsipeptides (-CO-O-), iminomethylenes (-CH2 -NH-), trans-alkenes (-CH=CH-), beta-enaminonitriles (-C(=CH-CN)-NH-), thioamides (-CS-NH-), thiomethylenes (-S-CH2 - or -CH2 -S-), methylenes (-CH2 -C2 -) and retro-amides (-NH-CO-).

Compounds of this invention form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include, for example, ammonium salts, alkali metal salts (such as sodium and potassium salts) and alkali earth salts (such as calcium and magnesium salts). Acetate, hydrochloride, and trifluoroacetate salts are preferred.

Throughout the application, the amino acid sequences may be referred to as amino acids at position a to position b adjacent to a reference peptide. For example, 1-7 hAmylin refers to the amino acid sequence from position 1 to position 7, inclusive, of human amylin (SEQ ID NO:1), the reference peptide in this example. Modification to the reference peptide may be shown as the position of modification adjacent to the modification. For example, (2Asp 7Lys) 1-7 hAmylin represents the amino acid sequence at positions 1 to 7 of human amylin with a modification of the Cys to Asp at position 2 and a modification of the Cys to Lys at position 7. For another example, ¹⁸Arg^{25,28}Pro-h-amylin represents the amino acid sequence of human amylin with a modification of the His to Arg at position 18, a modification of the Ala to Pro at position 25, and a modification of the Ser to Pro at position 28.

Exemplary compounds include, but are not limited to des-¹Lys-h-amylin (SEQ ID NO:4), ²⁸Pro-h-amylin (SEQ ID NO:5), ^{25,28,29}Pro-h-amylin (SEQ ID NO:6), ¹⁸Arg^{25,28}Pro-h-amylin (SEQ ID NO:7), and des-¹Lys¹⁸Arg^{25,28}Pro-h-amylin (SEQ ID NO:8), all show amylin activity in vivo in treated test animals, (e.g., provoking marked hyperlactemia followed by hyperglycemia). In addition to having activities characteristic of amylin, certain of the preferred compounds of the invention have also been found to possess more desirable solubility and stability characteristics when compared to human amylin. Examples of these compounds include ²⁵Pro²⁶Va^{28,29} Pro-h-amylin (SEQ ID NO:9), ^{25,28,29}Pro-h-amylin (SEQ ID NO:10), and ¹⁸Are^{25,28}Pro-h-amylin (SEQ ID NO:7).

Other compounds include ¹⁸Arg^{25,28,29}Pro-h-amylin (SEQ ID NO:11), des-¹Lys¹⁸Arg^{25,28,29}Pro-h-amylin (SEQ ID NO:12), des-¹ Lys^{25,28,29}Pro-h-amylin (SEQ ID NO:13), ²⁵Pro²⁶Val^{28,29}Pro-h-amylin (SEQ ID NO:14), ²³Leu²⁵Pro²⁶Val^{28,29}Pro-h-amylin (SEQ ID NO:15), ²³Leu²⁵Pro²⁶Val²⁸Pro-h-amylin (SEQ ID NO:16), des-¹Lys²³Leu²⁵pro²⁶Val²⁸Pro-h-amylin (SEQ ID NO:17), ¹⁸Arg²³Leu²⁵Pro²⁶Val²⁸Pro-h-amylin (SEQ ID NO:18), ¹⁸Arg²³Leu^{25,28,29}Pro-h-amylin (SEQ ID NO:19), ¹⁸Arg²³Leu^{25,28}Pro-hamylin (SEQ ID NO:20), ¹⁷Ile²³Leu^{25,28,29}Pro-h-amylin (SEQ ID NO:21), ¹⁷Ile^{25,28,29}Pro-hamylin (SEQ ID NO:22), des-¹Lys¹⁷Ile²³Leu^{25,28,29}Pro-h-amylin (SEQ ID NO:23), ¹⁷Ile¹⁸Arg²³Leu-h-amylin (SEQ ID NO:24), ¹⁷Ile¹⁸Arg²³Leu²⁶Val²⁹Pro-h-amylin (SEQ ID NO:25), ¹⁷Ile¹⁸Arg²³Leu²⁵Pro²⁶Val^{28,29}Pro-h-amylin (SEQ ID NO:26), ¹³Thr²¹His²³Leu²⁶Ala²⁸Leu²⁹Pro³¹Asp-h-amylin (SEQ ID NO:27), ¹³Thr²¹His²³Leu²⁶Ala²⁹Pro³¹Asp-hamylin (SEQ ID NO:28), des-¹Lys¹³Thr²¹His²³Leu²⁶Ala²⁸Pro³¹Asp-h-amylin (SEQ ID NO:29), ¹³Thr¹⁸Arg²¹His²³Leu²⁶Ala²⁹Pro³¹Asp-h-amylin (SEQ ID NO:30), ¹³Thr¹⁸Arg²¹His²³Leu^{28,29}Pro³¹Asp-h-amylin (SEQ ID NO:31), and ¹³Thr¹⁸Arg²¹His²³Leu²⁵Pro²⁶Ala^{28,29}Pro³¹Asp-h-amylin (SEQ ID NO:32).

Useful amylin agonist analogs include those identified in PCT Application Publication No. WO 93/10146, the contents of which is also hereby incorporated by reference.

Amylin agonists useful in the invention may also include fragments of amylin and its analogs as described above as well as those described in EP 289287, the contents of which are herein incorporated by reference. Amylin agonists may also be compounds having at least 60, 65, 70, 75, 80, 85, 90, 95, or 99% amino acid sequence identity to SEQ ID NO:1 having amylin activity. Amylin agonists also include small molecules, non-peptide molecules, for example those based on small molecule chemistry. "Amylin activity" as used herein includes the ability of amylin to affect ghrelin levels in a body-Amylin agonists also include analogs of amylin having insertions, deletions, extensions and/or substitutions in at least one or more amino acid positions of SEQ ID NO:1. The number of amino acid insertions, deletions, or substitutions may be not more than 5, 10, 15, 20, 25, or 30. Insertions, extensions, or substitutions may be with other natural amino acids, synthetic amino acids, peptidomimetics, or other chemical compounds. Amylin agonists, as contemplated in the invention may also be calcitonin, such as teleost calcitonins, and their analogs, as well as calcitonin-gene-related peptides (CGRP) and their analogs.

Amylin agonists also include polypeptides (referred to herein as LHC (loop helix C-terminus) peptides) described in U.S. Patent Application No. 60/543,275 and in PCT application No. PCT/US2005/004631, filed Febrary 11, 2005, each of which is incorporated herein by reference, as well as their analogs and derivatives. The LHC peptides for use in the invention act as an agonist for at least one biological effect of calcitonin, amylin, CGRP, or any combination of the three herein disclosed or bind to at least one of the receptors of amylin, calcitonin, or CGRP. Receptor binding activity and biological activity of exemplary LHC peptides are described in U.S. Patent Application No. 60/543,275 and in PCT application No. PCT/US2005/004631. In a general aspect, these polypeptide agonists have at least a loop region of amylin or calcitonin and analogs thereof, an α helix region of at least a portion of an α helix region of calcitonin or analogs thereof or an α helix region having a portion of an amylin α helix region and a calcitonin α helix region or their respective analogs, and a C-terminal tail of amylin or calcitonin or analogs thereof, with the proviso that the C-terminal tail of calcitonin or a calcitonin analog is not proline (Pro), hydroxyproline (Hyp), homoserine (Hse) or derivatives of Hse.

In certain embodiments, these LHC peptides have an amylin or amylin analog loop region, at least a portion of a calcitonin or calcitonin analog α helix region, and an amylin or amylin analog C-terminal tail. In other embodiments, these LHC peptides have a calcitonin or calcitonin analog loop region, at least a portion of a calcitonin or calcitonin analog α helix region, and an amylin or amylin analog C-terminal tail. In still other embodiments, these LHC peptides have an amylin or amylin analog loop region, at least a portion of an amylin or amylin analog α helix region and at least a portion of a calcitonin or calcitonin analog α helix region, and an amylin or amylin, analog C-terminal tail. In yet other embodiments, these LHC peptides have a calcitonin or calcitonin analog loop region, at least a portion of an amylin or amylin analog α helix region and at least a portion of a calcitonin or calcitonin analog α helix region, and an amylin or amylin analog C-terminal tail. In still yet other embodiments, these LHC peptides have an amylin or amylin analog loop region, a portion or a calcitonin or calcitonin analog α helix region or at least a portion of an amylin or amylin analog α helix region and at least a portion of a calcitonin or calcitonin analog α helix region, and a calcitonin or calcitonin analog C-terminal tail.

In certain embodiments, the loop region of these LHC peptides may further comprise no more than one, two, three, or four modifications including substitutions, insertions, or deletions from the amylin or calcitonin loop, and analogs thereof. It is further contemplated that these LHC peptides may have additional modifications at the N-terminal portion of the loop comprising a N-cap region, that may have hydrophobic or hydrophilic characteristics such as acetyl, isocaproyl, 3,6-dioxyoctanoic acid, or 1-amino-4,7,10-trioxa-13-tridecanamine succinimic acid. Modifications may further include one, two, three or more additional amino acids. This is an area which allows for many modifications too numerous to mention, but would be understood by one of skill in the art based upon what is exemplified further in the present application.

These LHC peptides may also be further derivatized by chemical alterations such as amidation, glycosylation, acylation, sulfation, phosphorylation, acetylation, and cyclization. Such chemical alterations may be obtained through chemical or biochemical methodologies, as well as through *in vivo* processes, or any combination thereof. Derivatives of these LHC peptides may also include conjugation to one or more polymers or small molecule substituents. One type of polymer conjugation is linkage or attachment of polyethylene glycol ("PEG") polymers, polyamino acids (e.g., poly-his, poly-arg, poly-lys, etc.) and/or fatty acid chains of various lengths to the N- or C-terminus or amino acid residue side chains of the polypeptide. Small molecule substituents include short alkyls and constrained alkyls (e.g., branched, cyclic, fused, adamantyl), and aromatic groups. In addition, basic residues such as R and K may be replaced with homoR and homoK, citrulline, or ornithine to improve metabolic stability of the peptide. Polypeptides for use in the invention include acid as well as amide forms.

In certain embodiments, the α helix region of the LHC peptides comprise at least four consecutive amino acids of a calcitonin or calcitonin analog α helix region. In other embodiment, the α helix region comprises at least 5, 6, 7, or 8 consecutive amino acids of a calcitonin or calcitonin analog α helix region. In other embodiments, the α helix region comprises at least 10, 11, 12, 13, 14, 15, 16, 17, 18, 19, 20, 21 or more consecutive amino acids of a calcitonin or calcitonin analog α helix region. In certain embodiments, when the number of consecutive amino acids are less than 8, it is contemplated that the α helix region further comprises at least 4, 5, 6, 7, 9, 10, 11, or more consecutive amino acids of an amylin or amylin analog α helix region. In certain embodiments, it is envisioned that the less amino acids of calcitonin or calcitonin analog, the more amino acids of an amylin or amylin analog may be found in the α helix region of the novel compounds. The number of amino acids comprising the α helix region may be from about 10 to 23 amino acids. Accordingly, the α helix region may be 10, 11, 12, 13, 14, 15, 16, 17, 18, 19, 20, 21, 22, or 23 amino acids long. Moreover, the amino acids should provide for about three to about six α helical turns. It is further contemplated that the a helix region of the compounds may further comprise no more than one, two, three, four, five, six, seven, eight, nine or ten modifications including substitutions, insertions, or deletions from that of the calcitonin and/or amylin α helix region, and analogs thereof.

In certain embodiments, the C-terminal tail of the LHC peptides comprise at least the last six, five, or four amino acids of either amylin or calcitonin, and analogs thereof. In certain embodiments, the C-terminal tail of the novel compounds comprise at least a portion of the C-terminal end having a β turn. In certain embodiments, the β turn is introduced by the amino acid combination of Gly-Ser. Accordingly, the LHC peptides may have a C-terminal end comprising a portion of an amylin or calcitonin C-terminal tail (and analogs thereof) having Gly-Ser or starting at Gly-Ser.

In certain embodiments, the C-terminal tail of the LHC peptides may further comprise no more than one, two, or three, modifications including substitutions, insertions, or deletions from the amylin or calcitonin loop, and analogs thereof. It is further contemplated that the LHC peptides may have additional modifications at the C-terminal portion of the C-terminal tail which may include, for example, L-octylglycine, 4ABU (4-aminobutyric acid), 9Anc (9 amiononanoic acid), 3,6-dioxyoctanoic acid or 1-amino-4,7,10-trioxa-13-tridecanamine succinimic acid. Modification may further include one, two, three or more additional amino acids. The types of modification contemplated in this area would be understood by one of skill in the art based upon what is exemplified further in the present application.

In one aspect, a loop region is defined as that region found at the N-terminal end comprising at least 5 to 8 amino acids, wherein the first and last amino acid are capable of creating a bond, for example, residues at positions 2-7 of amylin or residues at positions 1-7 of calcitonin and their corresponding regions in their respective analogs. In another aspect, a α helix region is defined as the internal portion of amylin or calcitonin flanked by the loop region and the C-terminal tail which structurally forms an α helix, for example, residues at positions 8-23 of amylin or residues at positions 8-27 of calcitonin and their corresponding regions in their respective analogs. In yet another aspect, a C-terminal tail is defined as that region after the a helix, *e*.*g*., residues at positions 33-37 of amylin or longer such as residues at positions 27-37 or residues at positions 27 or 28 to 32 of calcitonin. Included in the LHC peptides are both the amide and acid forms of the disclosed compounds.

Amylin and calcitonin, as herein defined, includes all native and species variations. Examples of amylin and calcitonin include, but are not limited to:
human amylin (SEQ ID NO:1), rat amylin (SEQ ID NO:2), salmon calcitonin (sCT) CSNLSTCVLGKLSQELHKLQTYPRTNTGSGTP (SEQ ID NO:33), and
human calcitonin (hCT) CGNLSTCMLGTYTQDFNKFHTFPQTAIGVGAP (SEQ ID NO:34).

In a general aspect, the LHC peptides comprise at least a loop region, an α helix region, and a C-terminal tail. The loop region comprises an amino sequence comprising the formula (II) X-Xaa1 sequence-Y wherein X and Y are capable of creating a bond and are independently selected residues having side chains which are, or are capable of being, chemically bonded to each other to form an intramolecular linkage such as, for example, a disulfide bond; an amide bond; a cyclic lactam formed, for example, by an alkyl acid and an alkyl amine; an alkyl amine or imine bridge formed, for example, by condensing and reducing an alkyl aldehydes or alkyl halides and alkylamines; and an alkyl, alkenyl, alkynyl, ether or thioether bond formed, for example, by connection of side chains. Alkyl chains may include lower alkyl groups having from about 1 to about 6 carbon atoms. In certain embodiments, the intramolecular linkage may be a disulfide, amide, imine, amine, alkyl and alkene bond. In certain embodiments, X and Y of formula (II) are independently selected from Ser, Asp, Glu, Lys, Orn (ornithine), or Cys. In certain embodiments, X and Y of formula (II) are Cys and Cys. In other embodiments, X and Y of formula (II) are Ser and Ser. In still other embodiments, X and Y of formula (II) are Asp and Lys or Lys and Asp.

The Xaa1 sequence of formula (II) comprises an amino acid sequence of 3, 4, 5, or 6 amino acids between X and Y. In certain embodiments, the Xaa1 sequence comprises an amino acid sequence having a region with one or more substituted or unsubstituted hydroxyl-containing residues next to Y. For example, the hydroxyl containing residue region may have at least 2 of the 3 amino acids adjacent Y that are either a Ser or Thr. The other amino acids in the Xaa1 sequence may be any amino acid. In certain embodiments, the Xaa1 sequence is 3 amino acids. In other embodiments, the Xaa1 sequence is 4 amino acids. In still other embodiments, the Xaa1 sequence is 5 amino acids. In yet other embodiments, the Xaa1 sequence is 6 amino acids. Accordingly, Xaa1 of formula (II) can be represented by Xaa2-Xaa3-Xaa4-Xaa5-Xaa6-Xaa7 (SEQ ID NO:35). In certain embodiments, Xaa2, Xaa3, Xaa4, any two, or all three may absent. In certain embodiments, Xaa5, Xaa6, and Xaa7 comprise the hydroxy-containing residue region. As such, at least two of the three amino acids can be a Ser, Hse, Thr, allo-Threonine (alloThr), d-Threonine (d-Thr), or other unnatural analog thereof. Xaa2 can be any amino acid or absent, Xaa3 can be any amino acid or absent, Xaa4 can be any amino acid or absent, Xaa5 can be any amino acid if Xaa6 is a Ser or Thr and Xaa7 is a Ser or Thr, Xaa6 can be any amino acid if Xaa5 is a Ser or Thr and Xaa7 is a Ser or Thr, Xaa7 can be any amino acid if Xaa5 is Ser or Thr and Xaa6 is Ser or Thr. Accordingly, in certain embodiment, Xaa1 can be represented as Xaa2 absent, Xaa3 is Ala, Gly, Ser, Asp or absent, Xaa4 is Asn, Ala, Asp, Gly or absent; Xaa5 is Ala, Leu, Thr, or Ser; Xaa6 is Ala, Ser, or Thr; and Xaa7 is Ala, Ser, Val, Hse, (S)-2-amio-3-hydroxy-methylbutanoic acid (Ahb), (2S,3R)-2-amino-3hydroxy-methylpentanoic acid (Ahp), d-Thr, Thr, or a derivative thereof. In other embodiments Xaa1 can be represented as Xaa2 is absent, Xaa3 is Ser, Gly, or absent, Xaa4 is Asn or Asp, Xaa5 is Ala, Ser, Thr or Leu, Xaa6 is Ala, Thr or Ser, and Xaa7 is Ser, d-Thr, alloThr or Thr. In certain embodiments, the loop region of formula (II) comprises the above-described representations of Xaa1 wherein Xaa3 is Ala, wherein Xaa3 is Ser or wherein Xaa3 is Gly. Alternatively or additionally, the loop region comprises the above described representations of Xaa1 wherein Xaa4 is Ala, wherein Xaa4 is Asn, wherein Xaa4 is Asp, or wherein Xaa4 is Gly. Alternatively or additionally, the loop region comprises the above-described representations of Xaa1 wherein Xaa5 is Ala, wherein Xaa5 is Thr, or wherein Xaa5 is Leu. Alternatively or additionally, the loop region comprises the above described representations of Xaa1 wherein Xaa6 is Ser or wherein Xaa6 is Ala. Alternatively or additionally, the loop region comprises the above-described representations of Xaa1 wherein Xaa7 is Thr or wherein Xaa7 is d-Thr. It is further contemplated that no more than one, two, or three modifications such as substitutions, insertions, deletions, and/or derivatizations may be made to the loop region.

Examples of the loop region of the invention include, but are not limited to, CNTATC (SEQ ID NO:36); CATATC (SEQ ID NO:37); CDTATC (SEQ ID NO:38); CGTATC (SEQ ID NO:39); CNAATC (SEQ ID NO:40); CNTSTC (SEQ ID NO:41); CNTA-dThr-C (SEQ ID NO:42); CNTA-T(OPO₃H₂)-C (SEQ ID NO:43); CNTASC (SEQ ID NO:44); CNTAAC (SEQ ID NO:45); CNTAVC (SEQ ID NO:46); CNTA-Hse-C (SEQ ID NO:47); CNTA-Ahb-C (SEQ ID NO:48); CNTA-Ahp-C (SEQ ID NO:49); CSNLSTC (SEzQ ID NO:50); CGNLSTC (SEQ ID NO:51); CANLSTC (SEQ ID NO:52); CSALSTC (SEQ ID NO:53); CSNASTC (SEQ ID NO:54); CSNLATC (SEQ ID NO:55); and CSNLSAC (SEQ ID NO:56). As previously noted, it is further contemplated that no more than one, two, or three modifications such as substitutions, insertions, deletions, and/or derivatizations may be made to the loop region.

The loop region of the LHC peptides may further comprise modifications or additional amino acids at the N-terminal end. Such modifications include the addition of compounds such as Lys, Ala, Phe, Ile, Ser, Octylglycine, Isocap, Fmoc-3,6-dioxyoctanoic acid, Fmoc-1-amino-4,7,10-trioxa-13-tridecanamine succinimic acid, acetyl, and/or groups for solubility, delivery, signaling. Exemplary modified loops include the addition of Lys to the sequence of Xaa1 or the addition of Ile to the sequence of Xaa1. For example, the modified loop region may be KCNTATC (SEQ ID NO:57). In certain embodiments, the additions and/or modifications at the N-terminal end of the loop region may change the loop region. For example, the loop region may be modified as follows: cyclo(2,7) 1-7 hAmylin, cyclo(²Asp ⁷Lys) 1-7 hAmylin, N-isocaproyl 1-7 hAmylin, N-3,6 dioxaoctanoyl 1-7 hAmylin, L-actylglycine 1-7 hAmylin, Acetyl (²Agy, ⁷Agy) 1-7 hAmylin wherein Agy is Allylglycine, Acetyl (¹Ala) 1-7 hAmylin, (¹Thr ³Asp) 1-7 hAmylm, Isocap (⁷Ala) 5-7 sCT, Acetyl (²Agy, ⁷Agy) 1-7 sCT, and cyclo (1,7) (¹Asp ⁷Lys) 1-7 sCT. Therefore, taking the example of Isocap (⁷Ala) 5-7 sCT, certain embodiments comprise a modification at the N-terminal region of the loop region such that amino acids Xaa2 to Xaa5 are absent.

The α helix region of the LHC peptides may be about 8 to 23 amino acids in length. In certain embodiments, the α helix region is amphiphatic. In certain embodiments, the α helix region comprises about 3 to 6 helical turns. In certain embodiments, the α helix region comprises 3, 4, 5, or 6 helical turns. In other embodiments, the α helix region is a rigid structure equivalent to about 3, 4, 5, or 6 helical turns. An example of an idealized helix is LLQQLQKLLQKLKQY (SEQ ID NO:58). In certain embodiments, the α helix is an ampliphatic structure. Accordingly, characteristics of desirable amino acids that would provide this type of structure may be selected.

It has been found that the calcitonin α helix region, a combination of an amylin and a calcitonin α helix region, or parts thereof, and/or some CGRP elements are desirable in the α helix region of the LHC peptides. It is contemplated that, as with the loop region, the α helix region can be from any amylin or calcitonin, and analogs thereof. Accordingly, in certain embodiments, the α helix region is at least a portion of an α helix region of a calcitonin or calcitonin analog. In other embodiments, the α helix region is at least a portion of an α helix region of a calcitonin or calcitonin analog and at least a portion of an α helix of an amylin or amylin analog. In still other embodiments, the α helix region of the LHC peptides contain elements of CGRP. It is further contemplated that novel compounds may have no more than one, two, three, four, five, six, seven, eight, nine, or ten further modifications such as substitutions, insertions, deletions, and/or derivatizations.

In certain embodiments, the α helix region of the LHC may comprise a helix region type I. An α helix region type I comprises amino acids from position 8 of sCT to position 18, 19, 20, 21, 22, 23, 24, 25, 26, or 27 of sCT. Moreover, the α helix region type I may comprise more than one portion of a calcitonin or calcitonin analog α helix region of the same or different species, for example 8-21 sCT 19-27 sCT; 8-21 sCT 18-27 sCT; or 8-16 hCT 17-27 sCT, or (¹¹Arg) 8-16 hCT (¹⁸Arg) 17-27 sCT. Alternatively or additionally, the above described α helix of 8-18 sCT to 8-27 sCT may further comprise the substitutions of one or more of (¹⁰Aib), (¹¹Arg), (¹¹Orn), (¹¹hArg), (¹¹Cit), (¹¹hLys), (¹¹Lys(for)), (¹⁷Aib), (¹⁸Arg), (¹⁸Orn), (¹⁸hATg), (¹⁸Cit), (¹⁸hLys), (¹⁸Lys(for)), (¹⁸Lys(PEG5000)), (²²Leu), ²⁴Pro) or any combination thereof.

In one embodiment, an α helix region type I of the LHC peptides can be represented by: X1 V L Xaa10 Xaa11 L S Q Xaa15 L Xaa17 Xaa18 L Q T Xaa22 P Xaa24 T N T X1 (SEQ ID NO:59), wherein
Xaa10 is Gly or Aib;
Xaa11 is Lys, Arg, Orn, Arg, Cit, hLys, or Lys(for);
Xaa15 is Glu or Phe;
Xaa17 is His or Aib;
Xaa18 is Lys, Arg, Orn, hArg, Cit, hLys,Lys(for), Lys(PEG 5000);
Xaa22 is Try or Leu;
Xaa24 is Arg or Pro; or
X1 is absent or comprises 1-4 additional amino acids.

It should be remembered that each member of the Markush group, or a combination thereof, is another embodiment of the invention and is not to be read as a single unit. This is a shorthand method for stating, as an example, embodiments of the LHC peptides include an α helix region type I Formula where, Xaa18 can be a Lys, Arg, Orn, hArg, Cit, hLys, or Lys(for), and each variation is a separate embodiment of the invention. Accordingly, the α helix region type I formula has one embodiment where Xaa18 is Lys. It has another embodiment where Xaa18 is Arg, and so on. It is further contemplated that the a helix region may contain no more than one, two, three, four, five, six, seven, eight, nine, or ten modifications such as substitutions, insertions, deletions, and/or derivatizations. Accordingly, the compounds of α helix region type I may have further deletions at the C-terminal end. In certain embodiments, the amino acids of X1 are capable of forming an α helix turn.

Example of an α helix region type I of the LHC peptides include, but are not limited to, 8-18 sCT, 8-21 sCT, 8-24 sCT, 8-27 sCT, (¹¹Arg) 8-18 sCT, (¹⁸Arg) 8-18 sCT, (¹¹Arg ¹⁸Arg) 8-18 sCT, (¹¹Orn ¹⁸Orn) 8-18 sCT, (¹¹Arg ¹⁸Cit) 8-18 sCT, (¹¹hArg ¹⁸hArg) 8-18 sCT, (¹¹Arg ¹⁸Orn) 8-18 sCT, (¹¹Cit ¹⁸Arg) 8-18 sCT, (¹¹Cit ¹⁸Cit) 8-18 sCT, (¹¹hLys ¹⁸hLys) 8-18 sCT, (¹⁰Aib ¹¹Arg ¹⁷Aib ¹⁸Arg) 8-18 sCT, (¹¹Lys(for) ¹⁸Lys(for)) 8-18 sCT, (¹⁰Aib ¹¹Lys(for)¹⁷Aib ¹⁸Lys(for)) 8-18 sCT, (¹¹Arg ¹⁸Lys(PEG 5000)) 8-18 sCT, (¹¹Arg) 8-21 sCT, ¹⁸Arg) 8-21 sCT, (¹¹Arg ¹⁸Arg) 8-21 sCT, (¹¹Orn ¹⁸Orn) 8-21 sCT, (¹¹Arg ¹⁸Cit) 8-21 sCT, (¹¹hArg ¹⁸hArg) 8-21 sCT, (¹¹Arg ¹⁸Orn) 8-21 sCT, (¹¹Cit ¹⁸Arg) 8-21 sCT, (¹¹Cit ¹⁸Cit) 8-21 sCT, (¹¹hLys ¹⁸hLys) 8-21 sCT, (¹⁰Aib ¹¹Arg ¹⁷Aib ¹⁸Arg) 8-21 sCT, (¹¹Lys(for) ¹⁸Lys(for)) 8-21 sCT, (¹⁰Aib ¹¹Lys(for) ¹⁷Aib ¹⁸Lys(for)) 8-21 sCT, (¹¹Arg ¹⁸Lys(PEG 5000)) 8-21 sCT, (¹¹Arg) 8-24 sCT, (¹⁸Arg) 8-24 sCT, (¹¹Arg ¹⁸Arg) 8-24 sCT, (¹¹Arg ¹⁸Arg ²²Leu) 8-24 sCT, (¹¹Arg ¹⁸Arg ²⁴Pro) 8-24 sCT, (¹¹Orn ¹⁸Orn) 8-24 sCT, (¹¹Arg ¹⁸Cit) 8-24 sCT, (¹¹hArg ¹⁸hArg) 8-24 sCT, (¹¹Arg ¹⁸Orn) 8-24 sCT, (¹¹Cit ¹⁸Arg) 8-24 sCT, (¹¹Cit ¹⁸Cit) 8-24 sCT, (¹¹hLys ¹⁸hLys) 8-24 sCT, (¹⁰Aib ¹¹Arg ¹⁷Aib ¹⁸Arg) B-24 sCT, (¹¹Lys(for) ¹⁸Lys(for)) 8-24 sCT, (¹⁰Aib ¹¹Lys(for) ¹⁷Aib ¹⁸Lys(for)) 8-24 sCT, (¹¹Arg ¹⁸Lys(PEG 5000)) 8-24 sCT, (¹¹Arg) 8-27 sCT, ¹⁸Arg) 8-27 sCT, (¹¹Arg ¹⁸Arg) 8-27 sCT, (¹¹Arg ¹⁸Arg ²²Leu) 8-27 sCT, (¹¹Arg ¹⁸Arg ²⁴Pro) 8-27 sCT, (¹¹Om ¹⁸Orn) 8-27 sCT, (¹¹Arg ¹⁸Cit) 8-27 sCT, (¹¹hArg ¹⁸hArg) 8-27 sCT, (¹¹Arg ¹⁸Orn) 8-27 sCT, (¹¹Cit ¹⁸Arg) 8-27 sCT, (¹¹Cit ¹⁸Cit) 8-27 sCT, (¹¹hLys ¹⁸hLys) 8-27 sCT, (¹⁰Aib ¹¹Arg ¹⁷Aib ¹⁸Arg) 8-27 sCT, (¹¹Lys(for) ¹⁸Lys(for)) 8-27 sCT, (¹⁰Aib ¹¹Lys(for) ¹⁷Aib ¹⁸Lys(for)) 8-27 sCT, (¹¹Arg ¹⁸Lys(PEG 5000)) 8-27 sCT, (¹¹Arg ¹⁸Arg) 8-21 sCT-19-27 sCT, and (¹¹Arg ¹⁸Arg) 8-21 sCT-(¹⁸Leu) 18-27 sCT.

In certain embodiments, the α helix region of the LHC peptides may comprise α helix region type II. An α helix region type II comprises a portion of an α helix region of an amylin or amylin analog and a portion of an α helix region of a calcitonin or calcitonin analog. The α helix region type II may comprise amino acids from position 8 of hAmylin to 11, 12, 13, 14, 15, 16, 17, 18 or 19 of hAmylin and amino acids from position 13, 14, 15, 16, 17, 18, and 19 of sCT to position 18, 19, 20, 21, 22, 23, 24, 25, 26, or 27 of sCT. Alternatively or additionally, the above described α helix region of amylin and calcitonin may further comprise the substitutions of one or more of (⁸Val), (⁹Leu), (⁹Met), (¹⁰Gly), (¹⁰His), (¹²Thr), (¹³Tbr), (¹³Asn), (¹³Phe), (¹³Tyr), (¹⁴Arg), (¹⁴Ala), (¹⁴Asp), (¹⁴Gln), (¹⁴Gln), (¹⁴Thr), (¹⁴Gly), (¹⁵Leu), (¹⁵Ser), (¹⁵Glu), (¹⁵Ala), (¹⁵Tyr), (¹⁶Asp), (¹⁷Ser), (¹⁷Phe), (¹⁸Arg), (¹⁷Aib), (¹⁸Arg), (¹⁸Orn), (¹⁸hArg), (¹⁸Cit), (¹⁸hLys), (¹⁸Lys(for)), (¹⁸Lys(PEG5000)), (¹⁹Phe), (²⁰His), (²¹Asn), (²²Met), (²²Val), (²²Phe), (²²Leu), (²⁴Pro), or any combination thereof. In certain embodiments, the number of amino acids in the α helix region type II of the LHC peptides is at least 10 amino acids. In other embodiments, the number of amino acids in the α helix region type II of the LHC peptides is 11, 12, 13, 14, 15, 16,17,18,19,20,21,22, or 23. In other embodiments, the number of amino acids in the α helix region type II of the LHC peptides is 24 or more.

In one embodiment, an α helix region type II of the LHC peptides can be represented by: X1 Xaa8 Xaa9 Xaa10 R Xaa12 Xaa13 Xaa14 Xaa15 Xaa16 Xaa17 Xaa18 Xaa19 Xaa20 Xaa21 Xaa22 P Xaa24 T N T X1 (SEQ ID NO:60) wherein
Xaa8 is Ala or Val;
Xaa9 is Thr, Met or Leu;
Xaa10 is Gln, Gly, His;
Xaa12 is Leu, or Thr;
Xaa13 is Ala, Thr, Asn, Phe, Tyr, Ser, or Thr;
Xaa14 is Asn, Arg, Ala, Asp, Glu, Gln, Thr, or Gly;
Xaa15 is Phe, Leu, Ser, Glu, Ala, Asp, or Tyr;
Xaa16 is Leu or Asp;
Xaa17 is Val, His, Ser, Phe, or Aib;
Xaa18 is His, Arg, Lys, Orn, hArg, Cit, hLys, Lys(for), or Lys(PEG5000);
Xaa19 is Leu, Ser or Phe;
Xaa20 is Gln or His;
Xaa21 is Thr or Asn;
Xaa22 is Tyr, Val, Phe,Leu or Met;
Xaa24 is Arg or Pro; and
X1 is absent or comprises 1-4 additional amino acids.

Again, each member in the Markush group, or a combination thereof, is another embodiment of the invention and is not to be read as a single unit. It is further contemplated that the α helix region type II may contain no more than one, two, three, four, five, six, seven, eight, nine, or ten modifications such as substitutions, insertions, deletions, and/or derivatizations of the compounds described herein. For example, in certain embodiments, the α helix region type II may have deletions at the C-terminal end resulting in the deletion of position 27, 26, 25, 24, or 22. In other embodiments, however, the deletions do not remove amino acids of positions 19, 20,21, or 22.

Examples of an α helix region of type II of the LHC peptides include, but are not limited to, (⁸Val ⁹Leu ¹⁰Gly) 11-15 hAmylin 16-27 sCT, (⁸Val ⁹Leu ¹⁰Gly) 11-15 hAmylm (¹⁸Arg) 16-27 sCT, 8-12 hAmylin (¹⁸Arg) 13-27 sCT, 8-18 hAmylin 19-23 sCT, 8-18 HAmylin 19-27 sCT, (¹⁵Glu ¹⁸Arg) 8-18 hAmylin 19-24 sCT, (¹⁴Arg ¹⁵Ser) 8-18 hAmylin 19-22. sCT, (¹³Ala ¹⁴Ala ¹⁵Ala) 8-1 hAmylin 19-27 sCT, (¹³Ala ¹⁴Asp ¹⁵Ala) 8-18 hAmylin 19-22 sCT, (¹³Ala ¹⁴Asp) 8-18 hAmylin 19-23 sCT, (¹³Ala ¹⁴Asp) 8-18 hAmylin 19-27 sCT, (¹³Ala ¹⁴Ala) 8-18 hAmylin 19-22 sCT, (¹³Ala ¹⁴Glu) 8-18 hAmylin 19-22 sCT, (¹³Thr ¹⁴Asp ¹⁵Tyr) 8-18 hAmylm 19-22 sCT, (¹³Ala ¹⁴Gln) 8-18 hAmylin 19-22 sCT, (¹³Am ¹⁴Glu ¹⁵Tyr) 8-18 hAmylin 19-27 sCT, (¹³Phe ¹⁴Asp) 8-18 hAmylin 19-27 sCT, (¹³Ala ¹⁴Asp) 8-18 hAmylin (¹⁵Glu ¹⁸Arg) 8-18 hAmylin 19-24 sCT, (¹⁹Phe ²²Phe) 19-27 sCT, (¹³Ala ¹⁴Asp) 8-18 hAmylin (¹⁹Phe ²⁰His ²²Phe) 19-27 sCT, (¹³Ala ¹⁴Asp) 8-18 hAmylin (¹⁹Phe ²²Phe) 19-27 sCT, (⁹Thr ¹⁰His) 8-18 hAmylin 19-22 sCT, (⁹Thr ¹⁰His ¹⁴Gly ¹⁵Leu ¹⁷Ser ¹⁸Arg) 8-19 hAmylin 20-23 sCT, 8-18 hAmylin (²¹Asn ²²Phe ²³Val) 19-23 sCT, 8-18 hAmylin (²²Met) 19-27 sCT, 8-18 hAmylin (²²Val) 19-27 sCT, (⁹Met ¹²Thr ¹³Tyr ¹⁴Thr ¹⁵Glu ¹⁶Asp ¹⁷Phe) 8-17 hAmylin (¹⁸Arg) 18-20 sCT). In other embodiments, novel compound include variations of the above exemplary compounds with the α helix terminating at corresponding to 22, 23, 24, 25, 26 or 27 of sCT. In other words, compound 8-1 hAmylin 19-24 sCT is also specifically described as this compound is merely 8-18 hAmylin 19-27 sCT described above truncated to position 24. As another example, compound (13Ala 14Asp 15Ala) 8-18 hAmylin 19-23 is specifically described because of the above language applied to (13Ala 14Asp 15Ala) 8-18 hAmylin 19-22.

In certain embodiments, the C-terminal tail of the LHC peptides comprises amino acids from position 27, 28, 29, 30, 31, 32, or 33 to position 36 or 37 of hAmylin. In other embodiments, the C-terminal tail of the LHC peptides comprises amino acids from position 27 or 28 to position 32 of sCT; however, when the loop region is from a calcitonin or calcitonin analog and the α helix region is from a calcitonin or calcitonin analog, the last position of the C-terminal tail is not Pro, Hyp, Hse or derivatives of Hse. Alternatively or additionally, the above described a helix of amylin and calcitonin may further comprise the substitutions of one or more of (²⁷Tyr) hAmylin, (²⁹Arg) hAmylin, (³²Val) hAmylin, (³²Thr) hAmylin, (³⁴Glu) hAmylin, (³⁵Lys) hAmylin, (³⁶Phe) hAmylin, (³⁶Ala) hAmylin, (³⁷Phe) hAmylin, (³⁰Asn) sCT, (³²Tyr) sCT, or any combination thereof.

In one embodiment, a C-terminal tail of the LHC peptides can be represented by Xaa28 Xaa29 Xaa30 Xaa31 Xaa32 Xaa33 G Xaa35 Xaa36 Xaa37 Xaa38 (SEQ ID NO:61), wherein
Xaa28 is Lys, Tyr, or absent;
Xaa29 is Ser, Pro, or absent;
Xaa30 is Ser, Pro, Arg, or absent;
Xaa31 is Thr, or absent;
Xaa32 is Asn or absent;
Xaa33 is Val, Thr, or absent;
Xaa35 is Ser, Glu
Xaa36 is Asn, Lys, or Gly;
Xaa37 is Thr, Phe, or Ala;
Xaa38 is Tyr, Phe, Pro, or absent;
with the proviso that when the loop region of the LHC agonist is from a calcitonin or calcitonin analog and the α helix region is from a calcitonin or calcitonin analog, the last position of the C-terminal tail is not Pro, Hyp, Hse or derivatives of Hse.

Again, each member of the Markush group, or a combination thereof, is another embodiment of the invention and is not to be read as a single unit. It is further contemplated that the C-terminal tail may contain no more than one, two, or three modifications such as substitutions, insertions, deletions, and/or derivatizations of the compounds described herein.

Examples of the C-terminal tail of an LHC agonist include, but are not limited to, 27-37 rAmylin, (²⁷Tyr ²⁹Arg ³²Thr) 27-37 rAmylin, (²⁹Arg ³²Thr) 28-37 rAmylin, 30-37 hAmylin, ³²Thr) 30-37 hAmylin, (³⁵Lys ³⁶Ala ³⁷Phe) 30-37 hAmylin, 30-36 hAmylin, (³²Val) 30-36 hAmylin, (³⁴Glu ³⁶Phe) 30-36 hAmylin, 31-37 hAmyin, 31-36 hAmylin, 33-36 hAmylin, 33-37 hAmylin, 28-32 sCT, (³⁰Asn ³²Tyr) 28-32 sCT, and 27-32 sCT. In other embodiments, the C-terminal tail comprises the amino acid sequence KSNFVPTN (SEQ ID NO:62) or SNFVPTNV (SEQ ID NO:53),

It is further contemplated that no more than one, two, or three modifications such as substitutions, insertions, deletions, and/or derivatizations may be made to the C-terminal tail of the invention as described in the preceding paragraphs. The C-terminal tail of the LHC peptides may further comprise modifications or additional amino acids at the C-terminal end. Such modifications include the addition of compounds such as Lys, up to 4 Lys, L- Octylglycine, 4ABU (4-Aminobutyric acid), 9Anc (9-Amiononanoic acid), and/or groups for solubility, stability, or delivery. Examples include, but are not limited to, 33-37 hAmylin L-octylglycine, 33-37 hAmylin 4ABU, and 33-37 hAmylin 9Anc.

In a general aspect, LHC peptides for use in the invention comprise
(a) any of the LHC agonist loop regions as described herein;
(b) any of the LHC agonist α helix regions as described herein; and
(c) any LHC agonist C-terminal tails as described herein, with the proviso that when the loop region is from a calcitonin or calcitonin analog and the α helix region is from a calcitonin or calcitonin analog, the last position of the C-terminal tail is not Pro, Hyp, Hse or derivatives of Hse.

In another general aspect, LHC peptides for use in the invention comprise
(a) a loop region comprising formula (II) Xaa1 or Xaa1 with modifications at the N-terminal end;
(b) an α helix region comprising the α helix region type I or type II;
(c) a C-terminal tail represented by SEQ ID NO:61, with the proviso that when the loop region is from a calcitonin or calcitonin analog and the α helix region is from a calcitonin or calcitonin analog, the last position of the C-terminal tail is not Pro, Hyp, Hse or derivatives of Hse. The C-terminal end may comprise further modifications.

In yet another aspect, LHC peptides for use in the invention comprise an amino acid sequence of formula (III): Xaa1 X Xaa3 Xaa4 Xaa5 Xaa6 Y Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 Xaa23 Xaa24 Xaa25 Xaa25 Xaa27 Xaa28 Xaa29 Xaa30 Xaa31 Xaa32 (SEQ ID NO:64) wherein Xaa1 is A, C, hC, D, E, F, I, L, K, hK, R, hR, S, Hse, T, G, Q, N, M, Y, W, P,
Hyp, H, V or absent;
Xaa3 is A, D, E, N, Q, G, V, R, K, hK, hR, H, I, L, M, or absent;
Xaa4 is A, I, L, S, Hse, T, V, M, or absent;
Xaa5 is A, S, T, Hse, Y, V, I, L, or M;
Xaa6 is T, A, S, Hse, Y, V, I, L, or M;
Xaa8 is A, V, I, L, F, or M;
Xaa9 is L, T, S, Hse, V, I, or M;
Xaa10 is G, H, Q, K R, N, hK, or hR;
Xaa11 is K, R, Q, N, hK, hR, or H;
Xaa12 is L, I, V, F, M, W, or Y;
Xaa13 is A, F, Y, N, Q, S, Hse, orT;
Xaa14 is A, D, E, G, N, K, Q, R, H, hR, or hK;
Xaa15 is A, D, E, F, L, S, Y, I, V, or M;
Xaa16 is L, F, M, V, Y, or I;
Xaa17 is H, Q, N, S, Hse, T, or V;
Xaa18 is K, hK, R, hR, H, u (Cit), or n (Orn);
Xaa19 is F, L, S, Hse,V, I, T, or absent;
Xaa20 is H, R, K, hR, hK, N, Q, or absent;
Xaa21 is T, S, Hse, V, I, L, Q, N, or absent;
Xaa22 is F, L, M, V, Y, or I;
Xaa23 is P or Hyp;
Xaa24 is P, Hyp, R,K, hR, hK, or H;
Xaa25 is T, S, Hse, V, I, L, F, or Y;
Xaa26 is N,Q, D, or E;
Xaa27 is T, V, S, F, I, or L;
Xaa28 is G or A; Xaa29 is S, Hse, T, V, I, L, or Y;
Xaa30 is E, G, K, N, D, R, hR, hK, H, or Q;
Xaa31 is A, T, S, Hse, V, I, L, F, or Y; and
Xaa32 is F, P, Y, Hse, S, T, or Hyp;
wherein X and Y are capable of creating a bond and are independently selected residues having side chains which are chemically bonded to each other to form an intramolecular linkage such as disulfide bonds; amide bond; alkyl acids and alkyl amines which may form cyclic lactams; alkyl aldehydes or alkyl halides and alkylamines which may condensed and be reduced to form an alkyl amine or imine bridge; or side chains which may be connected to form an alkyl, alkenyl, alkynyl, ether or thioether bond. Alkyl chains may include lower alkyl groups having from about 1 to about 6 carbon atoms. In certain embodiments, the intramolecular linkage may be a disulfide, amide, imine, amine, alkyl and alkene bond. In certain embodiments, X and Y are independently selected from Ser, Asp, Glu, Lys, Orn, or Cys. In certain embodiments, X and Y are Cys and Cys. In other embodiments, X and Y are Ser and Ser. In still other embodiments, X and Y are Asp and Lys or Lys and Asp.

In yet another aspect, LHC peptides for use in the invention comprise an amino acid sequence of formula (IV): Xaa1 Xaa2 Xaa3 Xaa4 Xaa5 Xaa6 Xaa7 Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 P Xaa24 T N Xaa27 G S Xaa30 Xaa31 Xaa32 (SEQ ID NO.65) wherein
Xaa1 is A, C, D, F, I, K, S, T, or absent;
Xaa2 is C, D, S, or absent;
Xaa3 is A, D, N, or absent;
Xaa4 is A, L, T, or absent;
Xaa5 is A or S;
Xaa6 is T, A, S, or V;
Xaa7 is C, K, or A;
Xaa8 is A, V, L, or M;
Xaa9 is L or T;
Xaa10 is G, H, or Q;
Xaa11 is K, R, Q, or hArg;
Xaa12 is L, W, or Y;
Xaa13 is A, F, N, Q, S, or T;
Xaa14 is A, D, E, G, N, K, Q, or R;
Xaa15 is A, D, E, F, L, S, or Y;
Xaa16 is L, or F;
Xaa17 is H, Q, S, or V;
Xaa18 is K, R, hArg, u (Cit), or n (Orn);
Xaa19 is F, L, S, or absent;
Xaa20 is H, Q, or absent;
Xaa21 is T, N, or absent;
Xaa22 is F, L, M, V, or Y;
Xaa24 is P or R;
Xaa27 is T or V;
Xaa30 is E, G, K, or N;
Xaa31 is A or T; and
Xaa32 is F, P, or Y.

In yet another aspect, LHC peptides for use in the invention comprise an amino acid sequence of formula (V): Xaa1 Xaa2 Xaa3 Xaa4 Xaa5 T Xaa7 Xaa8 Xaa9 Xaa10 Xaa11 L Xaa13 Xaa14 Xaa15 L Xaa17 Xaa18 Xaa19 Xaa20 Xaa21 Xaa22 P Xaa24 TN Xaa27 G S Xaa30 Xaa31 Xaa32, (SEQ ID NO:66) wherein
Xaa1 is A, C, F, I, K, S, or absent;
Xaa2 is C, D, or S;
Xaa3 is A, D or N;
Xaa4 is A, L or T;
Xaa5 is A or S;
Xaa7 is C or K;
Xaa8 is A or V;
Xaa9 is L or T;
Xaa10 is G, H, or Q;
Xaa11 is K, R, or hArg;
Xaa13 is A, F, N, S, or T;
Xaa14 is A, D, E, G, N, Q, or R;
Xaa15 is A, E, F, L, S, or Y;
Xaa17 is H, S, or V;
Xaa18 is K, R, hArg, u (Cit), or n (Orn);
Xaa19 is F, L, or S;
Xaa20 is H or Q;
Xaa21 is T or N;
Xaa22 is F, L, M, V, or Y;
Xaa24 is P or R;
Xaa27 is T, or V;
Xaa30 is E, G, K, or N;
Xaa31 is A, or T; and
Xaa32 is F, P, or Y.

In a general aspect, the sequence of formula (III), (IV), or (V) further comprises 1, 2, 3, 4, 5, 6, 7, S, 9, 10, 11, 12, or more modifications of substitutions, insertions, deletions, elongations and/or derivatizations. In certain embodiments, the sequence of formula (III), (IV), or (V) comprises a Val inserted between amino acids at positions 22 and 23. In other embodiments, the sequence of formula (III), (IV), or (V) comprises a Gln inserted between positions 22 and 23. In still other embodiments, the sequence of formula (III), (IV), or (V) comprises a sequence of Gln-Thr-Tyr between positions 22 and 23. In yet other embodiments, the sequence of formula (III), (IV), or (V) comprises a sequence of Leu-Gln-Thr-Tyr (SEQ ID NO:67) between positions 22 and 23. In another general aspect, the modifications of formula (III), (IV), or (V) may be at the N-terminal end. In certain embodiments, the N-terminal portion of formula (III), (IV), or (V) has an added octylglycine. In other embodiments, the N-terminal portion of formula (III), (IV), or (V) has an added isocap.

In yet another aspect, LHC peptides for use in the invention comprise an amino acid sequence of formula (VI): Xaa1 Xaa2 Xaa3 Xaa4 Xaa5 Xaa6 Xaa7 Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 P Xaa24 T N Xaa27 G S Xaa30 Xaa31 Xaa32 (SEQ ID NO:68) wherein
Xaa1 is A, C, D, F, K, T, or absent;
Xaa2 is A, C, D, S, or absent;
Xaa3 is A, D, N, or absent;
Xaa4 is A, L, T, or absent;
Xaa5 is A or S;
Xaa6 is A, S, T, or V;
Xaa7 is A, C, or K;
Xaa8 is A, L, M, or V;
Xaa9 is L or T;
Xaa10 is G, H, or Q;
Xaa11 is K, Q, or R;
Xaa12 is L, W, or Y;
Xaa13 is A, N, Q, S, or T;
Xaa14 is A, D, E, G, K, N, Q, or R;
Xaa15 is A, D, E, F, L, S, or Y;
Xaa16 is F or L;
Xaa17 is H, Q, S or V,
Xaa18 is K, or R;
Xaa19 is F, L, S, or absent;
Xaa20 is H, K, Q, or absent;
Xaa21 is Q, T, or absent;
Xaa22 is F, L, or Y;
Xaa24 is P or R;
Xaa27 is T or V;
Xaa30 is E, K or N;
Xaa31 is A or T; and
Xaa32 is F,Y, or absent

In a general aspect, the sequence of formula (VI) further comprises 1, 2, 3, 4, 5, 6, 7, 8, 9, 10, 11, 12, or more modifications of substitutions, insertions, deletions, elongations and/or derivatizations. In certain embodiments, the sequence of formula (III), (IV), (V) or (VI) comprises a deletion at position 24.

In yet another aspect, LHC peptides for use in the invention comprise an amino acid sequence comprising
a) a loop region comprising formula (H) Xaa1; wherein Xaa1 comprises an amino sequence of X Xaa2 Xaa3 Xaa4 Xaa5 Xaa6 Xaa7 Y (SEQ ID NO:69) wherein,
   Xaa2 is any amino acid or absent;
   Xaa3 is Ala, Gly, Ser, Asp or absent;
   Xaa4 is Asn, Ala, Asp, Gly or absent;
   Xaa5 is Ala, Leu, Thr, or Ser;
   Xaa6 is Ala, Ser, or Thr; and
   Xaa7 is Ala, Ser, Val, Hse, (S)-2-amino-3-hydroxy-methylbutanoic acid (Ahb), (2S,3R)-2-amino-3hydroxy-methylpentanoic acid(Ahp), d-Thr, Thr, or a derivative thereof;
   X and Y are amino acids capable of creating a bond and are independently selected residues having side chains which can be chemically bonded to each other to form an intramolecular linkage such as a disulfide bond; an amide bond; a cyclic lactam formed by an alkyl acid and an alkyl amine; an alkyl amine or imine bridge formed by condensing and reducing an alkyl aldehydes or alkyl halides and alkylamines; and an alkyl, alkenyl, alkynyl, ether or thioether bond formed by connection of side chains;
b) an α helix region type I comprising the sequence X1 V L Xaa10 Xaa11 L S Q Xaa15 L Xaa17 Xaa18 L Q T Xaa22 P Xaa24 T N T X1 (SEQ ID NO:70), wherein
   Xaa10 is Gly or Aib;
   Xaa11 is Lys, Arg, Orn, hArg, Cit, hLys, or Lys (for);
   Xaa15 is Glu or Phe;
   Xaa17 is His or Aib;
   Xaa18 is Lys, Arg, Orn, hArg, Cit, hLys,Lys(for), Lys(PEG 5000);
   Xaa22 is Try or Leu;
   Xaa24 is Arg or Pro; and
   X1 is absent or comprises 1-4 additional amino acids; and
c) a C-terminal tail comprising the sequence Xaa28 Xaa29 Xaa30 Xaa31 Xaa32 Xaa33 G Xaa35 Xaa36 Xaa37 Xaa38 (SEQ ID NO:71), wherein
   Xaa28 is Lys, Tyr, or absent;
   Xaa29 is Ser, Pro, or absent;
   Xaa30 is Ser, Pro, Arg, or absent;
   Xaa31 is Thr, or absent;
   Xaa32 is Asn or absent;
   Xaa33 is Val, Thr, or absent;
   Xaa35 is Ser, Glu
   Xaa36 is Asn, Lys, or Gly;
   Xaa37 is Thr, Phe, or Ala;
   Xaa38 is Tyr, Phe, Pro, or absent;
   with the proviso that when the loop region is from a calcitonin or calcitonin analog and the α helix region is from a calcitonin or calcitonin analog, the last position of the C-terminal tail is not Pro, Hyp, Hse or derivatives of Hse.

In yet another aspect, LHC peptides for use in the invention comprise an amino acid sequence comprising
a) a loop region comprising Xaa1;
a) a loop region comprising the formula (II) Xaa1; wherein Xaa1 comprises an amino sequence of XXaa2 Xaa3 Xaa4 Xaa5 Xaa6 Xaa7 Y (SEQ ID NO:72) wherein,
   Xaa2 is any amino acid or absent;
   Xaa3 is Ala, Gly, Ser, Asp or absent;
   Xaa4 is Asn, Ala, Asp, Gly or absent;
   Xaa5 is Ala, Leu, Thr, or Ser;
   Xaa6 is Ala, Ser, or Thr; and
   Xaa7 is Ala, Ser, Val, Hse, Ahb, Ahp, d-Thr, Thr, or a derivative thereof;
   X and Y are amino acids capable of creating a bond and are independently selected residues having side chains which can be chemically bonded to each other to form an intramolecular linkage such as a disulfide bond; an amide bond; a cyclic lactam formed by an alkyl acid and an alkyl amine; an alkyl amine or imine bridge formed by condensing and reducing an alkyl aldehydes or alkyl halides and alkylamines;
   and an alkyl, alkenyl, alkynyl, ether or thioether bond formed by connection of side chains;
b) an α helix region type II comprising the sequence X1 Xaa8 Xaa9 Xaa10 R Xaa12 Xaa13 Xaa14 Xaa15 Xaa16 Xaa17 Xaa18 Xaa19 Xaa20 Xaa21 Xaa22 P Xaa24 T N T X1 (SEQ ID NO:73) wherein
   Xaa8 is Ala or Val;
   Xaa9 is Thr, Met or Leu;
   Xaa10 is Gln, Gly, His;
   Xaa12 is Leu, or Thr;
   Xaa13 is Ala, Thr, Asn, Phe, Tyr, Ser, or Thr;
   Xaa14 is Asn, Arg, Ala, Asp, Glu, Gln, Thr, or Gly;
   Xaa15 is Phe, Leu, Ser, Glu, Ala, Asp, or Tyr;
   Xaa16 is Leu or Asp;
   Xaa17 is Val, His, Ser, Phe, or Aib;
   Xaa18 is His, Arg, Lys, Orn, hArg, Cit, hLys, Lys(for), or Lys(PEG5000);
   Xaa39 is Leu, Ser or Phe;
   Xaa20 is Gln or His;
   Xaa21 is Thr or Asn;
   Xaa22 is Tyr, Val, Phe, Leu or Met;
   Xaa24 is Arg or Pro; and
   X1 is absent or comprises 1-4 additional amino acids; and
c) a C-terminal tail comprising the sequence Xaa28 Xaa29 Xaa30 Xaa31 Xaa32 Xaa33 G Xaa35 Xaa36 Xaa37 Xaa38 (SEQ ID NO:74), wherein
   Xaa28 is Lys, Tyr, or absent;
   Xaa29 is Ser, Pro, or absent;
   Xaa30 is Ser, Pro, Arg, or absent;
   Xaa31 is Thr, or absent;
   Xaa32 is Asn, or absent;
   Xaa33 is Val, Thr, or absent;
   Xaa35 is Ser, or Glu
   Xaa36 is Asn, Lys, or Gly;
   Xaa37 is Thr, Phe, or Ala;
   Xaa38 is Tyr, Phe, Pro, or absent.

In still another aspect, LHC peptides for use in the invention include:
(SBQ ID NO:75) KCNTATCVLGKLSQELHRLQTYPRTNTGSNTY
(SEQ ID NO:76) KCNTATCVLGRLSQELHRLQTLPRTNTGSNTY
(SEQ ID NO:77) KCNTATCVLGRLSQELHRLQTYPPTNTGSNTY
(SEQ ID NO:78) KCNTATCVLGRLSQELHRLQTYPRTNVGSNTY
(SEQ ID NO:79) KCNTATCVLGRLSQELHRLQTLPPTNVGSNTY
(SEQ ID NO:80) KCNTATCVLGRLANFLHRLQTYPRTNTGSNTY
(SEQ ID NO:81) ACNTATCVLGRLSQELHRLQTYPRTNTGSNTY
(SEQ ID NO:82) KCNAATCVLGRLSQELHRLQTYPRTNTGSNTY
(SEQ ID NO:83) KCNTAACVLGRLSQELHRLQTYPRTNTGSNTY
(SEQ ID NO:94) CANLSTCVLGRLSQELHRLQTYPRTNTGSNTY
(SEQ ID NO:85) isocaproyl-STAVLGRLSQELHRLQTYPRTNTGSNTY
(SEQ ID NO:86) CSNASTCVLGRLSQELHRLQTYPRTNTGSNTY
(SEQ ID NO:87) CSNLATCVLGRLSQELHRLQTYPRTNTGSNTY
(SEQ ID NO:88) CSNLSACVLGRLSQELHRLQTYPRTNTGSNTY
(SEQ ID NO:89) KCNTATCVLGRLSQELHKLQTYPRTNTGSNTY
(SEQ ID NO:90) KCNTATCVLGRLSQELHRLQTYPRTNTGSGTP
(SEQ ID NO:91) CSALSTCVLGRLSQELHRLQTYPRTNTGSNTY
(SEQ ID NO:92) Ac-(Agy)SNLST(Agy)VLGRLSQELHRLQTYPRTNTGSNTY
(SEQ ID NO:92) Ac-K(Agy)NTAT(Agy)VLGRLSQELHRLQTYPRTNTGSNTY
(SEQ ID NO:93) Isocaproyl-STAVL(Aib)RLSQELRLQTYPRTNTGSGTP
(SEQ ID NO:94) Isocaproyl-STAVLG[K(For)]LSQELH[K(For)]LQTYPRTNTGSGTP
(SEQ ID NO:95) Isocaproyl-STAVL(Aib)[K(For)]LSQEL(Aib)[K(For)]LQTYPRTNTGSNTY
(SEQ ID NO:96) Isocaproyl-STAVL(Aib)[K(For)]LSQEL(Aib)[K(For)]LQTYPRTNTGSNTY
(SEQ 1D NO:97) KCNTATCLLQQLQKLLQKLKQYPRTNTGSNTY
(SEQ ID NO:98) KCNTASCVLGRLSQELHRLQTYPRTNTGSNTY
(SEQ ID NO:99) KCNTAVCVLGRLSQELHRLQTYPRTNTGSNTY
(SEQ ID NO:100) KCNTATCVLORLSQELHRYPRTNTGSNTY
(SBQ ID NO:101) KCNTATCVLGK(For)LSQELHK(For)LQTYPRTNTGSNTY
(SEQ ID NO:102) KCNTA(d-Thr)CVLGRLSQELHRLQTYPRTNTGSNTY
(SEQ 1D NO:103) KCNTA(dAh)CVLGRLSQELHRLQTYPRTNTGSNTY
(SEQ ID NO:104) Ac-ACNTATCVLGRLSQELHK(PEG5000)LQTYPRTNTGSNTY
(SEQ TD NO:105) KCNTATCVLGRLSQELHRLQTLQTYPRTNTGSNTY
(SEQ ID NO:106) KCNTATCVLGRLSQELHRLQTLLQTYPRTNTGSNTY
[SEQ ID NO:107) KCNTATCVLGKLSQELHKLQTYPRTNTGSNTY
(SEQ ID NO:108) KCNTATCVLGKLSQELHKLQTYPRTNTGSNTY
(SEQ ID NO:109) KCNTATCATQRLSQELHRLQTYPRTNTGSNTY
(SEQ ID NO:110) KCNTATCATQRLSQELHRLQTYPRTNVGSNTY
(SEQ ID NO:111) KCNTSTCATQRLANEL VRLQTYPRTNVGSNTY
(SEQ ID NO: 112) KCNTA(Hse)CVLGRLSQELHRLQTYPRTNTY
(SEQ ID NO: 113) KCNTA(Ahb)CVLGRLSQELHRLQTYPRTNTGNTY
(SEQ ID NO:114) KCNTA(Ahp)CVLGRLSQELHRLQTYPRTNTGSNTY
(SEQ ID NO:115) KCNTAT(OPO₃H₂)CVLGRLSQELHRLQTYPRTNTGSNTY
(SEQ ID NO:116) KCNTATCVLG(Om)LSQELH(Om)LQTYPRTNTGSNTY
(SEQ ID NO:117) KCNTATCVLG(Cit)LSQELH(Cit)LQTYPRTNTGSNTY
(SBQ ID NO:118) KCNTATCVLG(homoK)LSQEIH(homoK)LQTYPRTNTGSNTY
(SEQ ID NO:119) L-OctylglycineKCNTATCVLGRLSQELHRLQTYPRTNTGSNTY
(SEQ ID NO:120) N-3,6-dioxaoctanoyl-CNTATCVLGRLSQELHRLQTVPRTNTGSNTY
(SBQ ID NO:121) KCNTATCMLGRYTQDFHRLQTYPRTNTGSNTY
(SEQ ID NO: 122) DSNLSTKVLGRLSQELHRLQTYPRTNTGSNTY
(SBQ ID NO:123) KDNTATKVLGRLSQELHRLQTYPRTNTGSNTY
(SEQ ID NO:124) CNTATCVLGRLSQELHRLQTYPRTNTGSNTY
(SEQ ID NO:125) KCNTATCVLGRLSQELHRLQTYPRTNTGSN(9Anc)
(SEQ ID NO:126) KCNTATCVLGRLSQELHRLQTYPRTNTGSNTY
(SEQ ID NO:127) N-isocaproyl-KCNTATCVLGRLSQELHRLQTYPRTNTGSNTY
(SEQ ID NO:128) KCNTATCVLG(homoR)LSQELH(homoR)LQTYPRTNTGSNTY
(SEQ ID NO:129) FCNTATCVLGRLSQELHRLQTYPRTNTGSNTY
(SEQ ID NO:130) KCNTATCVLGRLSQELH(Cit)LQTYPRTNTGSNTY
(SEQ ID NO:131) KCNTATCVLGRLSQELH(Orn)LQTYPRTNTGSNTY
(SEQ ID NO:132) ICNTATCVLGRLSQELHRLQTYPRTNTGSNTY
(SEQ ID NO:133) 1-Octylglycine-CNTATCVLGRLSQELHRLQTYPRTNTGSNTY
(SEQ ID NO:134) Isocapxayl-CNTATCVLGRLSQELHRLQTYPRTGSNTY
(SEQ ID NO:135) KCNTATCVLG(Cit)LSQELHRLQTYPRTNTGSNTY
(SEQ ID NO:136) KCNTATCVLGRLSQELHRLQTYPRTNTGSNTY(4ABU)
(SEQ ID NO:137) Isocaproyl-KCNTATCVLGRLSQELHRLQTYPRTNTGSNTY(4ABU)
(SEQ ID NO:138) KCNTSTCATQRLANELVRLQTYPRTNVGSEAF
(SEQ ID NO:139) KCNTATCVLGRLSQELHRLQTYPTNVGSEAF
(SEQ ID NO:140) KCNTATCVLGRLSRSLHRLQTYPRTNTGSNTY
(SEQ ID NO:141) KCNTATCVTHRLSQELHRLQTYPRTNTGSNTY
(SEQ ID NO:142) KCNTATCVLGRLADFLHRLQTYPRTNTGSNTY
(SEQ ID NO:143) CNTATCVLGRLSQELHRLQTYPRTNTGSNT
(SEQ ID NO:144) KCNTATCVLGRLSQELHRLQNFVPRTNTGSNTY
(SEQ ID NO:145) KCNTATCVLGRLSQELHRLQTYPRTNTGSETF
(SEQ ID NO:146) ACDTATCVLGRLSQELHRLQTYPRTNTGSNTY
(SEQ ID NO:147) KCNTATCVLGRLSQELHRLQTYPRTNTGSKAF
(SEQ ID NO:148) KCDTATCVTHRLAGLLSRSQTYPRTNTGSNTY
(SEQ ID NO:149) KCNTATCVLGRLADALHRLQTYPRTNTGSNTY
(SEQ ID NO:150) KCNTATCVLGRLAAFLHRLQTYPRTNTGSNTY
(SEQ ID NO:151) SCNTATCVLGRLADFLHRLQTYPRTNTGSNTY
(SEQ ID NO:152) KCNTATCVLGRLSQELHRLQTMPRTNTGSNTY
(SEQ ID NO:153) KCNTATCVLGRLSQELHRLQTVPRTNTGSNTY
(SEQ ID NO:154) KCNTATCVLGRLNEYLHRLQTYPRTNTGSNTY
(SEQ ID NO:155) SCNTATCVLGRLSQELHRLQTYPRTNTGSNTY
(SEQ ID NO:156) KCNTATCVLGRLTEFLHRLQTYPRTNTGSNTY
(SEQ ID NO:157) KCNTATCVLGRLAEFLHRLQTYTRTNTGSNTY
(SEQ ID NO:158) KCNTATCVLGRLTDYLHRLQTYPRTNTGSNTY
(SEQ ID NO:159) KCNTATCVLGRLAQFLHRLQTYPRTNTGSNTY
(SEQ ID NO:160) KCNTATCVLGRLADFLHREFQTFPRTNTGSNTY
(SEQ ID NO:161) KCNTATCVLGRLADFLHRFHTFPRTNTGSNTY
(SEQ ID NO:162) KCNTATCVLGRLADFLHRFQTFPRTNTGSGTP
(SEQ ID NO:163) CNTATCVLGRLADFLHRLQTYPRTNTGSNTY
(SEQ ID NO:164) KCDTATCVLGRLSQELHRLQTYPRTNTGSNTY
(SEQ ID NO:165) KCNTATCVLGRLFDFLHRLQTYPRTNTGSNTY
(SEQ ID NO:166) KCNTATCVLGRLAAALHRLQTYPRTNTGSNTY
(SEQ ID NO:167) TCDTATCVLGRLSQELHRLQTYPRTNTGSNTY
(SEQ ID NO:168) CSNLSTCATQRLANELVRLQTYPRTNVGSNTY
(SEQ ID NO:169) KCNTATCATQRLANELVRLQTYPRTNVGSNTY
(SEQ ID NO:170) CSNLSTCVLGRLSQELHRLQTYPRTNTGSNTY
(SEQ ID NO:171) KCNTATCVLGRLSQELHRLQTYPRTNTGSNTY

In still another aspect, LHC peptides for use in the invention include biologically active fragments of SEQ ID NOS:75 to 171. Biologically active fragments may comprise deletions of 1, 2, 3, 4, 5, 6, 7, 8, 9, 10, 11, 12, 13, 14, 15 or more amino acids. In certain embodiments, the amino acid sequences of SEQ ID NOs:75 to 171 comprise at least 1, 2, 3, 4, 5, 6, 7, 8, 9, 10 or more modifications such as substitutions, insertions, deletions, and/or derivatizations. In other embodiments, the amino acid sequences of SEQ ID NOs:75 to 171 have no more than 1, 2, 3, 4, 5, 6, 7, 8, 9, or 10 modifications such as substitutions, insertions, deletions, and/or derivatizations. In still another aspect of the invention, compounds of the invention include those having at least 75, 80, 85, 90, 95, or 97 % amino acid sequence identity to any of SEQ ID NOS: 75 to 171. Percent identity is determined by analysis with the AlignX® module in Vector NTI® (Invitrogen; Carlsbad CA). It is intended that each percent identity described, or reference to biologically active fragments or modifications be applied to each SEQ ID NO: individually. For example, each embodiment described, fragments, modification, or % identity is applicable to SEQ ID NO: 75, 76, 77, 78, 44, etc., or to any group of SEQ ID NOs.

In general, amylin agonists or amylin agonist analogs are recognized as referring to compounds which, by directly or indirectly interacting or binding with one or more receptors, mimics an action of amylin. Accordingly, compounds of the invention may act as an agonist for at least one biological effect of calcitonin, amylin, CGRP, or any combination of the three herein disclosed or bind to at least one of the receptors of amylin, calcitonin, or CGRP. Conversely, amylin antagonists by directly or indirectly interacting or binding with one or more receptors, suppresses an action of amylin. Such interactions or binding events include those that affect ghrelin levels.

Amylin antagonists contemplated in the use of the invention include AC66 (sCT[8-32]) (SEQ ID NO:172) and derivatives such as AC187 (Ac 30Asn, 32Tyr-sCT[8-32]) (SEQ ID NO:173) a 25 amino acid peptide fragment of salmon calcitonin, developed as a selective amylin receptor antagonist over CGRP receptors. Other useful antagonists include antagonists described in U.S. Patent Nos. 5,625,032 and 5,580,953, which are incorporated herein by reference. Such antagonist compounds include those comprising formula (VB): X-R1-Thr-Gln-R2-Leu-Ala-Asn-R3-Leu-Val-Arg-Leu-Gln-Thr-Tyr-Pro-Arg-Thr-Asn-Val-Gly-R4-Asn-Thr-Tyr-NH2 (SEQ ID NO:174)
wherein
R1 is Ala or a bond;
R2 is Arg, Gln, Lys, Asn or Leu;
R3 is Gln, Glu, Asn, Asp or Phe;
R4 is Ala or Ser; and
X is hydrogen or an acetyl group.

Amylin antagonists may be acetylated or non-acetylated at the N-terminus and include acid as well as amide forms of the molecule. Examples of amylin antagonists include, but are not limited to, acetyl-Ala Thr Gln Arg Leu Ala Asn Glu Leu Val Arg Leu Gln Thr Tyr Pro Arg Thr Asn Val Gly Ser Asn Thr Tyr (SEQ ID NO:175), Ala Thr GInGIn Leu Ala Asn Gln Leu Val Arg Leu Gln Thr Tyr Pro Arg Thr Asn Val Gly Ser Asn Thr Tyr (SEQ m NO:176), Ala Thr Gln Leu Leu Ala Asn Gln Leu Val Arg Leu Gln Thr Tyr Pro Arg Thr Asn Val Gly Ser Asn Thr Tyr (SEQ ID NO:177), Ala Thr Gln Arg Leu Ala Asn Gln Leu Val Arg Leu Gln Thr Tyr Pro Arg Thr Asn Val Gly Ser Asn Thr Tyr (SEQ ID NO:178), Ala Thr Gln Leu Leu Ala Asn Glu Leu Val Arg Leu Gln Thr Tyr Pro Arg Thr Asn Val Gly Ser Asn Thr Tyr (SEQ ID NO: 179), Ala Thr Gln GIn Leu Ala Asn Glu Leu Val Arg Leu Gln Thr Tyr Pro Arg Thr Asn Val Gly Ser Asn Thr Tyr (SEQ ID NO:180).

Methods of testing compounds for amylin activity are known in the art. Exemplary screening methods and assays for testing amylin agonists or antagonists are described in the Examples, particularly Example 4 herein, and in U.S. Patent Nos. 5,264,372 and 5,686,411, which are incorporated herein by reference.

Activity as amylin agonists and/or analogs can be confirmed and quantified by performing various screening assays, including the nucleus accumbens receptor binding assay, followed by the soleus muscle assay, a gastric emptying assay, or by the ability to induce hypocalcemia or reduce postprandial hyperglycemia in mammals.

The receptor binding assay, a competition assay that measures the ability of compounds to bind specifically to membrane-bound amylin receptors, is described in U.S. Pat. Nos. 5,264,372 and 5,686,411, the disclosures of which are incorporated herein by reference. A preferred source of the membrane preparations used in the assay is the basal forebrain which comprises membranes from the nucleus accumbens and surrounding regions. Compounds being assayed compete for binding to these receptor preparations with 125I Bolton Hunter rat amylin. Competition curves, wherein the amount bound (B) is plotted as a function of the log of the concentration of ligand, are analyzed by computer using analyses by nonlinear regression to a 4-parameter logistic equation (INPLOT program; GraphPad Software, San Diego, Calif.) or the ALLFIT program of DsLean et al. (ALLFIT, Version 2.7 (NIH, Bethesda, Md. 20892)). Munson and Rodbard (1980) Anal. Biochem. 107:220-239.

Assays of biological activity of amylin agonists/analogs in the soleus muscle may be performed using previously described methods (Leighton et al. (1988) Nature 335:632-635; Cooper et al. (1988) Proc. Natl. Acad. Sci. USA 85:7763-7766), in which amylin agonist activity may be assessed by measuring the inhibition of insulin-stimulated glycogen synthesis. In brief, an exemplary method includes soleus muscle strips prepared from 12-h fasted male Wistar rats. The tendons of the muscles are ligated before attachment to stainless steel clips. Muscle strips are pre-incubated in Erlenmeyer flasks containing 3.5 ml Krebs-Ringer bicarbonate buffer, 7mM N-2-hydroxyethyl-peperazine-N'-2-ethane-sulphonic acid, pH 7.4, and 5.5 mM pyruvate. Flasks are sealed and gassed continuously with O₂ and CO₂ in the ratio 19:1 (v/v). After pre-incubation of muscles in this medium for 30 min at 37°C in an oscillating water bath, the muscles strips are transferred to similar vials containing identical medium (except pyruvate) with added [U-14C] glucose (0.5 µCi/ml) and insulin (100 µU/ml). The flasks are sealed and re-gassed for an initial 15 min in a 1-h incubation. At the end of the incubation period, muscles are blotted and rapidly frozen in liquid N2. The concentration of lactate in the incubation medium can be determined spectrophotometrically and [U-14C]glucose incorporation in glycogen measured. Amylin antagonist activity is assessed by measuring the resumption of insulin-stimulated glycogen synthesis in the presence of 100 nM rat amylin and an amylin antagonist.

Methods of measuring the rate of gastric emptying are disclosed in, for example, Young et al. In a phenol red method, conscious rats receive by gavage an acoloric gel containing methyl cellulose and a phenol red indicator. Twenty minutes after gavage, animals are anesthetized using halothane, the stomach exposed and clamped at the pyloric and lower esophageal sphincters, removed and opened into an alkaline solution. Stomach content may be derived from the intensity of the phenol red in the alkaline solution, measured by absorbance at a wavelength of 560 nm. In a tritiated glucose method, conscious rats are gavaged with tritiated glucose in water. The rats are gently restrained by the tail, the tip of which is anesthetized using lidocaine. Tritium in the plasma separated from tail blood is collected at various timepoints and detected in a beta counter. Test compounds are normally administered about one minute before gavage.

Amylin agonist and antagonist compounds may exhibit activity in the receptor binding assay on the order of less than about 1 to nM, preferably less than about 1 nM and more preferably less than about 50 pM. In the soleus muscle assay, amylin agonist compounds may show EC50 values on the order of less than about 1 to 10 micromolar. In the soleus muscle assay, amylin antagonists may show IC₅₀ values on the order of less than about 1 to 2 micromolar. In the gastric emptying assays, preferred agonist compounds show ED₅₀ values on the order of less than 100 µg/rat. Antagonist compounds would show no effect or the opposite effect in the gastric emptying assay.

In one exemplary method of making the compounds, compounds of the invention may be prepared using standard solid-phase peptide synthesis techniques and preferably an automated or semiautomated peptide synthesizer. Typically, using such techniques, an α -N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, with t-butyloxycarbonyl (tBoc) and fluorenylmethoxycarbonyl (Fmoc) being preferred herein. Other methods of synthesizing or expressing amylin and amylin agonists and purifying them are known to the skilled artisan.

Anti-obesity agents for use in the present invention also include exendin peptide hormones and exendin agonists. Native exendin peptide hormones are known in art, as are functional peptide analogs and derivatives. Certain native peptides, peptide analogs and derivatives are described herein, however it should be recognized that any known exendin peptides that exhibit hormonal activity known in the art may be used in conjunction with the present invention. Exemplary exendin peptides include exendin-3 (His Ser Asp Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser (SEQ ID NO:181)) and exendin-4 (His Gly Glu Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser (SEQ ID NO:182)).

Any exendin peptide analog or derivative known in the art may be used in conjunction with the present invention. In one embodiment, the exendin peptide analogs and derivatives have at least one hormonal activity of a native exendin peptide. In certain embodiments, the exendin peptide analogs are agonists of a receptor which a native exendin peptide is capable of specifically binding. Exendin compounds include exendin peptide analogs in which one or more naturally occurring amino acids are eliminated or replaced with another amino acid(s). As known in the art, such exendin analogs are may be amidated or may be in the acid form.

In one embodiment, an exendin analog can have one or more amino acid substitutions, deletions, inversion, or additions compared to a native or naturally occurring exendin. Thus, exendin analogs can have an amino acid sequence that has one or more amino acid substitutions, additions or deletions as compared with a naturally occurring exendin, for example, exendin-4. In one embodiment, an exendin analog has an amino acid sequence that has about 30 or less, 25 or less, 20 or less, 15 or less, 10 or less, 5 or less, 4 or less, 3 or less, 2 or less, or 1 or less substitutions, additions, or deletions as compared to a naturally occurring exendin, such as exendin-4

Exemplary exendin compounds include agonist analogs of exendin-4, including, but are not limited to, ¹⁴Leu, ²⁵Phe-exendin-4 (His Gly Glu Gly Thr Phe Thr Ser Asp Leu Ser Lys Gln Leu Glu Glu Glu Ala Val Arg Leu Phe Ile Glu Phe Leu Lys Asn Gly Gly Pro Ser Ser Gly Ala Pro Pro Pro Ser (SEQ ID NO:183), ⁵Ala, ¹⁴Leu, ²⁵Phe-exendin-4 (His Gly Glu Gly Ala Phe Thr Ser Asp Leu Ser Lys Gln Leu Glu Glu Glu Ala Val Arg Leu Phe Ile Glu Phe Leu Lys Asn Gly Gly Pro Ser Ser Gly Ala Pro Pro Pro Ser (SEQ ID NO:184)), and ¹⁴Leu, ²²Ala, ²⁵Phe-exendin-4 (His Gly Glu Gly Thr Phe Thr Ser Asp Leu Ser Lys Gln Leu Glu Glu Glu Ala Val Arg Leu Ala Ile Glu Phe Leu Lys Asn Gly Gly Pro Ser Ser Gly Ala Pro Pro Pro Ser (SEQ ID NO:185)). Other exemplary exendin analogs include, but are not limited to, exendin-4 (1-30) (His Gly Glu Gly Thr Phe Thr Ser Asp Leu Ser Lys Gln Met Glu Glu Glu Ala Val Arg Leu Phe Ile Glu Trp Leu Lys Asn Gly Gly (SEQ ID NO:186)), exendin-4 (1-28) amide (His Gly Glu Gly Thr Phe Thr Ser Asp Leu Ser Lys Gln Met Glu Glu Glu Ala Val Arg Leu Phe Ile Glu Trp Leu Lys Asn-NH₂ (SEQ ID NO:187)), ¹⁴Leu, ²⁵Phe exendin-4 (1-28) amide (His Gly Glu Gly Thr Phe Thr Ser Asp Leu Ser Lys Gln Leu Glu Glu Glu Ala Val Arg Leu Phe Ile Glu Phe Leu Lys Asn-NH₂ (SEQ ID NO: 188)), and ¹⁴Leu,²²Ala, ²⁵Phe exendin-4 (1-28) amide (His Gly Glu Gly Thr Phe Thr Ser Asp Leu Ser Lys Gln Leu Glu Glu Glu Ala Val Arg Leu Ala Ile Glu Phe Leu Lys Asn-NH₂ (SEQ ID NO:189)).

Additional exemplary exendin agonists are described in U.S. patent application Ser. No. 10/181,102 and PCT Application No. PCT/US98/16387, both which claim the benefit of U.S. patent application Ser. No. 60/055,404, filed Aug. 8, 1997, all of which are herein incorporated by reference. Exemplary exendin agonists include compounds of the formula (I), formula (II) and formula (III) of U.S. patent application Ser. No. 10/181,102 and PCT Application No. PCT/US98/16387.

Other exendin agonists are described in U.S. patent application Ser. No. 09/554,533 and PCT Application Serial No. PCT/US98/24210, both of which claim the benefit of U.S. Provisional Application No. 60/065,442 filed Nov. 14, 1997, all of which are herein incorporated by reference. Still other exendin agonists are described in U.S. patent application Ser. No. 09/554,531 and PCT Application Serial No. PCT/US98/24273, both of which claim the benefit of U.S. Provisional Application No. 60/066,029 filed Nov. 14, 1997, all of which are herein incorporated by reference. Still other exendin agonists are described in PCT Application Serial No. PCT/US97/14199, filed Aug. 8, 1997, which is a continuation-in-part of U.S. patent application Ser. No. 08/694,954 filed Aug. 8, 1996, both of which are hereby incorporated by reference. Still other exendin agonists are described in U.S. Patent No. 6,956,026, which claims priority to U.S. Provisional Application No. 60/034,905 filed Jan. 7, 1997, both of which are hereby incorporated by reference. Yet other exendin analogs and derivatives are described in US 2004/0209803 A1, filed December 19, 2003, which is hereby incorporated by reference.

Anti-obesity agents in the present invention also include ciliary neurotrophic factor (CNTF), CNTF-related polypeptides, modified CNTF polypeptides, CNTF agonists, and CNTF analogs, including, but not limited to AXOKINE® (Regeneron). CNTF, CNTF-related polypeptides, and CNTF and/or CNTF-related polypeptide containing compositions appropriate for use in the methods of the invention are known in the art. CNTF polypeptides, CNTF-related polypeptides, modified CNTF polypeptides, CNTF agonists, analogs, derivatives, preparations, formulations, pharmaceutical compositions, doses, and administration routes have previously been described, for example, in U.S. Pat. Nos. 6,680,291 and 6,767,894, and in PCT Application Publication Nos. WO 94/09134, WO 98/22128, and WO 99/43813, which are hereby incorporated by reference in their entirety.

Anti-obesity agents in the present invention also include serotonin (5HT) transport inhibitors, including, but not limited to, paroxetine, fluoxetine, fenfluramine, fluvoxamine, sertraline, and imipramine. Anti-obesity agents in the present invention also include selective serotonin reuptake inhibitors, including, but not limited to dexfenfluramine, fluoxetine, sibutramine (e.g., MERIDIA®) and those described in U.S. Pat. No. 6,365,633 and PCT Patent Application Publication Nos. WO 01/27060 and WO 01/162341, which are hereby incorporated by reference in their entirety. Such 5HT transport inhibitors and serotonin reuptake inhibitors, analogs, derivatives, preparations, formulations, pharmaceutical compositions, doses, and administration routes have previously been described.

Anti-obesity agents for use in the present invention also include selective serotonin agonists and selective 5-HT2C receptor agonists, including, but not limited to, U.S. Pat. No. 3,914,250; and PCT Application Publication Nos. WO 02/36596, WO 02/48124, WO 02/10169, WO 01/66548, WO 02/44152; WO 02/51844, WO 02/40456, and WO 02/40457, which are hereby incorporated by reference in their entirety. Such selective serotonin agonists and 5-HT2C receptor agonists, compositions containing such agonists, and administration routes appropriate for use in the methods of the invention are known in the art. See, for example, Halford et al. (2005) Curr. Drug Targets 6:201-213 and Weintraub et al. (1984) Arch. Intern. Med. 144:1143-1148.

Anti-obesity agents for use in the present invention also include antagonists/inverse agonists of the central cannabinoid receptors (the CB-1 receptors), including, but not limited to, rimonabant (Sanofi Synthelabo), and SR-147778 (Sanofi Synthelabo). CB-1 antagonists/inverse agonsits, derivatives, preparations, formulations, pharmaceutical compositions, doses, and administration routes have previously been described, for example, in U.S. Pat. Nos. 6,344,474, 6,028,084, 5,747,524, 5,596,106, 5,532,237, 4,973,587, 5,013,837, 5,081,122, 5,112,820, 5,292,736, 5,624,941; European Patent Application Nos. EP-656 354 and EP-658546; and PCT Application Publication Nos. WO 96/33159, WO 98/33765, WO98/43636, WO98/43635, WO 01/09120, WO98/31227, WO98/41519, WO98/37061, WO00/10967, WO00/10968, WO97/29079, WO99/02499, WO 01/58869, and WO 02/076949, which are hereby incorporated by reference in their entirety.

Anti-obesity agents for use in the present invention also include melanocortins and melanocortin agonists. Melanacortins are peptides from the pro-opiomelanocortin gene, including α-melanocyte-stimulating hormone (α-MSH) and adrenocorticotrophic hormone (ACTH), and five melanocortin receptors are known, MC1-5R. MC4R appears to play a role in energy balance and obesity. See, for example, Anderson et al. (2001) Expert Opin. Ther. Patents 11:1583-1592, Speake et al. (2002) Expert Opin. Ther. Patents 12:1631-1638, Bednarek et al. (2004) Expert Opin. Ther. Patents 14:327-336. Melanocortin agonists, including, but not limited to, MC4R agonists, and composition containing such agonist appropriate for use in the methods of the invention are known in the art. MCR agonists, MC4R agonists, derivatives, preparations, formulation, pharmaceutical compositions, doses, and administration routes have previously been described, for example, in the following PCT patent applications, which are hereby incorporated by reference in their entirety: WO 03/007949, WO 02/06S38S, WO 02/068387, WO 02/067869, WO 03/040117, WO 03/066587, WO 03/068738, WO 03/094918, and WO 03/031410.

Anti-obesity agents for use in the present invention also include metabotropic glutamate subtype 5 receptor (mGluR5) antagonists, including, but are not limited to, compounds such as 2-methyl-6-(phenylethynyl)-pyridine (MPEP) and (3-[(2-methyl-1,3-thiazol-4-yl)ethynyl]pyridine) (MTEP) and those compounds described in Anderson et al. (2003) J. Eur. J Pharmacol. 473:35-40; Cosford et al. (2003) Bioorg. Med. Chem. Lett. 13(3):351-4; and Anderson et al. (2002) J. Pharmacol. Exp. Ther. 303:1044-1051.

Anti-obesity agents for use in the present invention also include topiramate (TOPIMAX® (Ortho McNeil Pharmaceuticals), indicated as an anti-convulsant and an anti-convulsant, but also shown to increase weight loss.

Anti-obesity agents for use in the present invention also include neuropeptide Y1 (NPY1) antagonists and NAY5 antagonists. NPY1 and NPY5 antagonists are known in the art. See, for example Duhault et al. (2000) Can. J Physiol. Pharm. 78:173-185, and U.S. Pat. Nos. 6,124,331, 6,214,853, and 6,340,683. NPY1 and NPY5 antagonists, derivatives, preparations, formulation, pharmaceutical compositions, doses, and administration routes have previously been described. NPY1 antagonists useful in the present invention, include; U.S. Pat. No. 6,001,836; and PCT Application Publication Nos. WO 96/14307, WO 01/23387, WO 99/51600, WO 01/85690, WO 01/85098, WO 01/85173, and WO 01/89528, which are hereby incorporated by reference in their entirety. NPY5 antagonists useful in the present invention, include, but are not limited to, the compounds described in: U.S. Pat. Nos. 6,140,354, 6,191,160, 6,258,837, 6,313,298, 6,337,332, 6,329,395, 6,340,683, 6,326,375, and 5,335,345; European Patent Nos. EP-01010691, and EP-01044970; and PCT Patent Publication Nos. WO 97/19682, WO 97/20820, WO 97/20821, WO 97/20822, WO 97/20823, WO 98/27063, WO 00/64880, WO 00/68197, WO 00/69849, WO 01/09120, WO 01/85714, WO 01/85730, WO 01/07409, WO 01/02379, WO 01/02379, WO 01/23388, WO,01/23389, WO 01/44201, WO 01/62737, WO 01/62738, WO 01/09120, WO 02/22592, WO 0248152, WO 02/49648, and WO 01/14376.

Anti-obesity agents for use in the present invention also include melanin-concentrating hormone (MCH) antagonists including melanin-concentrating hormone 1 receptor (MCH1R) antagonists, such as T-226296 (Takeda) and melanin-concentrating hormone 2 receptor (MCH2R) antagonists. MCH receptor antagonists, derivatives, preparations, formulation, pharmaceutical compositions, doses, and administration routes have previously been described, for example, in U.S. Patent Application Publication Nos. 2005/0009815, 2005/0026915, 2004/0152742, 2004/0209865; PCT Patent Application Publication Nos. WO 01/82925, WO 01/87834, WO 02/06245, WO 02/04433, and WO 02/51809; and Japanese Patent Application No. JP 13226269, which are hereby incorporated by reference in their entirety.

Anti-obesity agents for use in the present invention also include opioid antagonists, including, but not limited to those described in PCT Application No. WO 00/21509. Specific opioid antagonists useful in the present invention include, but are not limited to, nalmefene (REVEX®), 3-methoxynaltrexone naloxone, naltrexone, naloxonazine, beta-funaltrexamine, deltal ([D-Ala2,Leu5,Cys6]-enkephalin (DALCE), naltrindole isothiocyanate, and nor-binaltorphamine.

Anti-obesity agents for use in the present invention also include orexin antagonists, including, but not limited to, those described in PCT Patent Application Nos. WO 01/96302, WO 01/68609, WO 02/51232, and WO 02/51838. Specific orexin antagonists useful in the present invention include, but are not limited to, SB-334867-A.

Anti-obesity agents for use in the present invention also include neuropeptide Y2 (NPY2) agonists, including, but not limited to, compounds such as PYY3-36 (e.g., Batterham et al. (2003) Nature 418:650-654), NPY3-36, and other Y2 agonists such as N acetyl [Leu(28,31)] NPY 24-36 (White-Smith et al. (1999) Neuropeptides 33:526-533, TASP-V (Malis et al. (1999) Br. J. Pharmacol. 126:989-996), cyclo-(28/32)-Ac-[Lys28-Glu32]-(25-36)-pNPY (Cabrele et al. (2000) J. Pept. Sci. 6:97-122). Anti-obesity agents in the present invention also include neuropeptide Y4 (NPY4) agonists including, but not limited to, compounds such as pancreatic peptide (PP) (e.g., Batterham et al. (2003) J. Clin. Endocrinol. Metab. 88:3989-3992) and other Y4 agonists such as 1229U91 (Raposinho et al. (2000) Neuroendocrinology 71:2-7). NPY2 agonists and NPY4 agonsits, derivatives, preparations, formulations, pharmaceutical compositions, doses, and administration routes have previously been described, for example, in U.S. Pat Publication No. 2002/0141985 and PCT Application Publication No. WO 2005/077094.

Anti-obesity agents for use in the present invention also include histamine 3 (H3) antagonist/inverse agonists including but not limited to, those described in PCT Application No. WO 02/15905, O-[3-(1H-imidazol-4-yl)propanol]carbamates (Kiec-Kononowicz et al. (2000) Pharmazie 55:349-355), piperidine-containing histamine H3-receptor antagonists (Lazewska et al. (2001) Pharmazie 56:927-932), benzophenone derivatives and related compounds (Sasse et al. (2001) Arch. Pharm.(Weinheim) 334:45-52), substituted N-phsnylcarbamates (Reidemeister et al. (2000) Pharmazie 55:83-86), and proxifan derivatives (Sasse et al. (2000) J. Med. Chem. 43:3335-3343). Specific H3 antagonists/inverse agonists useful in the present invention include, but are not limited to, thioperamide, 3-(1H-imidazol-4-yl)propyl N-(4-pentenyl)carbamate, clobenpropit, iodophenpropit, imoproxifan, and GT2394 (Gliatech).

Anti-obesity agents for use in the present invention also include cholecystokinin (CCK) and CCK agonists. Cholecystokinin-A (CCK-A) agonists useful in the present invention include, but are not limited to, those described U.S. Pat. No. 5,739,106. Specific CCK-A agonists include, but are not limited to, AR-R 15849, GI 181771, JMV-180, A-71378, A-71623 and SR146131.

Anti-obesity agents for use in the present invention also include ghrelin antagonists such as those described in PCT Application Publication Nos. WO 01/87335 and WO 02/08250. Ghrelin antagonists are also known as GHS (growth hormone secretagogue receptor) antagonists. The compositions and methods of the present invention therefore comprehend the use GHS antagonists in place of ghrelin antagonists.

### Dosage/Formulation

Anti-obesity agents and weight-inducing agents (herein referred to in this section as the "compounds") may be administered alone or in combination with pharmaceutically acceptable carriers or excipients, in either single or multiple doses. These pharmaceutical compounds may be formulated with pharmaceutically acceptable carriers or diluents as well as any other known adjuvants and excipients in accordance with conventional techniques such as those disclosed in Remington's Pharmaceutical Sciences by E. W. Martin. See also Wang et al. (1988) J. of Parenteral Sci. and Tech., Technical Report No. 10, Supp. 42:2S.

In general, the compounds may be formulated into a stable, safe pharmaceutical composition for administration to a patient. Pharmaceutical formulations contemplated for use in the methods of the invention may comprise approximately 0.01 to 1.0% (w/v), in certain cases 0.05 to 1.0%, of the compound, approximately 0.02 to 0.5% (w/v) of an acetate, phosphate, citrate or glutamate buffer allowing a pH of the final composition of from about 3.0 to about 7.0; approximately 1.0 to 10% (w/v) of a carbohydrate or polyhydric alcohol tonicifier and, optionally, approximately 0.005 to 1.0% (w/v) of a preservative selected from the group consisting of m-cresol, benzyl alcohol, methyl, ethyl, propyl and butyl parabens and phenol. Such a preservative is generally included if the formulated peptide is to be included in a multiple use product.

In a particular embodiment of the present invention, a pharmaceutical formulation of the present invention may contain a range of concentrations of the compound, e.g., between about 0.01% to about 98% w/w, or between about 1 to about 98% w/w, or preferably between 80% and 90% w/w, or preferably between about 0.01% to about 50% w/w, or more preferably between about 10% to about 25% w/w in this embodiment A sufficient amount of water for injection may be used to obtain the desired concentration of solution.

Additional tonicifying agents such as sodium chloride, as well as other known excipients, may also be present, if desired. In some cases, such excipients are useful in maintenance of the overall tonicity of the compound. An excipient may be included in the presently described formulations at various concentrations. For example, an excipient may be included in the concentration range from about 0.02% to about 20% w/w, preferably between about 0.02% and 0.5% w/w, about 0.02% to about 10% w/w, or about 1 % to about 20% w/w. In addition, similar to the present formulations themselves, an excipient may be included in solid (including powdered), liquid, semi-solid or gel form.

The pharmaceutical formulations may be composed in various forms, e.g., solid, liquid, semisolid or liquid. The term "solid", as used herein, is meant to encompass all normal uses of this term including, for example, powders and lyophilized formulations. The presently described formulations may be lyophilized.

The terms buffer, buffer solution and buffered solution, when used with reference to hydrogen-ion concentration or pH, refer to the ability of a system, particularly an aqueous solution, to resist a change ofpH on adding acid or alkali, or on dilution with a solvent. Characteristic of buffered solutions, which undergo small changes of pH on addition of acid or base, is the presence either of a weak acid and a salt of the weak acid, or a weak base and a salt of the weak base. An example of the former system is acetic acid and sodium acetate. The change of pH is slight as long as the amount of hydronium or hydroxyl ion added does not exceed the capacity of the buffer system to neutralize it.

As described herein, a variety of liquid vehicles are suitable for use in the formulations of peptidic anti-obesity agents, for example, water or an aqueous/organic solvent mixture or suspension.

The stability of a peptide formulation for use in the present invention is enhanced by maintaining the pH of the formulation in the range of about 3.0 to about 7.0 when in liquid form. In certain embodiments, the pH of the formulation is maintained in the range of about 3.5 to 5.0, or about 3.5 to 6.5, in some embodiments from about 3.7 to 4.3, or about 3.8 to 4.2. In some embodiments, pH may be about 4.0. While not seeking to be bound by this theory, it is presently understood that where the pH of the pharmaceutical formulation exceeds 5.5, chemical degradation of the peptide may be accelerated such that the shelf life is less than about two years.

In certain embodiments, the buffer with the anti-obesity agents is an acetate buffer (preferably at a final formulation concentration of from about 1-5 to about 60 mM), phosphate buffer (preferably at a final formulation concentration of from about 1-5 to about to about 30 mM) or glutamate buffer (preferably at a final formulation concentration of from about 1-5 to about to about 60 mM). In some embodiments, the buffer is acetate (preferably at a final formulation concentration of from about 5 to about 30 mM).

A stabilizer may be included in the formulations of anti-obesity agents but, and importantly, is not necessarily needed. If included, however, a stabilizer useful in the practice of the present invention is a carbohydrate or a polyhydric alcohol. A suitable stabilizer useful in the practice of the present invention is approximately 1.0 to 10% (w/v) of a carbohydrate or polyhydric alcohol. The polyhydric alcohols and carbohydrates share the same feature in their backbones, i.e., -CHOH-CHOH-, which is responsible for stabilizing the proteins. The polyhydric alcohols include such compounds as sorbitol, mannitol, glycerol, and polyethylene glycols (PEGs). These compounds are straight-chain molecules. The carbohydrates, such as mannose, ribose, sucrose, fructose, trehalose, maltose, inositol, and lactose, on the other hand, are cyclic molecules that may contain a keto or aldehyde group. These two classes of compounds have been demonstrated to be effective in stabilizing protein against denaturation caused by elevated temperature and by freeze-thaw or freeze-drying processes. Suitable carbohydrates include: galactose, arabinose, lactose or any other carbohydrate which does not have an adverse affect on a diabetic patient, i.e., the carbohydrate is not metabolized to form unacceptably large concentrations of glucose in the blood. Such carbohydrates are well known in the art as suitable for diabetics. Sucrose and fructose are suitable for use with the compound in non-diabetic applications (e.g. treating obesity).

In certain embodiments, if a stabilizer is included, the compound is stabilized with a polyhydric alcohol such as sorbitol, mannitol, inositol, glycerol, xylitol, and polypropylene/ethylene glycol copolymer, as well as various polyethylene glycols (PEG) of molecular weight 200, 400, 1450, 3350, 4000, 6000, and 8000). Mannitol is the preferred polyhydric alcohol in some embodiments. Another useful feature of the lyophilized formulations of the present invention is the maintenance of the tonicity of the lyophilized formulations described herein with the same formulation component that serves to maintain their stability. In some embodiments, mannitol is the preferred polyhydric alcohol used for this purpose.

The United States Pharmacopeia (USP) states that anti-microbial agents in bacteriastatic or fungistatic concentrations must be added to preparations contained in multiple dose containers. They must be present in adequate concentration at the time of use to prevent the multiplication of microorganisms inadvertently introduced into the preparation while withdrawing a portion of the contents with a hypodermic needle and syringe, or using other invasive means for delivery, such as pen injectors. Antimicrobial agents should be evaluated to ensure compatibility with all other components of the formula, and their activity should be evaluated in the total formula to ensure that a particular agent that is effective in one formulation is not ineffective in another. It is not uncommon to find that a particular antimicrobial agent will be effective in one formulation but not effective in another formulation.

A preservative is, in the common pharmaceutical sense, a substance that prevents or inhibits microbial growth and may be added to pharmaceutical formulations for this purpose to avoid consequent spoilage of the formulation by microorganisms. While the amount of the preservative is not great, it may nevertheless affect the overall stability of the peptide.

While the preservative for use in the pharmaceutical compositions can range from 0.005 to 1.0% (w/v), in some embodiments range for each preservative, alone or in combination with others, is: benzyl alcohol (0.1-1.0%), or m-cresol (0.1-0.6%), or phenol (0.1-0.8%) or combination of methyl (0.05-0.25%) and ethyl or propyl or butyl (0.005%-0.03%) parabens. The parabens are lower alkyl esters of para-hydroxybenzoic acid. A detailed description of each preservative is set forth in Remington's Pharmaceutical Sciences by Martin.

Pramlintide, human ^{25,28,29}Pro-amylin, does not have a tendency to adsorb onto the glass in a glass container when in a liquid form, therefore, a surfactant is not required to further stabilize the pharmaceutical formulation. However, with regard to compounds which do have such a tendency when in liquid form, a surfactant should be used in their formulation. These formulations may then be lyophilized. Surfactants frequently cause denaturation of protein, both of hydrophobic disruption and by salt bridge separation. Relatively low concentrations of surfactant may exert a potent denaturing activity, because of the strong interactions between surfactant moieties and the reactive sites on proteins. However, judicious use of this interaction can stabilize proteins against interfacial or surface denaturation. Surfactants which could further stabilize the peptide may optionally be present in the range of about 0.001 to 0.3% (w/v) of the total formulation and include polysorbate 80 (i.e., polyoxyethylene(20) sorbitan monooleate), CHAPS® (i.e., 3-[(3-cholamidopropyl) dimethylammonio] 1-propanesulfonate), Brij® (e.g., Brij 35, which is (polyoxyethylene (23) lauryl ether), poloxamer, or another non-ionic surfactant.

It may also be desirable to add sodium chloride or other salt to adjust the tonicity of the pharmaceutical formulation, depending on the tonicifier selected. However, this is optional and depends on the particular formulation selected. Parenteral formulations preferably may be isotonic or substantially isotonic.

A preferred vehicle for parenteral products is water. Water of suitable quality for parenteral administration can be prepared either by distillation or by reverse osmosis. Water for injection is the preferred aqueous vehicle for use in the pharmaceutical formulations.

It is possible that other ingredients may be present in the pharmaceutical formulations. Such additional ingredients may include, e.g., wetting agents, emulsifiers, oils, antioxidants, bulking agents, tonicity modifiers, chelating agents, metal ions, oleaginous vehicles, proteins (e.g., human serum albumin, gelatin or proteins) and a zwitterion (e.g., an amino acid such as betaine, taurine, arginine, glycine, lysine and histidine). Additionally, polymer solutions, or mixtures with polymers provide the opportunity for controlled release of the peptide. Such additional ingredients, of course, should not adversely affect the overall stability of the pharmaceutical formulation of the present invention.

Containers are also an integral part of the formulation of an injection and may be considered a component, for there is no container that is totally inert, or does not in some way affect the liquid it contains, particularly if the liquid is aqueous. Therefore, the selection of a container for a particular injection must be based on a consideration of the composition of the container, as well as of the solution, and the treatment to which it will be subjected. Adsorption of the peptide to the glass surface of the vial can also be minimized, if necessary, by use of borosilicate glass, for example, Wheaton Type I borosilicate glass #33 (Wheaton Type I-33) or its equivalent (Wheaton Glass Co.). Other vendors of similar borosilicate glass vials and cartridges acceptable for manufacture include Kimbel Glass Co., West Co., Bünder Glas GMBH and Forma Vitrum. The biological and chemical properties of the compound may be stabilized by formulation and lyophilization in a Wheaton Type I-33 borosilicate serum vial to a final concentration of 0.1 mg/ml and 10 mg/ml of the compound in the presence of 5% mannitol, and 0.02% Tween 80.

For formulations to be delivered by injection, in order to permit introduction of a needle from a hypodermic syringe into a multiple-dose vial and provide for resealing as soon as the needle is withdrawn, the open end of each vial is preferably sealed with a rubber stopper closure held in place by an aluminum band.

Stoppers for glass vials, such as, West 44116/50, 416/50 (Teflon faced) and 4406/40, Abbott 5139 or any equivalent stopper can be used as the closure for pharmaceutical for injection. For formulations comprising peptidic anti-obesity agents, these stoppers are compatible with the peptide as well as the other components of the formulation. The inventors have also discovered that these stoppers pass the stopper integrity test when tested using patient use patterns, e.g., the stopper can withstand at least about 100 injections. Alternatively, the peptide can be lyophilized in to vials, syringes or cartridges for subsequent reconstitution. Liquid formulations of the present invention can be filled into one or two chambered cartridges, or one or two chamber syringes.

Each of the components of the pharmaceutical formulation described above is known in the art and is described in Pharmaceutical Dosage Forms: Parenteral Medications, Vol. 1, 2nd ed., Avis et al. Ed., Mercel Dekker, New York, N.Y. 1992, which is incorporated by reference in its entirety herein.

The manufacturing process for the above liquid formulations generally involves compounding, sterile filtration and filling steps. The compounding procedure involves dissolution of ingredients in a specific order (preservative followed by stabilizer/tonicity agents, buffers and peptide) or dissolving at the same time.

Alternative formulations, e.g., non-parenteral, may not require sterilization. However, if sterilization is desired or necessary, any suitable sterilization process can be used in developing the peptide pharmaceutical formulation of the present invention. Typical sterilization processes include filtration, steam (moist heat), dry heat, gases (e.g., ethylene oxide, formaldehyde, chlorine dioxide, propylene oxide, beta-propiolacctone, ozone, chloropicrin, peracetic acid methyl bromide and the like), exposure to a radiation source, and aseptic handling. Filtration is the preferred method of sterilization for liquid formulations of the present invention. The sterile filtration involves filtration through 0.45 µm and 0.22 µm (1 or 2) which may be connected in series. After filtration, the solution is filled into appropriate vials or containers.

In one embodiment, the anti-obesity agents are administered peripherally to the subjects. In some embodiments, the liquid pharmaceutical formulations of the present invention are intended for parenteral administration. Suitable routes of administration include intramuscular, intravenous, subcutaneous, intradermal, intraarticular, intrathecal and the like. In some embodiments, the subcutaneous route of administration is preferred. In certain embodiments, mucosal delivery is also preferred. These routes include, but are not limited to, oral, nasal, sublingual, pulmonary and buccal routes which may include administration of the peptide in liquid, semi-solid or solid form. For formulations comprising peptidic anti-obesity agents, administratron via these routes requires substantially more peptide to obtain the desired biological effects due to decreased bioavailability compared to parenteral delivery. In addition, parenteral controlled release delivery can be achieved by forming polymeric microcapsules, matrices, solutions, implants and devices and administering them parenterally or by surgical means. Examples of controlled release formulations are described in U.S. Patent Nos. 6,368,630, 6,379,704, and 5,766,627, which are incorporated herein by reference. These dosage forms may have a lower bioavailability due to entrapment of some of the peptide in the polymer matrix or device. See e.g., U.S. Pat. Nos. 6,379,704, 6,379,703, and 6,296,842.

The compounds may be provided in dosage unit form containing an amount of the compound with or without insulin or glucose (or a source of glucose) that will be effective in one or multiple doses to control the effects of ghrelin. Therapeutically effective amounts of the compounds for the treatment of ghtelin-associated diseases or disorders are those sufficient to treat, prevent, or ameliorate the physiological effects of undesirable levels of ghrelin.

As will be recognized by those in the field, an effective amount of the anti-obesity agents will vary with many factors including the age and weight of the patient, the patient's physical condition, the condition to be treated, and other factors. An effective amount of the anti-obesity agents will also vary with the particular combination administered. As described herein, administration of the agents in combination may allow for a reduced amount of any of the administered agents to be an effective amount.

However, typical doses may contain from a lower limit of about 1 µg, 5 µg, 10 µg, 50 µg to 100 µg to an upper limit of about 100 µg, 500 µg, 1 mg, 5 mg, 10 mg, 50 mg or 100 mg of the pharmaceutical compound per day. Also contemplated are other dose ranges such as 0.1 µg to 1 mg of the compound per dose. The doses per day may be delivered in discrete unit doses, provided continuously in a 24 hour period or any portion of that the 24 hours. The number of doses per day may be from 1 to about 4 per day, although it could be more. Continuous delivery can be in the form of continuous infusions. Exemplary doses and infusion rates include from 0.005 nmol/kg to about 20 nmol/kg per discrete dose or from about 0.01/pmol/kg/min to about 10 pmol/kg/min in a continuous infusion. These doses and infusions can be delivered by intravenous administration (i.v.) or subcutaneous administration (s.c.). Exemplary total dose/delivery of the pharmaceutical composition given i.v. may be about 2 µg to about 8 mg per day, whereas total dose/delivery of the pharmaceutical composition given s.c may be about 6 µg to about 6 mg per day.

Leptin and leptin derivatives may be administerad, for example, at a daily dosage of from about 0.01 mg/kg to about 20 mg/kg, in some cases, from about 0.01 mg/kg to about 0.3 mg/kg. Administration may be by injection of a single dose or in divided doses.

Sibutramine may be administered, for example, at a daily dosage of from about 0.01 mg/kg to about 10 mg/kg, in some cases from about 0.01 mg/kg to about 1 mg/kg in a single dose or in divided doses 2 to 3 times per day, or in sustained release form. In some instances, sibutramine may be administered the single daily dose of 5 mg, 10 mg, 15 mg, 20 mg or 30 mg orally.

Rimonabant may be administered, for example, at a daily dosage of from about 0.01 mg/kg to about 8 mg/kg, in some instances from about 0.3 mg/kg to about 3 mg/kg of body weight in a single dose or in divided doses 2 to 3 times per day, or in sustained release form.

The following Examples are provided to illustrate, but not limit, the invention.

### EXAMPLES

### EXAMPLE 1

Diet-induced obesity (DIO) in the in the Sprague-Dawley rat is a valuable model for the study of obesity and regulation of energy homeostasis. These rats were developed from a line of (Crl:CD®(SD)BR) rats that are prone to become obese on a diet relatively high in fat and energy. See, for example, Levin (1994) Am. J. Physiol. 267:R527-R535, Levin et al. (1997) Am. J. Physiol. 273:R725-R730. DIO male rats were obtained from Charles River Laboratories, Inc. (Wihnington, MA). The rats were housed individually in shoebox cages at 22 °C in a 12/12-hour light dark cycle. Rats were maintained *ad-libitum* on a moderately high fat diet (32% kcal from fat; Research Diets D1226B) for 6-7 weeks prior to drug treatment. At the end of the fattening period (prior to drug administration), the animals typically achieve a mean body weight of about 500 g.

The DIO animals were divided into four treatment groups. Each group was implanted with subcutaneous osmotic mini-pumps (DURECT Corp., Cupertino, CA) designed to deliver vehicle, leptin (500 µg/kg/day), amylin (100 µg/kg/day) or leptin (500 µg/kg/day) + amylin (100 µg/kg/day) for a 14 day period. As described herein, amylin acts on structures in the hindbrain involved in food intake and/or body weight modulation and leptin acts on structures in the hypothalamus involved in food intake and/or body weight modulation. Food intake and body weight were recorded daily. Body composition was measured prior to and after drug treatment using NMR (Echo Medical Systems, Houston, TX). Indirect calorimetry was used to measure changes in energy expenditure on days 4, 5 and 6 of drug treatment (Oxymax; Columbus Instruments, Columbus, OH). All data are represented as mean ± SEM. Analysis of variance (ANOVA) and post-hoc tests were used to test for group difference. A P-value <0.05 was considered significant. Statistical analysis and graphing were performed using PRISM® 4 for Windows (GraphPad Software, Inc., San Diego, CA), In some early studies, SYSTAT® for Windows (Systat Software, Inc., Point Richmond CA) was used for analysis.

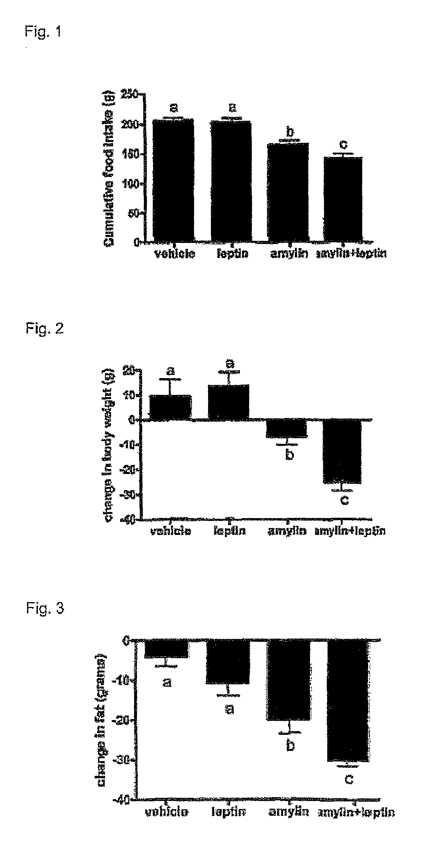

Figures 1 and 2 show the effects of amylin and leptin on cumulative food intake and total changes in body weight after 14 days of drug administration. Of particular interest is that (1) leptin, the forebrain acting agent, was ineffective on its own in this obesity model (no effect on either food intake or body weight), and (2) rats treated with the combination of amylin and leptin consumed significantly less food and lost significantly more weight relative to rats treated with either vehicle or amylin or leptin alone (p<0.05; different letters indicate that groups differed significantly from one another).

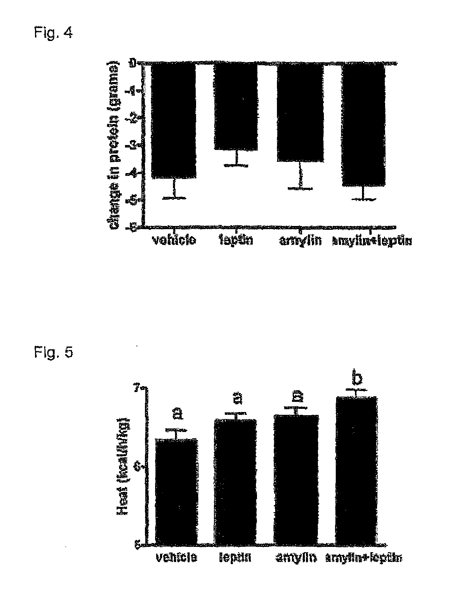

Similar effects were observed on body composition. Figure 3 depicts changes in body fat produced by the treatments and Figure 4 depicts changes in body protein produced by the treatments. Fat loss in animals treated with the combination of amylin+leptin was significantly greater than that in animals treated with either individual agent or vehicle (p<0.05). These changes in body fat were not accompanied by significant decreases in lean tissue (p>0.05; Figure 4).

Figure 5 depicts changes in energy expenditure during the dark cycle of the treatment groups. While drug (or diet)-induced reduction in food intake and body weight is often accompanied by a slowing of metabolic rate (Heat, kcal/h/kg), rats treated with the combination of leptin and amylin had a significantly higher metabolic rate during the dark cycle relative to the other groups (p<0.05). Thus, simultaneously targeting hindbrain and forebrain feeding centers with the amylin + leptin combination resulted in a significant and sustained reduction in food intake, body weight and body fat while increasing metabolic rate. In addition, the reductions in body weight and body fat were not accompanied by a reduction in lean tissue mass.

### EXAMPLE 2

Another series of experiments were performed to further explore the synergistic effects of the combination of amylin and leptin on changes in body weight and body composition. In-bred DIO (Levin) rats were obtained from Charles Rivers Labs for these studies. These rats were developed by Barry Levin from a line of Crl:CD®(SD)BR rats that are prone to become obese on a diet relatively high in fat and energy. They were housed individually in shoebox cages at 22 C in a 12/12-hour light dark cycle. Rats were maintained *ad-libitum* on a moderately high fat diet (32% kcal from fat; Research Diets D1226B) for approximately 6 weeks prior to drug treatment and throughout the experiment with the exception of pair-fed controls (PF). PF rats were restricted to the intake of either the amylin-treated group. Prior to drug administration rats had typically attained a mean body weight of 500 g.

The animals were divided into treatment groups counter-balanced for body weight and were implanted with subcutaneous osmotic mini-pumps (Durect Corp., Cupertino, CA). Each rat was implanted with two mini-pumps containing drug or the appropriate vehicle. Rat amylin (AC0128; Lot#28) was dissolved in 50% DMSO in sterile water and murine leptin (Peprotech, catalog#450-31) was dissolved in sterile water. The pumps were designed to deliver vehicle, amylin at 100 µg/kg/day or murine leptin at 500 µg/kg/day for a 14 day period.

Each group was implanted with subcutaneous osmotic mini-pumps (DURECT Corp., Cupeatino, CA) designed to deliver vehicle, leptin (500 µg/kg/day), amylin (100 µg/kg/day) or leptin (500 µg/kg/day) + amylin (100 µg/kg/day) for a 14 day period. Body weight and food intake were recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment -% body fat at baseline = change in % body fat).

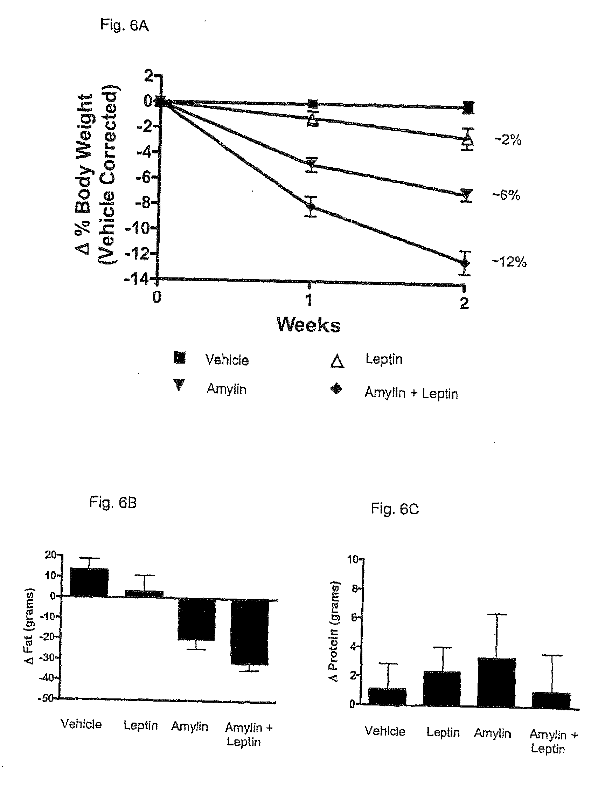

The graph in Figure 6A depicts vehicle-corrected changes in percent body weight of the treatment groups over the two weeks of treatment. In this experiment, leptin administration resulted in an overall 2% decrease in body weight and amylin administration resulted in an overall 6% decease in body weight. Notably, the percent decrease in body weight in response to administration of a combination of amylin and leptin was about 12%, an effect greater than the combined effect from the individual agents administered alone. Accordingly, amylin and leptin acted synergistically to reduce body weight. Figures 6B and 6C depict changes in body fat and changes in body protein, respectively, produced after the two weeks of treatment. Again, the reduction in body fat as a result of the combination of agents is more than the combined reduction in body fat as a result of the individual agents. These changes in body fat were not accompanied decreases in body protein but rather by a gain in percent protein. These results support a metabolic effect of the combination of agents as well as a weight reducing effect

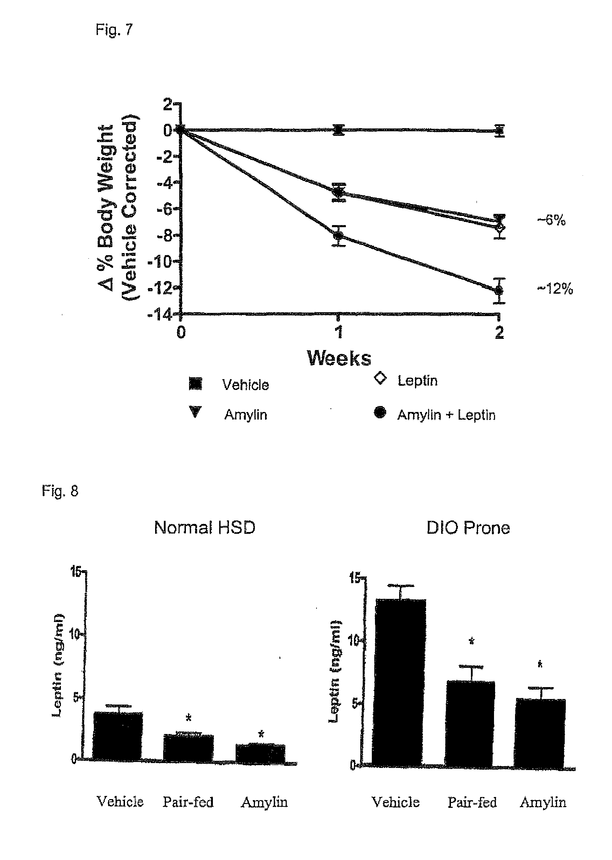

Amylin is known to have an anorectic effect on a recipient. In order to examine the effect af amylin + leptin in the context of an anorectic effect of amylin, a pair-feeding experiment was performed. DIO rats and drug treatment groups were established as described above. DIO rats in the vehicle, amylin, and amylin + leptin treatment groups had *ad libitum* access to food, while intake in the pair-fed leptin treatment was restricted to the amount consumed by the amylin-treated group. Body weight was recorded daily for the two weeks of treatment. As shown in Figure 7, the amylin treatment group and the pair-fed, leptin treatment group both had approximately a 6% decrease in body weight relative to vehicle control. This result is consistent with the previous result of leptin having little or no effect on body weight in the DIO animals. The combination of amylin + leptin results in a decrease of about 12 % in body weight relative to vehicle control. Accordingly, the pair-fed experiment demonstrates that the combination of amylin + leptin reduces body weight over and above that conferred by caloric restriction.

### EXAMPLE 3

As discussed herein, serum leptin levels in the majority of humans with obesity are high, and a state of leptin resistance is thought to exist in these individuals. Plasma leptin levels and leptin resistance were examined in normal Harlan Sprague-Dawley (HSD) and in DIO prone rats.

DIO prone and normal HSD rats were divided into three treatment groups. Two groups were implanted with subcutaneous osmotic mini-pumps (DURECT Corp., Cupertino, CA) designed to deliver vehicle or amylin (100 µg/kg/day) and the third group was pair-fed to the amount consumed by the amylin-treated group for a 14 day period. Serum leptin levels were determined by immunoassays using a commercial kit (Linco Research, Inc., St. Charles, MO). As shown in Figure 8, the serum leptin level in the DIO prone animals is approximately three-times higher than that in the normal HSD animals. Thus, DIO prone rats are hyper-Ieptntemic. Both amylin treatment and caloric restriction to the amount of food eaten by the amylin-treated animals significantly reduced plasma leptin levels in both the DIO prone and the normal animals.

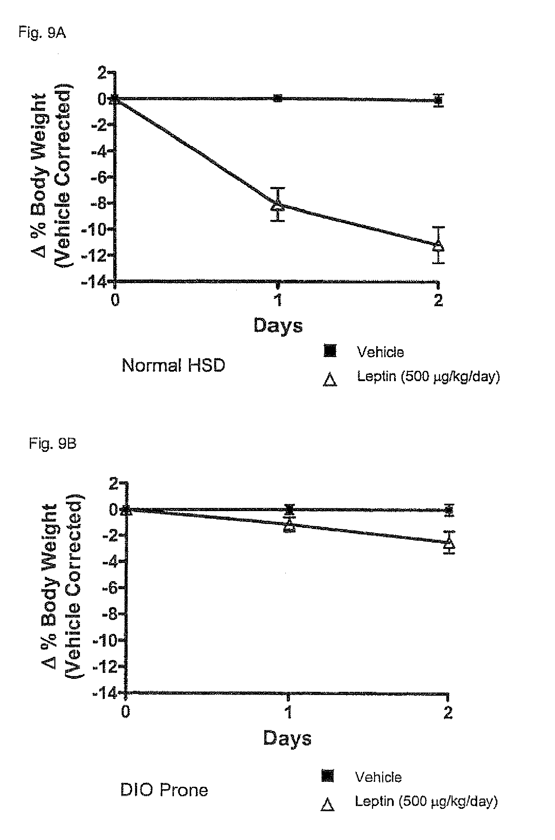

Normal, lean HSD rats were divided into two treatment groups. Each group was implanted with subcutaneous osmotic mini-pumps (DURECT Corp., Cupertino, CA) designed to deliver either vehicle or leptin (500 µg/kg/day) for a 14 day period and body weight was recorded weekly. As shown in Figure 9, the ineffective dose of leptin (500 mg/kg/day) in DIO prone animals, elicited a significant and sustained reduction in body weight in normal HSD rats. The DIO prone animals described herein appear resistant to the weight reducing effect of leptin.

### EXAMPLE 4

To demonstrate effects of the combination of amylin and a serotonergic/noradernergic reuptake inhibitor on changes in body weight and body composition, DIO male rats were fattened and divided into four treatment groups, as described in Example 2. Rat amylin was dissolved in 50% DMSO in sterile water and sibutramine was dissolved in sterile water. Each group was implanted with subcutaneous osmotic mini-pumps designed to deliver vehicle, sibutramine (3 mg/kg/day) or amylin (100 µg/kg/day) for a 14 day period. Body weight and food intake were recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment -% body fat at baseline = change in % body fat).

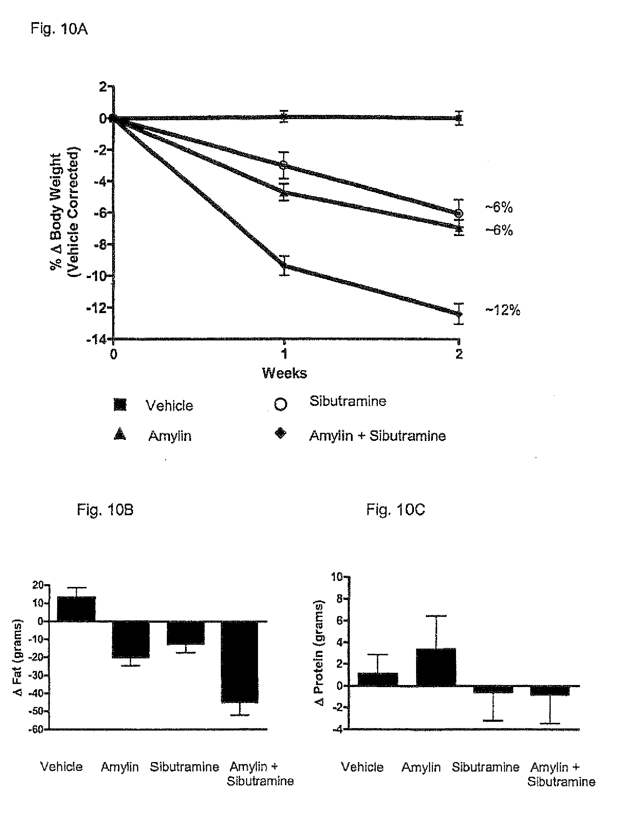

The graph in Figure 10A depicts vehicle-corrected changes in percent body weight of the treatment groups over the two weeks of treatment. Sibutramine administration alone and amylin administration alone resulted in about a 6% decease in body weight The percent decrease in body weight in response to administration of a combination of amylin and sibutramine was about 12%. Figures 10B and 10C depict changes in body fat and changes in body protein, respectively, produced after the two weeks of treatment. Fat mass loss was evident with the treatment of either amylin alone or sibutramine alone, and a synergistic effect was obtained when both amylin and sibutramine were administered in combination (Fig. 10B). Administration of amylin alone resulted in an increase in lean (protein) mass. Lean (protein) mass was relatively unchanged when sibutramine was administered alone or in combination with amylin (Fig. 10C). These results support a metabolic effect of the combination of agents as well as a weight reducing effect.

The combination of amylin and catecholaminergic agonist, phentermine, were also tested for effects on changes in body weight and body composition. Phentermine is classically referred to as a carecholaminergic agonist as it actually hits NA/5-HT receptors. DIO male rats were fattened and divided into four treatment groups, as described above. Each group was implanted with subcutaneous osmotic mini-pumps and/or inserted with an oral gavage, designed to deliver vehicle, phentermine (10 mg/kg/day), amylin (100 µg/kg/day) or phentermine (10 mg/kg/day) + amylin (100 µg/kg/day) for a 14 day period. The mini-pump contained either vehicle (50% DMSO in water) or amylin while the oral gavage administered either sterile water or phentermine. Body weight was recorded daily and body composition was measured prior to and after drug treatment using NMR.

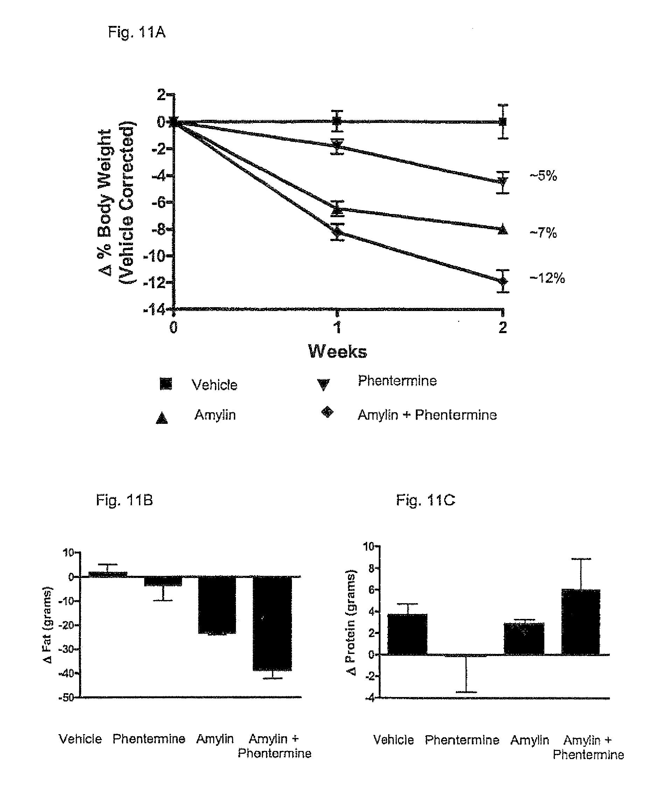

The graph in Figure 11A depicts vehicle-corrected changes in percent body weight of the treatment groups over the two weeks of treatment Phentermine administration alone resulted in about a 5 % decrease in body weight and amylin administration alone resulted in about a 7 % decease in body weight. The percent decrease in body weight in response to administration of a combination of amylin and phentermine was about 12%. Figures 11B and 11C depict changes in body fat and changes in body protein, respectively, produced after the two weeks of treatment. A modest amount of fat mass loss was evident with the treatment of phentermine alone and a greater amount of fat mass loss was evident with the treatment of amylin alone. When amylin and phentermine were administered in combination, a synergistic effect was obtained (Fig. 11B). Lean (protein) mass was unchanged or tended to be lost when phentermine was administered alone. Administration of amylin alone preserved lean (protein) mass and the combination of amylin and phentermine tended to have the greatest increase in lean (protein) mass, even while the animals underwent about 12% loss in body weight (Fig. 11C). These results support a metabolic effect of the combination of agents as well as a weight reducing effect.

### EXAMPLE 5

To demonstrate effects of the combination of amylin and a CB-1 antagonist on changes in body weight and body composition, in-bred DIO (Levin) rats were obtained from Charles Rivers Labs. These rats were developed by Barry Levin from a line of Crl.CD®(SD)BR rats that are prone to become obese on a diet relatively high in fat and energy. They were housed individually in shoebox cages at 22 C in a 12/12-hour light dark cycle. Rats were maintained *ad-libitum* on a moderately high fat diet (32% kcal from fat; Research Diets D1226B) for approximately 6 weeks prior to drug treatment and throughout the experiment. Prior to drug administration rats had typically attained a mean body weight of 500 g. Rats were habituated to oral gavage for 1 week prior to treatment Rimonabant was administered at a range of doses (0.1, 0.3, 1.0, 3.0. 10 mg/kg/day) by oral gavage. Amylin (dissolved in 50% DMSO sterile water) or vehicle was administered by mini-pump (100 µg/kg/day). Rimonabant was always delivered just prior to lights out. Food intake and body weight were measured at 1 and 2 weeks post-treatment. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment% body fat at baseline = change in % body fat).

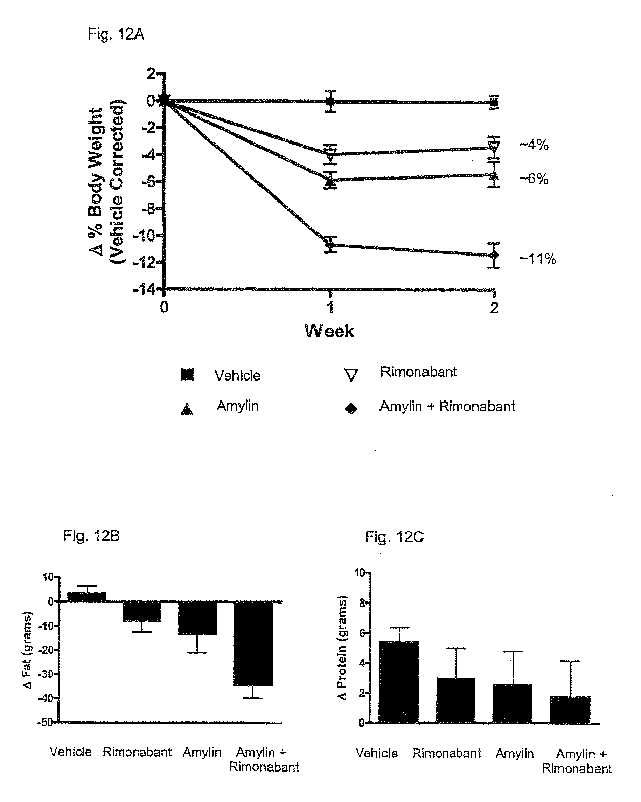

The graph in Figure 12A depicts vehicle-corrected changes in percent body weight of the treatment groups over the two weeks of treatment. Rimoaabant administration alone resulted in about a 4 % decrease in body weight and amylin administration alone resulted in about a 6% decease in body weight. The percent decrease in body weight in response to administration of a combination of amylin, and rimonabant was about 11%. Figures 12B and 12C depict changes in body fat and changes in body protein, respectively, produced after the two weeks of treatment. Fat mass loss was evident with the treatment of either amylin alone or rimonabant alone, and a synergistic effect was obtained when both amylin and rimonabant were administered in combination (Fig. 12B). Administration of amylin, alone, rimonabant alone, and amylin + rimonabant in combination resulted in a relatively equivalent increase in lean (protein) mass (Fig. 12C). These results support a metabolic effect of the combination of agents as well as a weight reducing effect.

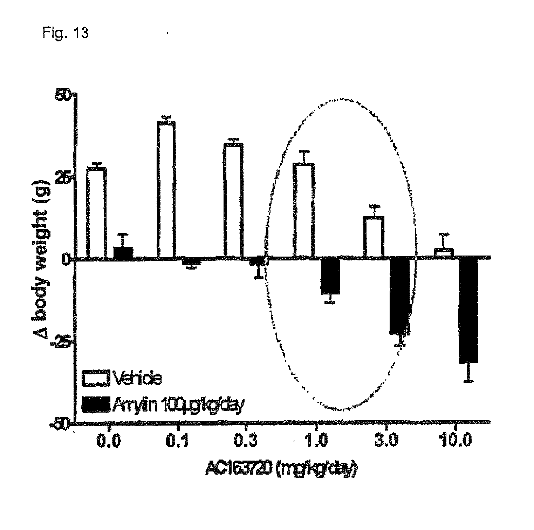

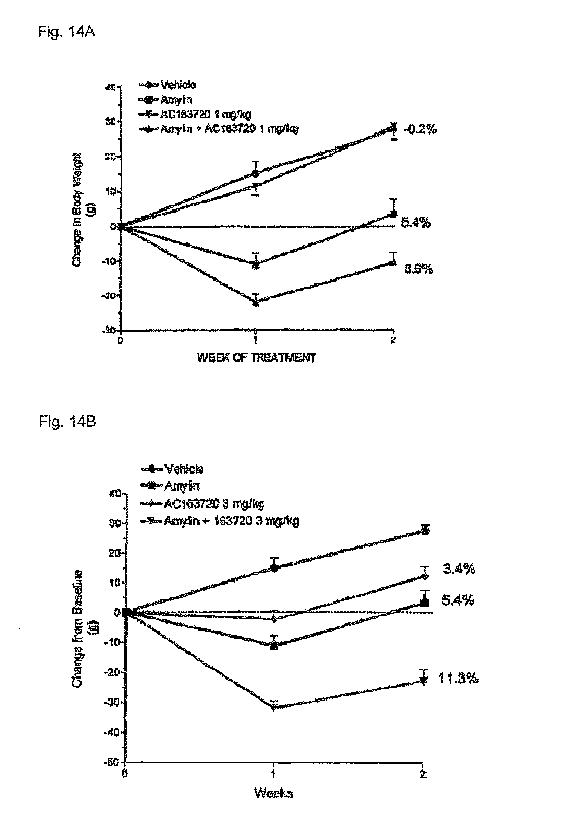

In another assay, the CB-1 antagonist rimonanbant (AC163720) was administered in combination with amylin. DIO prone rats were maintained *ad-libitum* on a moderately high fat diet (32% kcal from fat; Research Diets D1226B) for 6 weeks prior to drug treatment. At the end of the fattening period they typically have a mean body weight of 500 g. Rats were then divided into treatment groups and implanted with one subcutaneous mini-pump (Durect Corp) and inserted with an oral gavage. The mini-pump contained either vehicle (50% DMSO in water) or amylin (100 µg/kg/day) while the oral gavage administered either sterile water or a range of doses of rimonabant (AC163720) (0.1, 0.3, 1.0, 3.0, 10.0 mg/kg/day). Change in body weight after 2 weeks is depicted in Figure 13 and two of these combinations (circled) are highlighted in Figures 14A and 14B in more detail.

### EXAMPLE 6

To demonstrate effects of the combination of amylin and an exendin analog, ¹⁴Leu-exendin-4. His Gly Glu Gly Thr Phe Thr Ser Asp Leu Ser Lys Gln Leu Glu Glu Glu Ala Val Arg Leu Phe Ile Glu Trp Leu Lys Asn Gly Gly Pro Ser Ser Gly Ala Pro Pro Pro Ser (SEQ ID NO:190), on changes in body weight and body composition, DIO male rats were fattened and divided into four treatment groups, as described above. Prior to drug administration rats had typically attained a mean body weight of 500 g Exendin-4 analog was administered by minipump at a range of doses (0.3,1, 3, 10, 30 µg/kg/day). Amylin (dissolved in 50% DMSO in water) or vehicle was administered by mini-pump (100 µg/kg/day). Body weight and food intake was recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment - % body fat at baseline = change in % body fat).

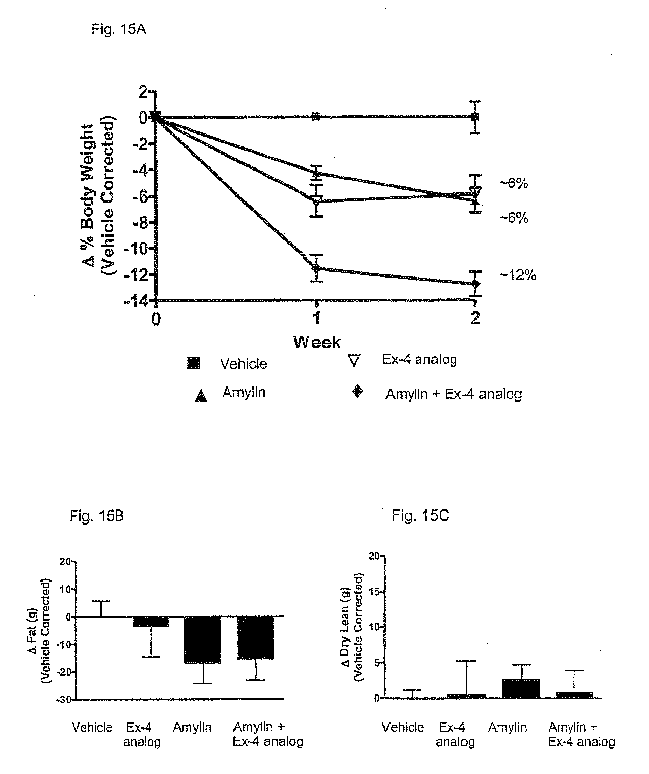

The graph in Figure 15A depicts vehicle-corrected changes in percent body weight of the treatment groups over the two weeks of treatment. Exendin-4 analog administration alone and amylin administration alone each resulted in about a 6% decrease in body weight. The percent decrease in body weight in response to administration of a combination of amylin and Exendin-4 analog was about 12%. Figures 15B and 15C depict changes in body fat and changes in body protein, respectively, produced after the two weeks of treatment. Fat mass loss was evident with the treatment of Exendin-4 analog alone. Administration of amylin alone and amylin + Exendin-4 analog in combination resulted in a relatively equivalent decrease in fat mass (Fig. 15B). Administration of amylin alone, Exendin-4 analog alone, and amylin + AC3174 in combination resulted in a relatively equivalent increase in lean (protein) mass (Fig. 15C). These results support a metabolic effect of the combination of agents as well as a weight reducing effect.

### EXAMPLE 7

To demonstrate effects of the combination of amylin and PYY agonists on changes in body weight and body composition, DIO male rats were fattened and divided into four treatment groups, as described above. Each group was implanted with subcutaneous osmotic mini-pumps, designed to deliver vehicle, PYY(3-36) (1000 µg/kg/day), amylin (100 µg/kg/day) or PYY(3-36) (1000 µg/kg/day) + amylin (100 µg/kg/day) for a 14 day period. PYY(3-36) was administered by mini-pump at a range of doses (100, 200, 400, 800, 1000 µg/kg/day). Amylin 100 µg/kg/day (dissolved in 50% DMSO sterile water), PYY(3-36) (dissolved in 50% DMSO sterile water) or vehicle was administered by mini-pump. Food intake and body weight was recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment -% body fat at baseline = change in % body fat).

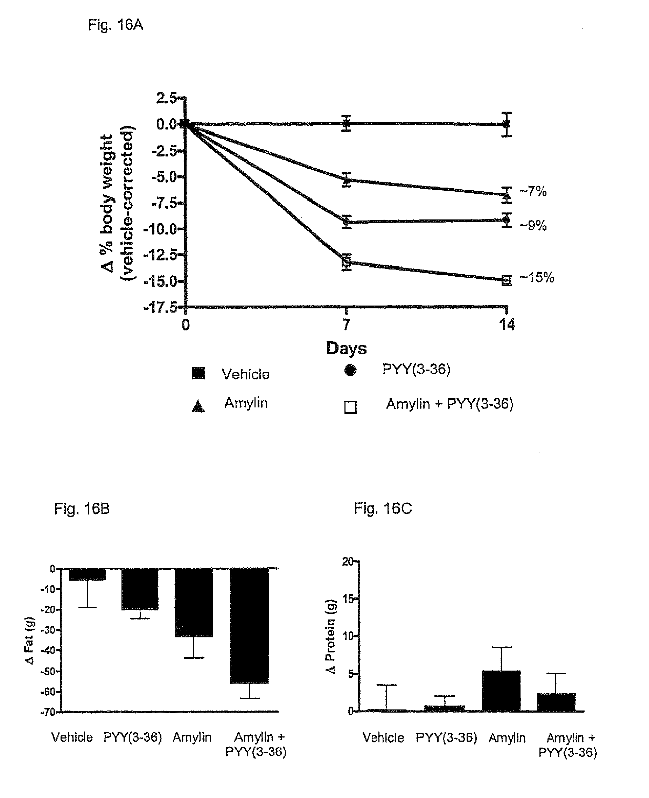

The graph in Figure 16A depicts vehicle-corrected changes in percent body weight of the treatment groups over the two weeks of treatment. PYY(3-36) administration alone resulted in about a 9% decrease in body weight and amylin administration alone resulted in about a 7% decrease in body weight. The percent decrease in body weight in response to administration of a combination of amylin and PYY(3-36) was about 15%. Figures 16B and 16C depict changes in body fat and changes in body protein, respectively, produced after the two weeks of treatment. Increasing amount of fat mass loss was evident with the treatment of PYY(3-36) alone, amylin, alone, and amylin + PYY(3-36) in combination (Fig. 16B). Administration of amylin alone and amylin + PYY(3-36) in combination resulted in an increase in lean (protein) mass (Fig. 16C). These results support a metabolic effect of the combination of agents as well as a weight reducing effect.

While the foregoing description discloses the present invention, with examples provided for the purpose of illustration, it will be understood that the practice of the present invention encompasses all of the usual variations, adaptations, or modifications as being within the scope of the claimed invention. Therefore, descriptions and examples should not be construed as limiting the scope of the invention, which is delineated by the appended claims.

### PREFERRED EMBODIMENTS

1. A method of treating obesity in a subject comprising peripherally administering therapeutically effective amounts of at least two different anti-obesity agents, wherein at least one anti-obesity agent acts upon structures in the forebrain involved in food intake or body weight modulation and at least one anti-obesity agent that acts upon structures in the hindbrain involved in food intake or body weight modulation;
   wherein when one anti-obesity agent is a PYY(3-36) a PYY(3-36) analog, or a PYY(3-36) agonist, then another anti-obesity agent is not an amylin, an amylin agonist, an amylin analog, a CCK, a CCK analog, or a CCK agonist; and
   wherein when one anti-obesity agent is an exendin, an exendin derivative or an exendin agonist, then another anti-obesity agent is not an amylin, an amylin agonist, an amylin analog.
2. The method according to embodiment 1 wherein at least one of the anti-obesity agents is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate, a CCK, a CCK analog, a CCK agonist, an amylin, an amylin analog, and an amylin agonist.
3. The method according to embodiment 2 wherein the at least one anti-obesity agent is phentermine, rimonabant, sibutramine or pramlintide.
4. A method of treating obesity in a subject comprising peripherally administering therapeutically effective amounts of at least two anti-obesity agents,
   wherein the first anti-obesity agent is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate,
   wherein the second anti-obesity agent is selected from the group consisting of a CCK, a CCK analog, a CCK agonist, an amylin, an amylin analog, and an amylin agonist,
   wherein when the first anti-obesity agent is not a PYY(3-36) a PYY(3-36) analog, or a PYY(3-36) agonist, and
   wherein when the first anti-obesity agent is an exendin, an exendin derivative or an exendin agonist, then the second anti-obesity agent is not an amylin, an amylin agonist, an amylin analog.
5. The method according to any of embodiments 1-4 wherein the subject reduces body weight by least 10%.
6. The method according to any of embodiments 1-5 wherein the subject reduces body fat mass.
7. A method of reducing nutrient availability in a subject in need thereof comprising peripherally administering to the subject at least two different anti-obesity agents, wherein at least one anti-obesity agent acts upon structures in the forebrain involved in food intake or body weight modulation and at least one anti-obesity agent that acts upon structures in the hindbrain involved in food intake or body weight modulation;
   wherein when one anti-obesity agent is a PYY(3-36) a PYY(3-36) analog, or a PYY(3-36) agonist, then another anti-obesity agent is not an amylin, an amylin agonist, an amylin analog, a CCK, a CCK analog, or a CCK agonist;
   wherein when one anti-obesity agent is an exendin, an exendin derivative or an exendin agonist, then another anti-obesity agent is not an amylin, an amylin agonist, an amylin analog, and
   wherein the anti-obesity agents are administered in amounts effect to reduce nutrient availability in the subject.
8. The method according to embodiment 7 wherein at least one of the anti-obesity agents is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate, a CCK, a CCK analog, a CCK agonist, an amylin, an amylin analog, and an amylin, agonist.
9. The method according to embodiment 8 wherein the at least one anti-obesity agent is phentermine, rimonabant, sibutramine or pramlintide.
10. The method according to embodiment 8 wherein one anti-obesity agent is an amylin, an amylin, analog or an amylin agonist and another anti-obesity agent is a leptin, a leptin derivative or a leptin agonist.
11. A method of reducing nutrient availability in a subject comprising peripherally administering therapeutically effective amounts of at least two anti-obesity agents,
   wherein the first anti-obesity agent is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate,
   wherein the second anti-obesity agent is selected from the group consisting of a CCK, a CCK analog, a CCK agonist, an amylin, an amylin analog, and an amylin agonist,
   wherein when the first anti-obesity agent is not a PYY(3-36) a PYY(3-36) analog, or a PYY(3-36) agonist, and
   wherein when the first anti-obesity agent is an exendin, an exendin derivative or an exendin agonist, then the second anti-obesity agent is not an amylin, an amylin agonist, an amylin analog, and
   wherein the anti-obesity agents are administered in amounts effect to reduce nutrient availability in the subject.
12. The method according to any of embodiments 7-11 wherein reduction in nutrient availability is indicated in the subject by body weight loss, by loss of fat mass, or by loss of ectopic fat.
13. The method according to any of embodiments 7-12 wherein the subject has at least one condition selected from the group consisting of obesity, an obesity-related disorder, an obesity related disease, an obesity-related condition, diabetes, insulin-resistance syndrome, lypodystrpohy, nonalcoholic steatohepatitis, a cardiovascular disease, polycystic ovary syndrome, and metabolic syndrome.
14. A method of reducing body weight in a subject comprising peripherally administering therapeutically effective amounts of at least two different anti-obesity agents, wherein at least one anti-obesity agent acts upon structures in the forebrain involved in food intake or body weight modulation and at least one anti-obesity agent that acts upon structures in the hindbrain involved in food intake or body weight modulation;
   wherein when one anti-obesity agent is a PYY(3-36) a PYY(3-36) analog, or a PYY(3-36) agonist, then another anti-obesity agent is not an amylin, an amylin agonist, an amylin analog, a CCK, a CCK analog, or a CCK agonist,
   wherein when one anti-obesity agent is an exendin, an exendin derivative or an exendin agonist, then another anti-obesity agent is not an amylin, an amylin agonist, an amylin, analog, and
   wherein the anti-obesity agents are administered in amounts effective to reduce the body weight of the subject by at least 10%.
15. The method according to embodiment 14 wherein at least one of the anti-obesity agents is selected from the group consisting of a NPY1 receptor antagonist, an NPY5 receptor antagonist, an NPY2 receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonistlinverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate, a CCK, a CCK analog, a CCK agonist, an amylin, an amylin analog, and an amylin agonist.
16. The method according to embodiment 15 wherein the at least one anti-obesity agent is phentermine, rimonabant, sibutramine or pramlintide.
17. The method according to embodiment 15 wherein one anti-obesity agent is an amylin, an amylin analog or an amylin agonist and another anti-obesity agent is a leptin, a leptin derivative or a leptin agonist.
18. A method of reducing body weight in a subject comprising peripherally administering therapeutically effective amounts of at least two anti-obesity agents,
   wherein the first anti-obesity agent is selected from the group consisting of a NPY1 receptor antagonist, an NPY5 receptor antagonist, an NPY2, receptor agonist, an NPY4 receptor agonist, a leptin, a leptin derivative, a leptin agonist, a CNTF, a CNTF agonist/modulator, a CNTF derivative, a MCH1R antagonist, a MCH2R antagonist, a melanocortin 4 agonist, a MC4 receptor agonist, a cannabinoid receptor (CB-1) antagonist/inverse agonist, a ghrelin antagonist, a 5HT2c agonist, a serotonin reuptake inhibitor, a serotonin transport inhibitor, an exendin, an exendin derivative, an exendin agonist, a GLP-1, a GLP-1 analog, a GLP-1 agonist, a DPP-IV inhibitor, an opioid antagonist, an orexin antagonist, a metabotropic glutamate subtype 5 receptor antagonist, a histamine 3 antagonist/inverse agonist, topiramate,
   wherein the second anti-obesity agent is selected from the group consisting of a CCK, a CCK analog, a CCK agonist, an amylin, an amylin analog, and an amylin agonist,
   wherein when the first anti-obesity agent is not a PYY(3-36) a PYY(3-36) analog, or a PYY(3-36) agonist,
   wherein when the first anti-obesity agent is an exendin, an exendin derivative or an exendin agonist, then the second anti-obesity agent is not an amylin, an amylin agonist, an amylin analog, and
   wherein the anti-obesity agents are administered in amounts effective to reduce the body weight of the subject by at least 10%.
19. A method of reducing body weight in a subject comprising administering at least one of a leptin, a leptin derivative or a leptin agonist and at least one of an amylin, an amylin agonist or an amylin analog in amounts effective to reduce the body weight of the subject by at least 10%.
20. Use of a first anti-obesity agent selected from amylin, an amylin agonist or an amylin analog for the manufacture of a medicament for reducing body weight in a subject, for treating obesity or reducing nutrient availability, wherein the medicament is to be administered in combination with a second anti-obesity agent selected from a leptin, a leptin derivative or a leptin agonist, wherein the agents are to be administered in amounts effective to reduce the body weight of the subject by at least 10%.
21. Use of embodiment 20, wherein the medicament is for reducing body weight in a subject.
22. Use of embodiment 20, wherein the medicament is for reducing nutrient availability.
23. Use of embodiment 20, wherein the medicament is for treating obesity.
24. Use of any of embodiments 20 to 23, wherein the anti-obesity agents in combination are to be administered by simultaneous, concurrent or sequential administration.
25. Use of any of embodiments 20-24, wherein the second anti-obesity agent is selected from recombinant human leptin and recombinant human methionyl leptin.
26. Use of any of embodiment 20-25, wherein the first anti-obesity agent is pramlintide.
27. A kit comprising a first anti-obesity agent selected from amylin, an amylin agonist or an amylin analog and a second anti-obesity agent selected from a leptin, a leptin derivative or a leptin agonist for reducing body weight in a subject, for treating obesity or reducing nutrient availability, wherein the agents are to be administered in combination, and wherein the agents are to be administered in amounts effective to reduce the body weight of the subject by at least 10%.
28. Kit of embodiment 27, wherein the kit is for reducing body weight in a subject.
29. Kit of embodiment 27, wherein the kit is for reducing nutrient availability.
30. Kit of embodiment 27, wherein the kit is for treating obesity.
31. Kit of any of embodiments 27 to 30, wherein the anti-obesity agents in combination are to be administered by simultaneous, concurrent or sequential administration.
32. Kit of any of embodiments 27 to 31, wherein the second anti-obesity agent is selected from recombinant human leptin and recombinant human methionyl leptin.
33. Kit of any of embodiments 27 to 32, wherein the first anti-obesity agent is pramlintide.

## Claims

1. Use of a therapeutically effective amount of a first anti-obesity agent selected from the group consisting of: an amylin; an amylin analog; and an amylin agonist; for the manufacture of a medicament for treating obesity in a leptin-resistant obese subject, for reducing body weight in a leptin-resistant obese subject, or for reducing nutrient availability in a leptin-resistant obese subject, wherein the therapeutically effective amount of the first anti-obesity agent is administered in combination with a therapeutically effective amount of a second anti-obesity agent selected from the group consisting of: a leptin; a leptin derivative; and a leptin agonist;
wherein the therapeutically effective amount of the first anti-obesity agent and the therapeutically effective amount of the second anti-obesity agent are effective for treating the obesity in the leptin-resistant obese subject when administered in combination, wherein the therapeutically effective amount of the first anti-obesity agent and the therapeutically effective amount of the second anti-obesity agent are effective to reduce the body weight of the leptin-resistant obese subject when administered in combination, or wherein the therapeutically effective amount of the first anti-obesity agent and the therapeutically effective amount of the second anti-obesity agent are effective to reduce the nutrient availability in the leptin-resistant obese subject when administered in combination.

2. Use of a therapeutically effective amount of a first anti-obesity agent selected from the group consisting of: a leptin; a leptin derivative; and a leptin agonist; for the manufacture of a medicament for treating obesity in a leptin-resistant obese subject, for reducing body weight in a leptin-resistant obese subject, or for reducing nutrient availability in a leptin-resistant obese subject, wherein the therapeutically effective amount of the first anti-obesity agent is administered in combination with a therapeutically effective amount of a second anti-obesity agent selected from the group consisting of: an amylin; an amylin analog; and an amylin agonist;
wherein the therapeutically effective amount of the first anti-obesity agent and the therapeutically effective amount of the second anti-obesity agent are effective for treating the obesity in the leptin-resistant obese subject when administered in combination, wherein the therapeutically effective amount of the first anti-obesity agent and the therapeutically effective amount of the second anti-obesity agent are effective to reduce the body weight of the leptin-resistant obese subject when administered in combination, or wherein the therapeutically effective amount of the first anti-obesity agent and the therapeutically effective amount of the second anti-obesity agent are effective to reduce the nutrient availability in the leptin-resistant obese subject when administered in combination.

3. Use of a therapeutically effective amount of a first anti-obesity agent selected from the group consisting of: an amylin; an amylin analog; and an amylin agonist; and a therapeutically effective amount of a second anti-obesity agent selected from the group consisting of: a leptin; a leptin derivative; and a leptin agonist; for the manufacture of a medicament for treating obesity in a leptin-resistant obese subject, for reducing body weight in a leptin-resistant obese subject, or for reducing nutrient availability in a leptin-resistant obese subject, wherein the therapeutically effective amount of the first anti-obesity agent and the therapeutically effective amount of the second anti-obesity agent are administered in combination, and
wherein the therapeutically effective amount of the first anti-obesity agent and the therapeutically effective amount of the second anti-obesity agent are effective for treating the obesity in the leptin-resistant obese subject when administered in combination, wherein the therapeutically effective amount of the first anti-obesity agent and the therapeutically effective amount of the second anti-obesity agent are effective to reduce the body weight of the leptin-resistant obese subject when administered in combination, or wherein the therapeutically effective amount of the first anti-obesity agent and the therapeutically effective amount of the second anti-obesity agent are effective to reduce the nutrient availability in the leptin-resistant obese subject when administered in combination.

4. A composition comprising a therapeutically effective amount of a first anti-obesity agent selected from the group consisting of: an amylin; an amylin, analog; and an amylin agonist; for use in a method of treating obesity in a leptin-resistant obese subject, for reducing body weight in a leptin-resistant obese subject, or for reducing nutrient availability in a leptin-resistant obese subject, wherein the therapeutically effective amount of the first anti-obesity agent is administered in combination with a therapeutically effective amount of a second anti-obesity agent selected from the group consisting of: a leptin; a leptin derivative; and a leptin agonist;
wherein the therapeutically effective amount of the first anti-obesity agent and the therapeutically effective amount of the second anti-obesity agent are effective for treating the obesity in the leptin-resistant obese subject when administered in combination, wherein the therapeutically effective amount of the first anti-obesity agent and the therapeutically effective amount of the second anti-obesity agent are effective to reduce the body weight of the leptin-resistant obese subject when administered in combination, or wherein the therapeutically effective amount of first anti-obesity agent and the therapeutically effective amount of second anti-obesity agent are effective to reduce the nutrient availability in the leptin-resistant obese subject when administered in combination.

5. A composition comprising a therapeutically effective amount of a first anti-obesity agent selected from the group consisting of: a leptin; a leptin derivative; and a leptin agonist; for use in a method of treating obesity in a leptin-resistant obese subject, for reducing body weight in a leptin-resistant obese subject, or for reducing nutrient availability in a leptin-resistant obese subject, wherein the therapeutically effective amount of the first anti-obesity agent is administered in combination with a therapeutically effective amount of a second anti-obesity agent selected from the group consisting of: an amylin; an amylin analog; and an amylin agonist; and
wherein the therapeutically effective amount of the first anti-obesity agent and the therapeutically effective amount of the second anti-obesity agent are effective for treating the obesity in the leptin-resistant obese subject when administered in combination, wherein the therapeutically effective amount of the first anti-obesity agent and the therapeutically effective amount of the second anti-obesity agent are effective to reduce the body weight of the leptin-resistant obese subject when administered in combination, or wherein the therapeutically effective amount of the first anti-obesity agent and the therapeutically effective amount of the second anti-obesity agent are effective to reduce the nutrient availability in the leptin-resistant obese subject when administered in combination.

6. A composition comprising a first anti-obesity agent selected from the group consisting of: an amylin; an amylin analog; and an amylin agonist; and a therapeutically effective amount of a second anti-obesity agent selected from the group consisting of: a leptin; a leptin derivative; and a leptin agonist; for use in a method of treating obesity in a leptin-resistant obese subject, for reducing body weight in a leptin-resistant obese subject, or for reducing nutrient availability in a leptin-resistant obese subject, wherein the therapeutically effective amount of first anti-obesity agent and the therapeutically effective amount of the second anti-obesity agent are administered in combination,
wherein the therapeutically effective amount of the first anti-obesity agent and the therapeutically effective amount of the second anti-obesity are effective for treating the obesity in the leptin-resistant obese subject when administered in combination, wherein the therapeutically effective amount of first anti-obesity agent and the therapeutically effective amount of second anti-obesity are effective to reduce the body weight of the leptin-resistant obese subject when administered in combination, or wherein the therapeutically effective amount of first anti-obesity agent and the therapeutically effective amount of second anti-obesity are effective to reduce the nutrient availability in the leptin-resistant obese subject when administered in combination.

7. The use or composition according to claims 1 to 6 wherein the effective amount of the first anti-obesity agent and the effective amount of the second anti-obesity agent each comprises an amount such that a greater amount of weight loss is achieved when the first anti-obesity agent is administered in combination with the second anti-obesity agent to said subject than the amount of weight loss achieved when either the first anti-obesity agent or the second anti-obesity agent is administered alone.

8. The use or composition of any one of claims 1 to 7 wherein the first anti-obesity agent and the second anti-obesity agent are administered at the same time.

9. The use or composition of any one of claims 1 to 8 wherein the first anti-obesity agent and the second anti-obesity agent are mixed together.

10. The use or composition according to any one of claims 1 to 9 wherein the amylin, the amylin analog, or the amylin agonist is administered at from about 1 microgram per day, from about 5 micrograms per day, about 10 micrograms per day, from about 50 micrograms per day, or from about 100 micrograms per day; to about 100 micrograms per day, to about 500 micrograms per day, to about 1 milligram per day, to about 5 milligrams per day, to about 50 milligrams per day, or to about 100 milligrams per day; and/or the leptin, the leptin derivative, or the leptin agonist is administered at: from about 1 microgram per day, from about 5 micrograms per day, about 10 micrograms per day, from about 50 micrograms per day, or from about 100 micrograms per day; to about 100 micrograms per day, to about 500 micrograms per day, to about 1 milligram per day, to about 5 milligrams per day, to about 50 milligrams per day, or to about 100 milligrams per day; or from about 0.01 milligram per kilogram to about 0.3 milligram, or from about 0.01 milligram per kilogram to about 20 milligrams per kilogram.

11. The use or composition of any one of claims 1 to 10 wherein the leptin, the leptin derivative, or the leptin agonist is a dry formulation and the amylin, the amylin, analog, or the amylin agonist is a liquid formulation; wherein the leptin, the leptin derivative, or the leptin agonist dry formulation is reconstituted with the amylin, the amylin analog, or the amylin agonist liquid formulation; or wherein the dry formulation is a lyophilized formulation.

12. The use or composition of any one of claims 1 to 11 wherein the amylin, the amylin analog, or the amylin agonist and the leptin, the leptin derivative are formulated separately but packaged together; wherein the amylin, the amylin analog, or the amylin agonist and the leptin, the leptin derivative, or the leptin agonist are in separate chambered cartridges; and/or wherein the amylin, the amylin analog, or the amylin agonist and the leptin, the leptin derivative, or the leptin agonist are in separate chambers of a chambered syringe prior to reconstitution of the leptin, the leptin derivative, or the leptin agonist.

13. The use or composition according to any one of claims 1 to 12, wherein the subject has at least one condition selected from the group consisting of obesity, an obesity-related disorder, an obesity related disease, being overweight, an obesity-related condition, diabetes, insulin-resistance syndrome, nonalcoholic steatohepatitis, a cardiovascular disease, polycystic ovary syndrome, and metabolic syndrome.

14. The use or composition according to any one of claims 1 to 13 wherein the BMI is greater than 40; greater than 30; or wherein the BMI is 25 to 30.

15. The use or composition according to any one of claims 1 to 14, wherein the subject is a human adult female.

16. The use or composition according to any one of claims 1 to 15, wherein the subject reduces body weight by at least about 1%, by at least about 5%, by at least about 10%, by at least about 15%, or by at least about 20%.

17. A pharmaceutical composition for use in the method of any one of claims 1 to 16, wherein the composition comprises a therapeutically amount of the amylin, the amylin analog, or the amylin agonist and a therapeutically effective amount of the leptin, the leptin derivative, or the leptin agonist.

18. A pharmaceutical composition for the treatment of obesity in a leptin-resistant obese subject, for effecting weight loss in a leptin-resistant obese subject, or for reducing nutrient availability in a leptin-resistant obese subject, wherein said composition comprises a therapeutically effective amount of an amylin, an amylin analog, or an amylin agonist and a therapeutically effective amount of a leptin, a leptin derivative, or a leptin agonist according to any one of claims 1 to 16.

19. A pharmaceutical composition for the treatment of obesity in a leptin-resistant obese subject, for effecting weight loss in an obese subject, or for reducing nutrient availability in a leptin-resistant obese subject, wherein the composition comprises a therapeutically effective amount of a first anti-obesity agent selected from the group consisting of: an amylin, an amylin analog, and an amylin agonist; and a therapeutically effective amount of a second anti-obesity agent selected from the group consisting of: a leptin, a leptin derivative, and a leptin agonist;
wherein the therapeutically effective amount comprises an amount such that a greater amount of weight loss is achieved when the first anti-obesity agent and the second anti-obesity agent are administered in combination to said subject than the amount of weight loss achieved when either the first anti-obesity agent or the second anti-obesity agent is administered alone.

20. The use of a composition comprising: an amylin, an amylin analog,or an amylin agonist; and a leptin, a leptin derivative, or a leptin agonist; in the manufacture of a medicament for the treatment of obesity in a leptin-resistant obese subject, for effecting weight loss in a leptin-resistant obese subject, or for reducing nutrient availability in a leptin-resistant obese subject, according to any one of claims 1 to 19.

21. The use of a composition according to any of claims 1 to 16, wherein the subject is a leptin-resistant obese human subject.

22. The pharmaceutical composition according to any of claims 17-19, wherein the subject is a leptin-resistant obese human subject.

23. The use according to claim 20, wherein the human subject is a leptin-resistant obese human subject.

24. The use or composition according to any of claims 1-23, wherein the amylin agonist has: 60% amino acid sequence identity to SEQ ID NO:1; 65% amino acid sequence identity to SEQ ID NO:1; 70% amino acid sequence identity to SEQ ID NO:1; 75% amino acid sequence identity to SEQ ID NO:1; 80% amino acid sequence identity to SEQ ID NO:1; 85% amino acid sequence identity to SEQ ID NO:1; 90% amino acid sequence identity to SEQ ID NO:1; 95% amino acid sequence identity to SEQ ID NO:1; or 99% amino acid sequence identity to SEQ ID NO:1.

25. The use or composition according to any of claims 1-23, wherein the amylin agonist has: at least 75% amino acid sequence identity to any of SEQ ID NOS: 75 to 171; at least 80% amino acid sequence identity to any of SEQ ID NOS: 75 to 171; at least 85% amino acid sequence identity to any of SEQ ID NOS: 75 to 171; at least 90% amino acid sequence identity to any of SEQ ID NOS: 75 to 171; at least 95% amino acid sequence identity to any of SEQ ID NOS: 75 to 171; or at least 97% amino acid sequence identity to any of SEQ ID NOS: 75 to 171.
